(19) Europäisches Patentamt
European Patent Office
Office européen des brevets

(11) **EP 3 426 719 B1**

(12) # EUROPEAN PATENT SPECIFICATION

(45) Date of publication and mention of the grant of the patent:
**19.02.2025 Bulletin 2025/08**

(21) Application number: **17764084.4**

(22) Date of filing: **09.03.2017**

(51) International Patent Classification (IPC):
***C08J 3/09*** *(2006.01)* ***C08L 1/02*** *(2006.01)*
***C08L 5/08*** *(2006.01)* ***C08L 89/00*** *(2006.01)*
***B01J 20/24*** *(2006.01)* ***A61L 15/28*** *(2006.01)*
***A61L 15/32*** *(2006.01)* ***C07B 57/00*** *(2006.01)*
***A61P 31/04*** *(2006.01)*

(52) Cooperative Patent Classification (CPC):
(C-Sets available)
**A61L 15/225; A61L 15/46; A61P 31/04; B01J 20/06; B01J 20/24; B01J 20/28007; C07B 57/00; C08L 1/02; C08L 5/00; C08L 5/08; C08L 89/00;** A61L 2300/102; A61L 2300/104; A61L 2300/404; A61L 2400/12; (Cont.)

(86) International application number:
**PCT/US2017/021552**

(87) International publication number:
**WO 2017/156256 (14.09.2017 Gazette 2017/37)**

(54) **COMPOSITE MATERIALS CONTAINING STRUCTURAL POLYSACCHARIDES AND STRUCTURAL PROTEINS AND FORMED FROM IONIC LIQUID COMPOSITIONS**

VERBUNDWERKSTOFFE MIT STRUKTURELLEN POLYSACCHARIDEN UND STRUKTURELLEN PROTEINEN, HERGESTELLT AUS IONISCHEN FLÜSSIGKEITSZUSAMMENSETZUNGEN

MATÉRIAUX COMPOSITES CONTENANT DES POLYSACCHARIDES STRUCTURAUX ET DES PROTÉINES STRUCTURELLES ET FORMÉS À PARTIR DE COMPOSITIONS DE LIQUIDE IONIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2016 US 201662305757 P**

(43) Date of publication of application:
**16.01.2019 Bulletin 2019/03**

(73) Proprietor: **Marquette University**
**Milwaukee, WI 53201 (US)**

(72) Inventor: **TRAN, Chieu D.**
**Fox Point, WI 53217 (US)**

(74) Representative: **Boult Wade Tennant LLP**
**Salisbury Square House**
**8 Salisbury Square**
**London EC4Y 8AP (GB)**

(56) References cited:
**EP-A1- 3 528 856** **WO-A1-2014/186702**
**WO-A1-2018/075614** **WO-A2-2004/084627**

- **ROSEWALD MEGHANN ET AL.: "Cellulose-Chitosan-Keratin Composite Materials: Synthesis, Immunological and Antibacterial Properties", ECS TRANSACTIONS, vol. 64, no. 4, 2014, pages 499 - 505, XP055423639**
- **WU RONG-LAN ET AL.: "Cellulose/Soy Protein Isolate Blend Films Prepared via Room-Temperature Ionic Liquid", IND. ENG. CHEM. RES., vol. 48, 2009, pages 7132 - 7136, XP055423646**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PENG SHUAI ET AL.: "Nanoporous Magnetic Cellulose-Chitosan Composite Microspheres: Preparation, Characterization, and Application for Cu(II) Adsorption", IND. ENG. CHEM. RES., vol. 53, 2014, pages 2106 - 2113, XP055423648**
- **VENKATESWARLU SADA ET AL.: "Surfactant-Free Green Synthesis of Fe304 Nanoparticles capped with 3,4-Dihydroxyphenethylcarbamodithioate: Stable Recyclable Magnetic Nanoparticles for Rapid and Efficient Removal of Hg(II) Ions from Water", DALTON TRANS., vol. 44, 2015, pages 18427 - 18437, XP055423727**
- **TRAN CHIEU D. ET AL.: "Chitosan-cellulose composite materials: preparation, characterization and application for removal of microcystin", J. HAZARD MATER, 15 May 2013 (2013-05-15), pages 355 - 366, XP028541011**
- **DURI SIMON ET AL.: "Enantiomeric Selective Adsorption of Amino Acid by Polysaccharide Composite Materials", LANGMUIR, vol. 30, 2014, pages 642 - 650, XP055423731**
- **TRAN CHIEU D. ET AL.: "Recyclable Synthesis, Characterization, and Antimicrobial Activity of Chitosan-based Polysaccharide Composite Materials", J. BIOMED. MATERIALS RES. A, vol. 0, no. 8, 2013, pages 2248 - 2257, XP055423732**


(52) Cooperative Patent Classification (CPC): (Cont.)
B01J 2220/445; B01J 2220/46

C-Sets
**A61L 15/225, C08L 1/00;**
**A61L 15/225, C08L 5/08;**
**A61L 15/225, C08L 89/00;**
**C08L 1/02, C08L 89/00;**
**C08L 5/00, C08L 89/00;**
**C08L 5/08, C08L 1/02, C08L 89/00;**
**C08L 5/08, C08L 1/02, C08L 89/06;**
**C08L 5/08, C08L 89/00;**
**C08L 89/00, C08L 1/02;**
**C08L 89/00, C08L 5/00;**
**C08L 89/00, C08L 5/08**

## Description

STATEMENT REGARDING FEDERALLY SPONSORED RESEARCH OR DEVELOPMENT

This invention was made with government support under R15GM099033 awarded by the National Institutes of Health. The government has certain rights in the invention.

CROSS-REFERENCE TO RELATED PATENT APPLICATIONS

The present application claims the benefit of priority under 35 U.S.C. § 119(e) to U.S. Provisional Application No. 62/305,757.

BACKGROUND

The field of the invention relates to composite materials containing structural polysaccharides and structural proteins and ionic liquid composition for preparing the composite materials. Optionally, the composite materials may include metal or metal oxide particles. In particular, the field of the invention relates to composite materials containing structural polysaccharides, such as cellulose, chitin, or chitosan, structural proteins, such as keratin, optionally metal or metal oxide particles, such as gold, silver, or copper oxide particles, which composite materials are formed from ionic liquid compositions.

WO2018/075614 discloses composite materials containing structural polymers and photoreactive nitric oxide releasing agents and uses thereof for wound dressings. WO2004/084627 discloses a regenerated cellulose-encapsulated active substance and a method for encapsulating an active substance in a regenerated cellulose matrix. Wu et al. disclose cellulose/soy protein isolate blend films prepared via room-temperature ionic liquid (Industrial & Engineering Chemistry Research 2009 48 (15), 7132-7136). Peng et al. disclose nanoporous magnetic cellulose-chitosan composite microspheres (Industrial & Engineering Chemistry Research 2014 53 (6), 2106-2113). Venkateswarlu et al. disclose the surfactantfree green synthesis of Fe3O4 nanoparticles capped with 3,4-dihydroxyphenethylcarbamodithioate (Dalton Trans., 2015,44, 18427-18437). WO2014/186702 discloses composite materials containing structural polysaccharides and macrocyclic compounds formed from ionic liquid compositions.

SUMMARY

The invention is defined in the appended claims. Disclosed herein are composite materials comprising a structural polysaccharide which is a polymer comprising 6-carbon monosaccharides linked via beta-1,4 linkages and a structural protein which comprises keratin. The composite materials may be prepared from ionic liquid compositions of the invention comprising a polysaccharide which is a polymer comprising 6-carbon monosaccharides linked via beta-1,4 linkages and a structural protein comprising keratin dissolved in one or more ionic liquids forming liquid ionic compositions. The composite materials comprise metal nanoparticles and/or metal oxide nanoparticles, wherein the metal nanoparticles comprise gold, silver, or copper nanoparticles and/or wherein the metal oxide nanoparticles comprise gold, silver, or copper oxide nanoparticles.

The composite materials may be prepared from ionic liquid compositions of the invention. The metal and/or metal oxide nanoparticles are added to the one or more ionic liquid compositions, for example, as metal salts which subsequently are reduced *in situ.* The composite materials may be prepared from the ionic liquid compositions, for example, by removing the ionic liquid from the ionic liquid composition and retaining the structural polysaccharide, the structural protein, and the metal and/or metal oxide nanoparticles.

The compositions of the invention comprise a structural polysaccharide, which is a polymer of 6-carbon monosaccharides linked via beta-1,4 linkages. Suitable structural polysaccharides for the disclosed compositions may include, but are not limited to cellulose, chitin, and modified forms of chitin such as chitosan.

The disclosed composition may include any suitable concentration of the structural polysaccharide(s) for example, where the composition comprises at least about 5%, 10%, 15%, 20%, 25%, 30%, 35%, 40%, 45%, 50%, 55%, 60%, 65%, 70%, 75%, 80%, 85%, 90%, 95%, or 100% (w/w) of the structural polysaccharide(s), or the composition comprises less than about 100%, 95%, 90%, 85%, 80%, 75%, 70%, 65%, 60%, 55%, 50%, 45%, 40%, 35%, 30%, 25%, 20%, 15%, 10%, 5% (w/w) of the structural polysaccharide(s), or the composition comprises a concentration of the structural polysaccharide(s) within a range bounded by endpoints selected from any of the foregoing percentage concentrations (e.g., 5-25% (w/w)).

The disclosed compositions comprise keratin. Natural components that comprise keratin may be used to prepare the disclosed composite materials include, but are not limited to, wool, hair, and/or feathers.

The disclosed composition may include any suitable concentration of the structural protein(s) for example, where the composition comprises at least about 5%, 10%, 15%, 20%, 25%, 30%, 35%, 40%, 45%, 50%, 55%, 60%, 65%, 70%, 75%, 80%, 85%, 90%, 95%, or 100% (w/w) of the structural protein(s), or the composition comprises less than about 100%, 95%, 90%, 85%, 80%, 75%, 70%, 65%, 60%, 55%, 50%, 45%, 40%, 35%, 30%, 25%, 20%, 15%, 10%, 5% (w/w) of the structural protein(s), or the composition comprises a concentration of the structural protein(s) within a range bounded by end-points selected from any of the foregoing percentage concentrations (*e.g.*, 5-25% (w/w)).

The disclosed compositions may comprise a

selected ratio concentration of structural polysaccharide(s) to structural protein(s). For example, the compositions may comprise a percentage (w/w) of the structural polysaccharide(s) to percentage (w/w) of the structural protein(s) at a ratio selected from 100:0, 95:5, 90:10, 85:15, 80:20, 75:25, 70:30, 65:35, 60:40, 55:45, 50:50, 45:55, 40:60, 35:65, 30:70, 25:75, 20:80, 15:85, 10:90, 5:95, or 0:100, or a ratio range bounded by any of the foregoing ratios as end points for the ratio range *(e.g.,* 40:60 to 60:40 as a ratio range).

The disclosed composite materials may be formed from ionic liquid compositions of the invention. The metal and/or metal oxide nanoparticles are added to the ionic liquid composition (*e.g*., as metal salts which subsequently are reduced). Suitable ionic liquids for forming the ionic liquid compositions may include but are not limited to alkylated imidazolium salts. In some embodiments, the alkylated imidazolium salt is selected from a group consisting of 1-butyl-3-methylimidazolium salt, 1-ethyl-3-methylimidazolium salt, and 1-allyl-3-methylimidazolium salt. Suitable salts may include, but are not limited to chloride salts.

In the disclosed ionic liquid compositions, the structural polysaccharide is dissolved in an ionic liquid. In some embodiments, the ionic liquid may comprise at least about 2%, 4%, 6%, 8%, 10%, 15%, 20% w/w, dissolved structural polysaccharide, or a percentage range bounded by any of the foregoing percentages as end points for the percentage range *(e.g.,* 6% to 10%).

In the disclosed ionic liquid compositions, the structural protein is dissolved in the ionic liquid. In some embodiments, the ionic liquid may comprises at least about 2%, 4%, 6%, 8%, 10%, 15%, 20% w/w, dissolved structural protein, or a percentage range bounded by any of the foregoing percentages as end points for the percentage range *(e.g.,* 6% to 10%).

The disclosed ionic liquid compositions may be utilized in methods for preparing the disclosed composite materials that comprise the structural polysaccharide, the structural protein, and the metal and/or metal oxide nanoparticles. For example, in the disclosed methods, a composite material comprising the structural polysaccharide, the structural protein, and the metal and/or metal oxide nanoparticles may be prepared by: (1) obtaining or preparing an ionic liquid composition as disclosed herein comprising the structural polysaccharide and the structural protein, where the structural polysaccharide and the structural protein are dissolved in an ionic liquid to form an ionic liquid composition; optionally (2) adding a metal salt to the ionic liquid composition and optionally reducing the metal salt in situ, and (3) removing the ionic liquid from the ionic liquid composition; and (4) retaining the structural polysaccharide, the structural protein, and the optional metal and/or metal oxide salt in the form of particles. The ionic liquid may be removed from the compositions by steps that include, but are not limited to washing (*e.g.,* with an aqueous solution). The water remaining in the composite materials after washing may be removed from the composite materials by steps that include, but are not limited to drying (*e.g.,* in air) and lyophilizing (*i.e.,* drying under a vacuum). The composite material may be formed into any desirable shape, for example, a film or a powder (*e.g.,* a powder of microparticles and/or particles).

The disclosed composite materials may be utilized in a variety of processes. In some embodiments, the composite materials may be utilized to remove a contaminant from a stream (*e.g.,* a liquid stream or a gas stream). As such, the methods may include contacting the stream with the composite material and optionally passing the stream through the composite material. Contaminants may include, but are not limited to, chlorophenols (*e.g.,* 2-chlorophenol, 3-chlorophenol, 4-chlorophenol, 3,4-dichlorophenol, and 2,4,5-triochlorophenol), bisphenol A, 2,4,6-trichloroanisole (*e.g.,* as "cork taint" in wine), 1-methylocyclopropene, and metal ions (*e.g.,* $Cd^{2+}$, $Pb^{2+}$, and $Zn^{2+}$).

In other embodiments, the composite materials may be utilized to remove toxins from an aqueous environment, for example, as part of a filter treatment or as part of a batch treatment. For example, the composite material may be contacted with toxins in water whereby the toxins have an affinity for the composite material and the toxins are incorporated into the composite material thereby removing the toxins from the water. Toxins removed by the disclosed methods may include any toxins that have an affinity for the composite material, which may include bacterial toxins such as microcystins which are produced by cyanobacteria. After the composite material has been utilized to remove toxins from the aqueous environment, the composite material may be regenerated by treating the composite material in order to remove the toxins from the composite material and enable the composite material to be reused again (*i.e.,* via regeneration of the composite's capacity for adsorbing toxins).

In other embodiments, the composite material may be utilized to purify a compound (*e.g.,* from an aqueous solution, a liquid stream, or a gas stream). For example, the composite material may be utilized to purify a compound from an aqueous solution, a liquid stream, or a gas stream that comprises the compound by contacting the aqueous solution, the liquid stream, or the gas stream with the composite material where the composite material has an affinity for the compound to be purified. In some embodiments, the compound may be purified from a mixture of compounds in an aqueous solution, a liquid stream, or a gas stream, for example where the composite material had a greater affinity for the compound to be purified than for the other compounds in the mixture. The composite material may be contacted with the aqueous solution, the liquid stream, or the gas stream comprising the mixture of compounds in order to bind preferentially the compound to be purified to the composite material and remove the compound from the mixture of compounds in the aqueous solution, the liquid

stream, or the gas stream. In some embodiments, the compound to be purified is a specific enantiomer of the compound present in a racemic mixture of the compound, for example, where the composite material has a greater affinity for one enantiomer of the compound versus another enantiomer of the compound.

In other embodiments, the composite materials may be utilized to kill or eliminate microbes, including but not limited to bacteria and/or fungi. For example, the composite material may be contacted with bacteria including but not limited to *Staphylococcus aureus* (including methicillin-resistant strains), and *Enterococcus faecalis* (including vancomycinresistant strains), *Pseudomonas aeruginosa, Escherichia coli,* in order to kill or eliminate the bacteria. For example, the composite material may be contacted with fungi including but not limited to Candida species such as *Candida albicans.* The bacteria and/or fungi killed or eliminated in the disclosed methods may be present in an aqueous solution, a liquid stream, or a gas stream as contemplated herein.

In other embodiments, the composite material may be utilized to inhibit the attachment and biofilm formation in water of various microbes including but not limited to bacteria (such as *Pseudomonas aeruginosa, Escherichia coli, Staphylococcus aureus,* methicillin resistant S. *aureus* and vancomycin resistant *Enterococcus faecalis*) and/or fungi. For example, where a substrate is utilized in an aqueous environment, the substrate may be coated with the composite material in order to inhibit or prevent bacterial growth and/or fungal growth and biofilm formation on the substrate.

In other embodiments, the composite materials may be utilized for preparing a wound dressing or a bandage. For example, the composite materials may be utilized for preparing a wound dressing or a bandage for a wound where the composite material is in contact with the wound and promotes healing and inhibits growth of bacteria and/or fungi and/or kills bacteria and/or fungi. In some embodiments, the composite materials may further comprise a therapeutic agent, which may include but is not limited to, an anti-microbial agent (e.g., an anti-bacterial agent (such as an anti-biotic) and/or anti-fungal agent and/or an anti-viral agent).

Preferably, the composite material is biocompatible. For example, preferably the composite material is compatible with fibroblast adherence and viability, in particular, where the composite material is utilized as a wound dressing or as a bandage for a wound.

Preferably, the composite material exhibits anti-inflammatory activity. For example, preferably, the composite material inhibits production of pro-inflammatory cytokines such as interleukin-6 (IL-6) by immune cells such as macrophages. Optionally, an anti-inflammatory agent may be added to an ionic liquid composition for preparing the composite material in order to incorporate the anti-inflammatory agent into the composite material (e.g., after the ionic liquid is removed from the ionic liquid composition to obtain the composite material comprising the anti-inflammatory agent).

In other embodiments, the composite materials may be utilized to catalyze a reaction. For example, the composite materials may be utilized to catalyze a reaction by contacting a reaction mixture with the composite materials and optionally passing the reaction mixture through the composite material. In some embodiments, the composite material may include a reactive metal or metal cation for catalyzing the reaction (e.g., as metal or metal cation particles).

In other embodiments, the composite materials may be utilized to carry and release a compound such as a therapeutic compound *(e.g.,* an anti-microbial compound). For example, the composite materials may be utilized to carry and release a therapeutic compound gradually over an extended period of time *(e.g.,* a drug such as ciprofloxacin). As such, the composite material may be utilized in wound dressing material (*e.g.*, for ulcerous infected wounds).

In other embodiments, the composite materials may be utilized to carry and release an ethylene compound (*e.g.*, 1-methylocyclopropene). For example, the composite materials may be utilized to carry and release an ethylene compound in order to modulate ripening of fruit or freshness of flowers. As such, the composite material may be utilized in packaging material for fruit or flowers.

Accordingly, the disclosed composite materials may be configured for a variety of applications. These include, but are not limited to, filter material for use in filters for liquid or gas streams, fabric material for use in bandages for wounds, and/or packaging material for fruit or flowers.

BRIEF DESCRIPTION OF THE FIGURES

Figure 1. Procedure used to prepare the [CEL+CS+KER] composite materials.

Figure 2. FTIR spectra of materials with different compositions and concentrations; Hair, wool, feather, 100% CEL, 80:20 [Wool:CEL], 80:2 0 [Hair:CEL] and 80:20 [Feather:CEL].

Figure 3. X-ray diffraction spectra of (A): wool ( dashed curve), hair ( solid curve) and chicken feather (dotted curve); and (B): 80:20 wool:CEL (solid curve), 80:20 hair:CEL (dashed curve), 80:20 feather:CEL (dotted curve) and 100% CEL (line-dotted curve) composites.

Figure 4. Surface SEM images (top two rows) and cross-sectional images (last three rows) of CEL, Wool, [Wool+CEL], [Hair:CEL] and [Feather:CEL] composites with different compositions.

Figure 5. Plots of tensile strength as a function of % CEL in [CEL+Hair] composites (dotted curve), [CEL+Feather] composites (dashed-dotted curve) and [CEL+wool] composites (dashed curve).

Figure 6. Log of reduction in number of bacteria (A): *E. coli,* (B): *S. aureus,* (C): MRSA, (D): VRE) after exposure to [CEL+Hair], [CEL+Feather] and [CEL+Wool] composites for 24 hours compared to a control (no composite). Each bar represents an average of 3 measurements together with associated standard deviations.

Figure 7. Procedure used to prepare the [CEL+KER+AgNPs] composite materials.

Figure 8. FTIR spectra of [CEL+KER] composite (bottom line) and [CEL+KER+AgNPs] composite (top line).

Figure 9. Powder X-Ray diffraction spectra of [CEL+KER+$Ag^+$NPs] composite (top line) and [CEL+KER+$Ag^0$NPs] composite (bottom).

Figure 10. (A) SEM images of [CEL+KER+AgNPs] composite: left: surface image, right: cross section image; (B) EDS spectrum and (C ) EDS images, recorded for carbon (left), silver (middle) and oxygen (right) of [CEL+KER+AgNPs] composite.

Figure 11. Log of growth reduction for *E. coli, S. aureus,* VRE, MRSA and *P. aeruginosa* after 24 hrs exposure to: Top: [CEL+KER+$Ag^+$NPs] and [CEL+KER+$Ag^0$NPs] composites with 3.5 mmol of silver NPs concentrations; and Bottom: [CEL+KER+$Ag^+$NPs] and [CEL+KER+$Ag^0$NPs] composites with silver NPs concentrations of 3.5mmol, 0.72 mmol and 0.48 mmol for (A) *E. coli;* (B) *S. aureus,* (C) VRE; (D) MRSA; and (E ) *P. aeruginosa*). In these figures, CEL+KER was labeled as CK; hatched bars and black bars are for (CEL+KER+$Ag^+$] and [CEL+KER+$Ag^0$NPS] composites, respectively. Light grey bars are for both blank ([CEL+KER] composite with no AgNPs) and control.

Figure 12. Fibroblast viability based on absorbance at 490 nm after being exposed to [CEL+KER] composite, [CEL+KER+$Ag^0$NPs] composite and [CEL+KER+$Ag^+$NPs] composite for 3 days. In A) the composites of 15 mm in diameter were used, in (B) and (C) the composites were of 7 mm in diameter. Each bar represents an average of 3 experiments. Error bars represent standard error of the average. (P-values are indicated as follows: (*$P < 0.05$)). Results for control experiment (no material) are also presented. Composites causing <70% cell viability (dashed line) are considered cytotoxic.

Figure 13. Images (100x) of human fibroblasts after 3 days in the absence of any composite (A), with [CEL+KER] composite (B), with [CEL+KER] containing 0.48 mmol of $Ag^0$NPs (C), and with [CEL+KER] containing 0.72 mmol of $Ag^0$NPs (D).

Figure 14. Sample preparation for silver release from the [CEL+KER+$Ag^0$NPs] composites.

Figure 15. Schematic presentation of the FIA setup with thermal lens detection unit.

Figure 16. Plot of concentration of silver nanoparticle released from the composites against time the composites were immersed in the solution similar to the media used in the microbial and biocompatibility assays.

DETAILED DESCRIPTION

The disclosed subject matter further may be described utilizing terms as defined below.

Unless otherwise specified or indicated by context, the terms "a", "an", and "the" mean "one or more." For example, "a structural polysaccharide" and "a structural protein" should be interpreted to mean "one or more structural polysaccharides" and "one or more structural proteins," respectively.

As used herein, "about", "approximately," "substantially," and "significantly" will be understood by persons of ordinary skill in the art and will vary to some extent on the context in which they are used. If there are uses of the term which are not clear to persons of ordinary skill in the art given the context in which it is used, "about" and "approximately" will mean plus or minus ≤10% of the particular term and "substantially" and "significantly" will mean plus or minus >10% of the particular term.

As used herein, the terms "include" and "including" have the same meaning as the terms "comprise" and "comprising" in that these latter terms are "open" transitional terms that do not limit claims only to the recited elements succeeding these transitional terms. The term "consisting of," while encompassed by the term "comprising," should be interpreted as a "closed" transitional term that limits claims only to the recited elements succeeding this transitional term. The term "consisting essentially of," while encompassed by the term "comprising," should be interpreted as a "partially closed" transitional term which permits additional elements succeeding this transitional term, but only if those additional elements do not materially affect the basic and novel characteristics of the claim.

Disclosed are composite materials and ionic liquid compositions for preparing the composite materials. The composite materials include a structural polysaccharide which is a polymer comprising 6-carbon monosaccharides linked via beta-1,4 linkages, a structural protein comprising keratin, and metal and/or metal

oxide nanoparticles (e.g., metal microparticles and/or metal nanoparticles), wherein the metal nanoparticles comprise gold, silver, or copper nanoparticles and/or wherein the metal oxide nanoparticles comprise gold, silver, or copper oxide nanoparticles.

As used herein, "structural polysaccharides" refer to water insoluble polysaccharides that may form the biological structure of an organism. In the invention, structural polysaccharides are polymers of 6-carbon sugars such as glucose or modified forms of glucose (e.g., N-acetylglucosamine and glucosamine), which are linked via beta-1,4 linkages. Structural polysaccharides may include, but are not limited to cellulose, chitin, and chitosan, which may be formed from chitin by deacetylating one or more N-acetylglucosamine monomer units of chitin via treatment with an alkali solution (e.g., NaOH). Chitosan-based polysaccharide composite materials and the preparation thereof are disclosed in Tran et al., J. Biomed. Mater. Res. Part A 2013:101A:2248-2257 (hereinafter "Tran et al. 2013).

As used herein, a "structural protein" is a protein that is used to build structural components of an organism. Structural proteins may include fibrous structural proteins, which optionally may be referred to as "scleroproteins." Structural proteins typically do not include globular proteins and/or membrane proteins. Structural proteins typically form long filaments which are water-insoluble. Structural proteins may comprise hydrophobic side chains that protrude from the structural protein molecule and cause structural proteins to aggregate. The peptide sequence of structural proteins typical includes a limited variety of amino acid residues and includes repeat motifs that may form secondary structures such as helices having disulfide bond between the structural protein amino acid chains. In the invention, the structural protein comprises keratin. Other structural proteins include collagen, elastin, and fibroin.

In the invention, the structural protein comprises keratin . Suitable keratin proteins may include, but are not limited to, α-keratins and/or β-keratins. Keratin for use in the disclosed methods for preparing the disclosed composite materials may be derived from a number of sources, including but not limited to wool, hair (including human and non-human hair), feathers (including chicken feathers), beaks (including chicken beaks), claws (including chicken claws), and hooves of ungulates.

The disclosed composite materials may be prepared from ionic liquid compositions that comprise the structural polysaccharide and the structural protein dissolved in one or more ionic liquids. As used herein, an "ionic liquid" refers to a salt in the liquid state, typically salts whose melting point is less than about 100°C. Ionic liquids may include, but are not limited to salts based on an alkylated imidazolium cation, for example,

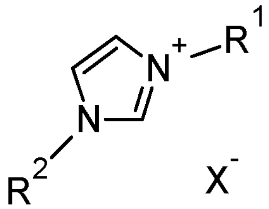

where $R^1$ and $R^2$ are C1-C6 alkyl (straight or branched), and $X^-$ is any cation (*e.g.,* a halide such as chloride, a phosphate, a cyanamide, or the like).

The disclosed composite materials may be utilized in methods for removing contaminants from aqueous solutions, liquid streams, or air streams. Chitosan-cellulose composite materials for removing microcystin are disclosed in Tran et al., J. of Hazard. Mat. 252-253 (2013) 355-366.

The disclosed composite materials may be utilized in methods for purifying compounds from aqueous solutions, liquid streams, or air streams. In particular, the composite materials may be utilized in methods for purifying compounds from mixtures of compounds. Methods of using a chitosan-cellulose composite material for purifying a specific enantiomer of an amino acid from a racemic mixture are disclosed in Duri et al. Langmuir, 2014, 30(2), pp 642-650 (hereinafter "Duri *et al.* 2014"). As disclosed in Duri *et al.* 2014, in methods for purifying an enantiomer of a compound from a racemic mixture of a compound, the composite material may consist of structural polysaccharides (*e.g.*, chitosan and cellulose). As such, the presence of a metal and/or metal oxide particles within the composite material may be optional but preferred where the composite material is utilized in methods for purifying an enantiomer of a compound from a racemic mixture of a compound.

The disclosed composite materials may be utilized in methods for inhibiting or preventing growth of microbes (*e.g.*, bacteria). For example, the disclosed composite materials may be contacted with an aqueous solution, a liquid stream, or an air stream comprising microbes to inhibit or prevent growth of microbes in the aqueous solution, the liquid stream, or the air stream. Alternatively, the disclosed composite materials may be used to coat a substrate in order to inhibit or prevent growth of microbes on the substrate. The antimicrobial properties of chitosan-based polysaccharide composite materials are disclosed in Tran et al., J. Biomed. Mater. Res. Part A 2013:101A:2248-2257 (hereinafter "Tran et al. 2013) and Harkins AL, Duri S, Kloth LC, Tran CD. 2014. "Chitosan-cellulose composite for wound dressing material. Part 2. Antimicrobial activity, blood absorption ability, and biocompatibility." J Biomed Mater Res Part B 2014: 00B: 000-000 (hereinafter "Harkins et al. 2014"). As disclosed in Tran *et al.* 2013 and Harkins *et al.* 2014, in methods of using the disclosed composite materials for inhibiting or preventing microbial growth, the composite material may consist of structural polysaccharides (*e.g.*, chitosan and cellulose).

The disclosed composite materials may include therapeutic agents. In order to prepare composite materials comprising therapeutic agents, the therapeutic agents may be added to an ionic liquid composition comprising the structural polysaccharide and structural protein dissolved therein. The present inventor has observed that the release rate for therapeutic agents incorporated in to the composite materials will vary based on the composition of the composite materials. Composite materials comprising cellulose [CEL] and chitosan [CS] or a combination of cellulose/chitosan [CEL+CS] exhibiting much faster release rates for ciprofloxacin than a composite material comprising keratin [KER]. Ciprofloxacin was released more slowly from composite materials comprising keratin and the release rate for ciprofloxacin from composite materials comprising keratin was dependent on the concentration of keratin in the composite material. Because the release rate of ciprofloxacin by [CEL+CS+KER] composites is relatively slower than a CEL composite, a CS composite, or a [CEL+CS] composite, and because the release rate is inversely proportional to the concentration of keratin in the composite, a drug such as ciprofloxacin can be encapsulated into a [CEL+CS+KER] composite, and the release of the drug from the composite can be adjusted to a selected release rate by judiciously selecting the concentration of KER in the composite.

The disclosed composite materials may include additional components such as macromolecules. In this regard, reference is made to Duri et al., "Supramolecular Composition Materials from Cellulose, Chitosan, and Cyclodextrins: Facile Preparation and Their Selective Inclusion Complex Formation with Endocrine Disruptors," Langmuir. 2013. 29(16):5037-49, available on-line on March 21, 2013. In this regard, reference also is made to Published International Application WO 2014/186702, published on November 20, 2014.

The disclosed composite materials include one or more metal and/or metal oxide nanoparticles, wherein the metal nanoparticles comprise gold, silver, or copper nanoparticles and/or wherein the metal oxide nanoparticles comprise gold, silver, or copper oxide nanoparticles. The disclosed metal and/or metal oxide nanoparticles may have an effective average diameter of less than about 10 $\mu$M, 5 $\mu$M, 1 $\mu$M, 0.5 $\mu$M or 0.1 $\mu$M, or the particles may have an effective average diameter within a range bounded by any of the foregoing values as endpoints (*e.g.*, particles having an effective average diameter within a range of 1 $\mu$M to 0.1 $\mu$M). In the invention, the disclosed metal and/or metal oxide particles are referred to as "nanoparticles."

In order to prepare composite materials comprising the metal and/or metal oxide nanoparticles, the metal and/or metal oxide nanoparticles may be added to an ionic liquid composition comprising the structural polysaccharide and structural protein dissolved therein. The ionic liquid then may be removed from the composition to prepare a composite material comprising the structural polysaccharide, structural protein, and the metal or metal oxide nanoparticles. In some embodiments, a metal salt comprising a metal cation and a non-metal cation may be added to an ionic liquid composition comprising the structural polysaccharide and structural protein dissolved therein. The ionic liquid then may be removed from the composition to prepare a composite material comprising the structural polysaccharide, structural protein, and the metal salt. The metal cation of the metal salt may then be reduced in the composite in situ to create metal particles comprising elemental metal. In the invention, metals and oxides thereof for the disclosed composites include silver (Ag), gold (Au), or copper (Cu). Other metals and oxides thereof described herein include platinum (Pt), nickel (Ni), palladium (Pd), rhodium (Rh), aluminum (Al), iron (Fe), zinc (Zn), manganese (Mn), cobalt (Co), molybdenum (Mo).

In the synthesis method, preferably the silver nanoparticles are homogenously encapsulated and distributed in the composite during its synthesis. In the synthesis method, the nanoparticles may be recovered and recycles after each use to prevent problems associated with contamination of samples by the nanoparticles. In the synthesis method, preferably the oxidation state of silver nanoparticles ($Ag^0$ or $Ag^+$) can be selected by adjusting the reduction reaction. For example, the reduction reaction may be controlled to provide a composite material having a desired ratio of reduced metal versus oxidized metal (*e.g.*, where $M^0$:$M^+$ is greater than about 50:50, 60:40, 70:30, 80:20, 90:10, 95:5, 96:4, 97:3, 98:2, or 99:1, or where $M^0$:$M^+$ is within a range bounded by any of the foregoing ratios such as a range of 70:30 to 90:10). The antimicrobial activity can be measured for composites containing different concentrations of $Ag^0$ or $Ag^+$.

## EXAMPLES

The following examples are illustrative and are not intended to limit the claimed subject matter.

### Example 1 - Synthesis, structure and antimicrobial property of green composites from cellulose, wool, hair and chicken feather

Reference is made to Tran et al., "Synthesis, structure and antimicrobial property of green composites from cellulose, wool, hair and chicken feather," Carbohydrate Polymers, 151 (2016) 1269-1276.

Abstract\

Novel composites between cellulose (CEL) and keratin (KER) from three different sources (wool, hair and chicken feather) were successfully synthesized in a simple one-step process in which butylmethylimidazolium chloride ($BMIm^+Cl^-$), an ionic liquid, was used as the sole solvent. The method is green and recyclable because

[$BMIm^+Cl^-$] used was recovered for reuse. Spectroscopy (FTIR, XRD) and imaging (SEM) results confirm that CEL and KER remain chemically intact and homogeneously distributed in the composites. KER retains some of its secondary structure in the composites. Interestingly, the minor differences in the structure of KER in wool, hair and feather produced pronounced differences in the conformation of their corresponding composites with wool has the highest $\alpha$-helix content and feather has the lowest content. These results correlate well with mechanical and antimicrobial properties of the composites. Specifically, adding CEL into KER substantially improves mechanical strength of [CEL+KER] composites made from all three different sources, wool, hair and chicken feathers (i.e., [CEL+wool], [CEL+hair] and [CEL+feather]. Since mechanical strength is due to CEL, and CEL has only random structure, [CEL+feather] has, expectedly, the strongest mechanical property because feather has the lowest content of $\alpha$-helix. Conversely, [CEL+wool] composite has the weakest mechanical strength because wool has the highest $\alpha$-helix content. All three composites exhibit antibacterial activity against methicillin resistant *S. aureus* (MRSA). The antibacterial property is due not to CEL but to the protein and strongly depends on the type of the keratin, namely, the bactericidal effect is strongest for feather and weakest for wool. These results together with our previous finding that [CEL+KER] composites can control release of drug such as ciprofloxacin clearly indicate that these composites can potentially be used as wound dressing.

## Introduction

Sustainability, industrial ecology, eco-efficiency, and green chemistry are directing the development of the next generation of materials. Biodegradable and biocompatible materials generated from renewable biomass feedstock are regarded as promising materials that could replace synthetic polymers and reduce global dependence on fossil fuel sources. The most abundant biorenewable biopolymers on the earth include polysaccharide such as cellulose and keratin (wool, hair and chicken feather).

Keratins (KER) are a group of cysteine-rich fibrous proteins found such materials as wools, hairs, chicken feather, nails (Dullaart, R. & Mousquès, J., 2012). Of particular interest are hairs and chicken feathers as these materials are an important waste product from the salons and poultry industry but are generally left untreated because they have limited solubility and cannot be easily and economically converted to environmentally benign products (Verma et al., 2008; Vilaplana et al., 2010). Keratins are known to possess advantages for wound care, tissue reconstruction, cell seeding and diffusion, and drug delivery as topical or implantable biomaterial (Cui et al., 2013; Hill et al., 2010; Justin et al. 2011; Vasconcelos et al., 2013). As implantable film, sheet, or scaffold, keratins can be absorbed by surrounding tissue to provide structural integrity within the body while maintaining stability under mechanical load, and in time can break down to leave neo-tissue (Cui et al., 2013; Hill et al., 2010; Justin et al. 2011; Vasconcelos et al., 2013; Verma et al., 2008). The abundance and regeneration nature of wools, hairs and feathers coupled with the ability to be readily to be converted into biomaterials have made KER a subject of intense study (Justin et al. 2011; Vasconcelos et al., 2013; Vilaplana et al., 2010).

Unfortunately, KER has relatively poor mechanical properties, and as a consequence, materials made from KER lack the stability required for medical applications (Cui et al., 2013; Hill et al., 2010; Sando et al., 2010; Vasconcelos et al., 2013; Verma et al., 2008). To increase the structural strength of KER-based materials, attempts have been made to crosslink KER chains with a crosslinking agent or convert functional groups on its amino acid residues via chemical reaction(s) (Justin et al. 2011; Sando et al., 2010; Vasconcelos et al., 2013). The rather complicated, costly and multistep process is not desirable as it may inadvertently alter its unique properties, making the KER-based materials less biocompatible and toxic, and removing or lessening its unique properties. A new method which can improve the structural strength of KER-based products not by chemical modification with synthetic chemicals and/or synthetic polymers but rather by use of naturally occurring polysaccharides such as CEL, is particularly needed.

We have demonstrated recently that a simple ionic liquid, butylmethylimmidazolium chloride ([$BMIm^+Cl^-$]), can dissolve polysaccharides such as CEL and chitosan (CS), and by use of this [$BMIm^+Cl^-$] as the sole solvent, we developed a simple, green and totally recyclable method to synthesize [CEL+CS] composites just by dissolution without using any chemical modifications or reactions (Duri & Tran, 2013; Harkins et al., 2014; Mututuvari & Tran, 2013; Mututuvari & Tran, 2014; Tran et al., 2013a; Tran et al, 2013b). The [CEL+CS] composite obtained was found to be not only biodegradable and biocompatible but also retain unique properties of its components. Since [$BMIm^+Cl^-$] can also dissolve wool keratin (Chen et al, 2014; Xie et al, 2005), it may be possible to use this IL as a solvent to synthesize composites containing CEL and keratin. In fact, Xie et al have shown that wool keratin can be regenerated by initially dissolving in [$BMIm^+Cl^-$] and subsequently precipitated from methanol, and with this procedure, there were able to synthesize a 1/5 wool keratin /cellulose composite (Xie et al, 2005). Recently, by using [$BMIm^+Cl^-$] as a sole solvent we were able to synthesize composites from cellulose, chitosan and wool keratin with different compositions and concentrations (Tran &Mututuvari). More importantly, we demonstrated that the composites can be used for drug delivery as the kinetics of the release can be controlled by adjusting the concentration of wool keratin in the composite (Mututuvari & Tran, 2014).

Such consideration prompted us to initiate this

study which aims to improve the mechanical properties of the KER-based composites by adding CEL to the composites, and to demonstrate that the composites will retain unique properties of their components. Since KER is known to have different structure and conformation depending on the source, (i.e., wool, hair or chicken feather) we synthesized [CEL+KER] composites with KER from either of wool, hair or chicken feather. Various spectroscopic and imaging techniques including FTIR, powder X-ray diffraction, SEM and tensile strength were employed to characterize the composites and to determine their structure and property. Microbial assays were carried out to determine antimicrobial property of the composites, results obtained were correlated with the structure and conformation of the composites to formulate structure-property relationship for the composites. The results of our initial investigation are reported herein.

Methods

Chemicals. Microcrystalline cellulose (DP≈300) was purchased from Sigma-Aldrich (Milwaukee, WI). Untreated hair from local saloons and chicken feathers from local poultry farms were washed with 0.5% SDS aqueous solution, rinsed with fresh water and air-dried, followed with additional cleaning by Soxhlet extraction with petroleum ether for 48 hrs. Raw sheep wool (untreated), obtained from a local farm, was cleaned by Soxhlet extraction with a 1:1 acetone/ethanol mixture for 48 hrs. [$BMIm^+Cl^-$] was prepared from freshly distilled 1-methylimidazole and n-chlorobutane (both from Alfa Aesar, Ward Hill, MA) using method previously reported ( Duri & Tran, 2013; Haverhals et al., 2012).

Instruments. FTIR spectra (from 450-4,000 $cm^{-1}$ were recorded on a Spectrum 100 Series FTIR spectrometer (Perkin Elmer, USA) at resolution of 2 $cm^{-1}$ by the KBr method. Each spectrum was an average of 64 individual spectra. X-ray diffraction (XRD) measurements were taken on a Rigaku MiniFlex II diffractometer utilizing the Ni filtered Cu Kα radiation (1.54059Å). The voltage and current of the X-ray tube were 30kV and 15mA respectively. The samples were measured within the 20 angle range from 2.0 to 85. The scan rate was 50 per minute. Data processing procedures were performed with the Jade 8 program package (Duri et al., 2010). The surface and cross-sectional morphologies of the composite films were examined under vacuum with a JEOL JSM-6510LV/LGS Scanning Electron Microscope with standard secondary electron (SEI) and backscatter electron (BEI) detectors. Prior to SEM measurement, the film specimens were made conductive by applying a 20 nm gold-palladium-coating onto their surfaces using an Emitech K575x Peltier Cooled Sputter Coater (Emitech Products, TX). The tensile strength of the composite films were evaluated on an Instron 5500R tensile tester (Instron Corp., Canton, MA) equipped with a 1.0 kN load cell and operated at a crosshead speed of 5 mm $min^{-1}$. Each specimen had a gauge length and width of 25 mm and 10 mm respectively. Thermogravimetric analyses (TGA) (TG 209 F1, Netzsch) of the composite films were investigated at a heating rate of 10 °C $min^{-1}$ from 30-600 °C under a continuous flow of 20 mL $min^{-1}$ nitrogen gas.

In vitro antibacterial assays. Nutrient broth (NB) and nutrient agar (NA) were obtained from VWR (Radnor, PA). The bacterial cultures used in this study were obtained from the American Type Culture Collection (ATCC, Rockville, MD). Seven different composites with different compositions and concentrations were used. They were 40:60 Hair:CEL; 40:60 Feather:CEL, 65:35 Hair:CEL, 65:35 Feather:CEL, 80:20 Hair:CEL, 75:25 Feather:CEL and 90:10 Hair:CEL.

The composites were tested for antibacterial activity on model bacterial strains *E. coli* (ATCC 8739), *Staphylococcus aureus* (ATCC 25923), methicillin resistant S. *aureus* (ATCC 33591), vancomycin resistant *Enterococcus faecalis* (ATCC 51299), and *Pseudomonas aeruginosa* (ATCC 9027) using previously published protocol (Harkins et al., 2014; Mututuvari et al., 2013; Tran et al., 2013a).

Preparation of the overnight bacterial culture included inoculation of 10 mL of nutrient broth medium with a culture that was maintained on a blood agar at 4°C using an inoculation loop. The culture was then incubated overnight at 37°C and 150 rpm. The next day the composites were placed in the sterile tubes with 2 mL of nutrient broth, which was then inoculated with 2 μL of the overnight culture. The tubes were then sampled at time 0 and placed into an incubator at 37°C and 600 rpm for 24-hour incubation. The samples taken at time 0 were then diluted to desirable dilutions, plated onto nutrient agar, and incubated overnight at 37°C. The next day the colony forming units (CFUs) were counted on statistically significant plates: 30 - 300 (CFUs) using the standard plate counts, also known as plate count agar (PCA) method (Jorgensen et al. 2009). The tubes were again sampled at time 24 hours and the dilution and plating procedure from the previous day was repeated. The plates were incubated overnight at 37°C. The next day the CFUs were counted again. From the CFU data obtained from time 0 and 24 hours, log of reduction of bacteria defined as follows was calculated for each experiment:

$$Log\ of\ reduction = \log \frac{N_0}{N_t}$$

where $N_0$ is the number of bacteria at the beginning of the experiment, and $N_t$ is the number of bacteria after 24 hours.

Results and Discussion

Fourier transform infrared (FTIR). FTIR was used to confirm that ionic liquid does not produce any chemical alterations during the dissolution of wool, hair,

chicken feather, and CEL and the synthesis the [Wool+CEL], [Hair+CEL] and [Feather+CEL] composites, and to characterize the composites. Shown in Figure 2 are the FT-IR spectra of the CEL powder, wool, hair and chicken feather as well as of the composites (80:20 wool:CEL, 80;20 hair:CEL and 80:20 feather:CEL). The spectra of the starting materials, wool, hair and feather are very similar which is as expected as these materials contain keratin, and the only difference among them is a few amino acid residues and some differences in their secondary structures. All three materials exhibit several bands including two large bands at around 1520 $cm^{-1}$ and 1643 $cm^{-1}$ (bending of the N-H of the amide bands), and the 1216 $cm^{-1}$ band which can be attributed to the in phase combination of the N-H bending and the C-N stretch vibrations (amide III) (Greve et al., 2008; Sowa et al., 1995). It is noteworthy to add that the FTIR spectrum of wool does not have any band at 1745 $cm^{-1}$, which is known to be due to lipid ester carbonyl vibrations (Tanabe et al., 2002). It seems, therefore, that the Soxhlet extraction effectively removed all residual lipids from wool. For reference, the spectrum of CEL powder was also taken. It exhibits several distinct different bands at around 1350$cm^{-1}$ , 1147 $cm^{-1}$ and 800 $cm^{-1}$ which can be tentatively, assigned to the O-H bending vibration, the C-O stretching (of the C-OH group) and the C-H stretching, respectively (Duri & Tran, 2013; Harkins et al., 2014; Mututuvari & Tran, 2014; Tran et al., 2013a; Tran et al, 2013b).

The spectra of composites between 20% CEL and 80% of either of wool, hair or feather are also presented in Figure 2. As expected, the spectra of these composites exhibit bands characteristic of their respective components, namely, the bands at 1520 $cm^{-1}$, 1643 $cm^{-1}$ and 1216 $cm^{-1}$ from KER and the 1350$cm^{-1}$ , 1147 $cm^{-1}$ and 800 $cm^{-1}$ bands of CEL. Furthermore, the magnitude of these bands seems to correlate well with the concentration of corresponding component in the film. For example; the bands due to CEL in the composites correspond to 20% to those in the CEL powder whereas the KER bands are about 80% to those of wool, hair and feather.

Powder X-ray diffraction (XRD). Figure 3A shows XRD spectra for wool, hair and chicken. Wool (dashed curve) exhibits two bands at 2θ of about 9° and 20°. They can be attributed to the α-helix and other structures including β-sheet and random form, respectively (Appelbaum et al., 2007; McKittrick et al., 2012). As expected, hair (solid curve) and feather (dotted curve) also have similar spectrum as that of wool. However, the relative intensity of the two bands at 9° and 20° for hair and feather are different from that of wool. Since the total intensity, or rather the area under these two bands are the same (i.e., 100% or total structure of the composite which includes α-helix and other structures including β-sheet and random form), the fact that the bands at 2θ=20° for both hair and feather are of relatively higher intensity than that of wool while their α-helix bands at 9° are similar to that of wool clearly indicates that the α-helix content is highest for wool followed by hair with feather has the lowest content.

XRD spectra of 80:20 wool:CEL (dashed curve), 80:20 hair:CEL (solid curve), 80:20 feather:CEL (dotted curve) and 100%CEL (line-dotted curve) composites are also presented in Figure 3B. Different from pure wool, hair and feather, all three composites exhibit a pronounced band at around 2θ=20° and a shoulder at 2θ=9°. In fact the spectra of all three composites are similar to the spectrum of the regenerated 100%CEL which is known to have only random structure. These results seem to indicate that adding CEL to these KER materials substantially decreases the α-helix structure while increase the β-sheet and other forms. It seems that during the dissolution with [$BMIm^+Cl^-$], the inter- and intra-molecular bonds in wool, hair and feather were broken thereby destroying its secondary structure while maintaining its primary structure. During gelation, regeneration from water and drying, these interactions were reestablished thereby partially reforming some of the original secondary structure. However, in the presence of CEL the chains are maintained in the extended form thereby hindering a significant reformation of the α-helix. Consequently, the composites formed may adopt structures with relatively lower content of α-helix and higher β-sheet content.

Scanning electron microscope (SEM). Figure 4 shows SEM images of the surfaces and cross sections of regenerated 100% CEL, 100% wool, [CEL+Wool], [CEL+Hair] and [CEL+Feather] composites with different compositions. While images for 100% CEL exhibit smooth and homogeneous morphologies without any pores, the images of 100% wool exhibit a rough and porous structure with a three dimensional interconnection throughout the film surface. This porous structure seems to reflect the physical properties of KER films, namely the brittleness of the regenerated 100% wool film, and the fact that it was not possible for us to regenerate 100% hair and 100% feather films as they were found to be too brittle. CEL was added to wool, hair and feather to improve mechanical property of the composites. From both surface and cross sections SEM images of [wool+CEL], [feather+CEL] and [hair+CEL] at various compositions (90:10, 80:20 and 65:35) it is clear that CEL forms homogenous composites with all three proteins and at all compositions. As expected, adding KER to the proteins introduces roughness to the composites. Moreover, the microstructures of the composites are dependent on the source of KER (i.e., wool, hair or feather) are noticeably different from one another. For example, 90:10 wool:CEL composite seems to be somewhat rougher than 100%CEL and 100%wool. It is, however, relatively finer than the corresponding 90:10 hair:CEL composite. On the other hand, the 90:10 feather:CEL composite exhibits highest degree of roughness. Again these results seem to correlate with results presented above on the conformation of the proteins,

namely, because wool has the highest α-helix content, when mix with CEL, it still can retain some of its structure, thereby producing composites with relatively finer structure than those of hair and feather. Conversely, feather which has the lowest α-helix content, does not seem to be able to mix well with CEL. As a consequence, the resultant composites have the highest degree of roughness compared to corresponding wool and hair composites. Since CEL has distinctly different structure from wool, hair and feather, increasing concentration of CEL in the composite from 10% to 20% and 35% leads to increase in the roughness of the composites. Again, as expected, for the same composition, the roughness is highest for the feather:CEL composite followed by hair:CEL composite with the wool:CEL composite has the lowest roughness structure.

Mechanical properties. It is known that KER can encapsulate and control release of drugs.[26] However, its poor mechanical properties continue to hamper its potential applications. For example, as previously reported and also observed in this study, regenerated KER film was found to be too brittle to be reasonably used in any application (Hill et al., 2010; Sando et al., 2010; Vasconcelos et al., 2013; Verma et al., 2008). Since CEL is known to possess superior mechanical strength, it is possible enhance the mechanical property of KER-based composite by adding CEL into it. Accordingly, CEL was added to either wool, hair or feather to prepare [Wool+CEL], [Hair+CEL] and [Feather+CEL] composites with different concentrations. In Figure 5, the tensile strength of the composites was plotted as a function of cellulose content. As expected, adding CEL to either wool, hair or feather substantially increases the tensile strength of the composites. For example, the tensile strength of 80:20 Feather:CEL composite (dashed-dotted curve) increased from 19.08 MPa to 45.93 MPs or ~2.5X when CEL loading was increased from 20% to 35% . Up to a 5X increase was observed when CEL loading was increased to 60% (i.e., 94.66 MPa). The same effect was also observed for [Wool+CEL] composites (dashed curve) and [Hair+CEL] composites (dotted curve) as well. Interestingly, enhancement effect induced by CEL is highest for [Feather+CEL] composites and lowest for [Wool+CEL] composites. This may be due to the effect CEL has on the secondary structure of KER in feather, hair and wool. As described in previous section, X-ray diffraction results indicate that for the same CEL loading, the α-helix content is highest for [wool+CEL] composites followed by [Hair+CEL] composites with [Feather+CEL] composites have the lowest content. That is, the interactions between CEL and feather are strongest whereas the weakest is between CEL and wool. KER can, therefore retain relatively less secondary structure or less α-helix content in the [Feather+CEL] composites compared to [Wool+CEL] and [Hair+CEL] composites. Since CEL can interact stronger with feather, it would impart more mechanical strength to feather than to wool or hair. Consequently, [Feather+CEL] composites have stronger mechanical strength than [Hair+CEL], and [Wool+CEL] composites have the weakest mechanical strength.

Antibacterial assays. Experiments were then to carry out to determine the composites have any effect on selected gram negative (*E. coli, P. aeruginosa*) and gram positive bacteria (*S. aureus,* MRSA, VRE). Different types of composites ([Hair+CEL], [Feather+CEL] and [Wool+CEL]) with different concentrations (40:60, 65:35, 75:25 and 80:20 of either wool, hair or feather and CEL) were evaluated by growing the bacteria in the presence of the composites for 24 hours and then plated out onto nutrient agar plates. The number of colonies formed after overnight incubation was compared to a standard growth control. Results obtained, plotted as Microbial Log Removal are shown in Figure 6A-D for *E. coli, S. aureus,* MRSA and VRE. It is evident from Fig 6A, B and D, that within experimental errors, all three composites ([CEL+Hair], [CEL+Feather] and [CEL+wool] ) did not inhibit any observable antimicrobial activity against *E. coli, S. aureus* and VRE. Interestingly, all three composites did show some antibacterial activity against MRSA, and the antimicrobial activity is dependent not only the on the type of the protein but also on its relative concentration as well. For examples, the 65:35 Wool:CEL exhibited very small if any effect whereas the 65:35 Feather:CEL did show substantially strong antimicrobial effect against MRSA. Hair:CEL composites seem to have relatively stronger effect than wool but weaker than feather, namely, at 80% protein content, the [Hair:CEL] exhibit somewhat stronger than that by 80:20 Wool:CEL but still much weaker than that of 80:20 Feather:CEL. Together, the results seem to indicate that similar to our previous work on the [CEL+chitosan] composites , CEL does not have any antimicrobial activity at all (Harkins et al., 2014; Tran et al., 2013a). The antibacterial property is due only to protein but also to the specific type of the keratin as well. That is, the bactericidal effect is strongest for feather followed by hair and the weakest is for wool. Taken together the antimicrobial effect and the secondary structure results presented in the previous section, suggest that feather with its highest content of random structure (i.e., lowest α-helix content) can readily interact with MRSA which enable it to exhibit strongest antimicrobial activity. Conversely, wool with its highest α-helix content, has relatively more defined structure which somewhat restricts its ability to interact with bacteria. As a consequence, it has the lowest antimicrobial activity. Hair with its structure in the middle of feather and wool, has the middle range of antimicrobial effect.

## Discussion

In summary, we have shown that composites between CEL and keratin from three different sources (wool, hair and feather) were successfully and readily synthesized in a simple one-step process in which [$BMIm^+Cl^-$], an ionic liquid, was used as the sole solvent.

The method is green and recyclable because majority of [$BMIm^+Cl^-$] used was recovered for reuse. Results of spectroscopy (FTIR, XRD) and imaging (SEM) measurements confirm that CEL and KER (from all three sources: wool, hair and chicken feather) remain chemically intact and homogeneously distributed in the composites. KER also retains some of its secondary structure in the composites. Interestingly, the minor differences in the compositions of KER in wool, hair and feather magnifies into pronounced differences in the structure of wool, hair and feather and their corresponding composites with wool has the highest content of α-helix, followed by hair and feather has the lowest content. These results correlate well with SEM results and properties (mechanical and antimicrobial properties) of the composites. Specifically, adding CEL into KER substantially improves mechanical strength of all three composites ([CEL+wool], [CEL+hair] and [CEL+feather]. Since mechanical strength is due to CEL, and CEL has only random structure, [CEL+feather] has, expectedly, the strongest mechanical property because feather has the lowest content of α-helix. Conversely, [CEL+wool] composite has the weakest mechanical strength because wool has the highest α-helix content. All three composites, [Feather+CEL], [Hair+CEL] and [Wool+CEL] were found to exhibit antibacterial activity against MRSA. The antibacterial property is due not to CEL but rather to the protein and is strongly dependent on the type of the keratin. That is, the bactericidal effect is strongest for feather followed by hair and the weakest is for wool. For example, up to 1.5 log and 1.75 logs of reduction of MRSA growth were observed in the presence of 80:20 Wool:CEL and Hair:CEL composites, respectively. Remarkably, the Feather:CEL composite with the same composition exhibits up to 5 log of reduction for growth of MRSA. These results together with our previous finding that [CEL+KER] composites can be used for drug delivery as the kinetics of the release can be controlled by adjusting the concentration of wool keratin in the composite (Mututuvari & Tran, 2014), clearly indicate that the composites can be used as dressing to treat ulcerous wounds. Moreover, the research reported here also has profound beneficial effect on the environment as it provide a facile, green and recyclable method to readily convert otherwise polluted substances such as wool (waste product from textile industry), hair and chicken feather into biocompatible and useful materials for water purification and wound healing.

References

Appelbaum, P. C. (2007). Microbiology of antibiotic resistance in Staphylococcus aureus. Clinical Infectious Diseases, 45(Suppl. 3), S165-S170.

Chen, J., Vongsanga, K., Wang, X., & Byrne, N. (2014). What happens during natural protein fibre dissolution in ionic liquids. Material, 7, 6158-6168.

Cilurzo, C., Selmin, F., Aluigi, A., & Bellosta, S. (2013). Regenerated keratin proteins as potential biomaterial for drug delivery. Polymers for Advance Technologies, 24, 1025-1028.

Cui, L., Gong, J., Fan, X., Wang, P., Wang, Q., & Qiu, Y. (2013). Trans glutaminase-modified wool keratin film and its potential application in tissue engineering. Engineering in Life Sciences, 13, 149-155.

Dullaart, R., & Mousquès, J. (Eds.). (2012). Keratin: structure, properties, and applications. In. Hauppauge, N.Y: Nova Science Publishers.

Duri, S., & Tran, C. D. (2013). Supramolecular composite materials from cellulose, chitosan and cyclodextrin: facile preparation and their selective inclusion complex formation with endocrine disruptors. Langmuir, 29, 5037-5049.

Duri, S., Majoni, S., Hossenlopp, J. M., & Tran, C. D. (2010). Determination of chemical homogeneity of fire retardant polymeric nanocomposite materials by near-infrared multispectral imaging microscopy. Analytical Letters, 43, 1780-1789.

Greve, T. M., Andersen, K. B., & Nielsen, O. F. (2008). Penetration mechanism of dimethyl sulfoxide in human and pig ear skin: an ATR-FTIR and near-FT Raman

Spectroscopic in vivo and in vitro study. Spectroscopy, 22, 405-417. Harkins, A. L., Duri, S., Kloth, L. C., & Tran, C. D. (2014). Chitosan-cellulose composite for wound dressing material. Part 2. Antimicrobial activity, blood absorption ability, and biocompatibility. Journal of Biomedical Materials Research Part B, 102, 1199-1206.

Haverhals, L. M., Reichert, W. M., Nazare, N., Zammarano, M., Gilman, J. W., De Long, H. C., et al. (2012). Ionic liquid facilitated introduction of functional materials into biopolymer polymer substrates, in molten salts and ionic liquids 18. ECS Transactions, Vol. 50(11), 631-640.

Hill, P., Brantley, H., & Van Dyke, M. (2010). Some properties of keratin biomaterials: kerateines. Biomaterials, 1, 585-593.

Jorgensen, J. H., Ferraro, M. J., Jorgensen, J. H., & Ferraro, M. J. (2009). Antimicrobial susceptibility testing: a review of general principles and contemporary practices. Clinical Infectious Diseases, 49(11), 1749-1755.

Justin, M., Saul, M. D., Ellenburg, R., de Guzman, C., & Van Dyke, M. (2011). Keratin hydrogels support the sustained release of bioactive ciprofloxacin. Journal of Biomedical Materials Research Part A, 98(A), 544-553.

McKittrick, J., Chen, P. Y., Bodde, S. G., Yang, W., Novitskaya, E. E., & Meyers, M. A. (2012). The structure, functions, and mechanical properties of keratin. JOM, 64, 449-468.

Mututuvari, T. M., & Tran, C. D. (2014). Synergistic adsorption of heavy metal ions and organic pollutants by polysaccharide supramolecular composite materials from cellulose, chitosan and crown ether. Journal of Hazardous Materials, 264, 449-459.

Mututuvari, T. M., Harkins, A. L., & Tran, C. D. (2013). Facile synthesis, characterization and antimicrobial activity of cellulose-Chitosan-Hydroxy apatite composite material, a potential material for bone tissue engineering. Journal of Biomedical Materials Research Part A, 101(11), 3266-3277.

Sando, L., Kim, M., Colgrave, M. L., Ramshaw, J. A., Werkmeister, J. A., & Elvin, C. M. (2010). Photochemical crosslinking of soluble wool keratins produces a mechanically stable biomaterial that supports cell adhesion and proliferation. Journal of Biomedical Materials Research Part A, 95, 901-911.

Sowa, M. G., Wang, J., Schultz, C. P., Ahmed, M. K., & Mantsch, H. H. (1995). Infrared spectroscopic investigation of in vivo and ex vivo human nails. Vibrational Spectroscopy, 10, 49-56.

Tanabe, T., Okitsu, N., Tachibana, A., & Yamauchi, K. (2002). Preparation and characterization of keratin-chitosan composite film. Biomaterials, 23, 817-825.

Tran, C. D., & Mututuvari, T. M. (2015). Cellulose, chitosan and keratin composite materials controlled drug release. Langmuir, 31, 1516-1526.

Tran, C. D., Duri, S., & Harkins, A. L. (2013). Recyclable synthesis, characterization, and antimicrobial activity of chitosan-based polysaccharide composite materials. Journal of Biomedical Materials Research Part A, 101, 2248-2257.

Tran, C. D., Duri, S., Delneri, A., & Franko, M. (2013). Chitosan-cellulose composite materials: preparation, characterization and application for removal of microcystin. Journal of Hazardous Materials, 252, 355-366.

Vasconcelos, A., & Cavaco-Paulo, A. (2013). The use of keratin in biomedical applications. Current Drug Targets, 14, 612-619.

Verma, V., Verma, P., & Ray, A. R. (2008). Preparation of scaffolds from human hair proteins for tissue-engineering applications. Biomedical Materials, 3, 2500.

Vilaplana, F., Stroemberg, E., & Karlsson, S. (2010). Environmental and resource aspects of sustainable biocomposites. Polymer Degradation and Stability, 95(11), 2147-2161.

Xie, H., Li, S., & Zhang, S. (2005). Ionic liquids as novel solvents for the dissolution and blending of wool keratin fibers. Green Chemistry, 7, 606-608.

Example 2 - One-Pot Synthesis of Biocompatible Silver Nanoparticle Composites from Cellulose and Keratin: Characterization and Antimicrobial Activity

Reference is made to Tran et al., "One-Pot Synthesis of Biocompatible Silver Nanoparticle Composites from Cellulose and Keratin: Characterization and Antimicrobial Activity," Applied Materials & Interfaces, 2016, 8, 34791-34801.

Abstract

A novel, simple method was developed to synthesize biocompatible composites containing 50% cellulose (CEL) and 50% keratin (KER) and silver in the form of either ionic ($Ag^+$) or $Ag^0$ nanoparticle ($Ag^+$NPs or $Ag^0$NPs). In this method, butylmethylimmidazolium chloride ([$BMIm^+Cl^-$]), a simple ionic liquid, was used as the sole solvent and silver chloride was added to the [$BMIm^+Cl^-$] solution of [CEL+KER] during the dissolution process. The silver in the composites can be maintained as ionic silver ($Ag^+$) or completely converted to metallic silver ($Ag^0$) by reducing it with $NaBH_4$ Results of spectroscopy (Fourier-transform infrared (FTIR), X-ray diffraction (XRD)) and imaging (scanning electron microscope (SEM)) measurements confirm that CEL and KER remain chemically intact and homogeneously distributed in the composites. Powder X-ray diffraction (XRD) and SEM results show that the silver in the [CEL+KER+$Ag^+$] and [CEL+KER+$Ag^0$] composites is homogeneously distributed throughout the composites in either $Ag^+$ (in the form of $Ag_2O$ nanoparticles (NPs)) or $Ag^0$NPs form with size of (9 ± 1) nm or (27 ± 2) nm, respectively. Both composites were found to exhibit excellent antibacterial activity against many bacteria including *Escherichia coli, Staphylococus aureus, Pseudomonas aeruginosa,* methicillin resistant *Staphylococus aureus* (MRSA), vancomycin resistant *Enterococus faecalis* (VRE). The antibacterial activity of both composites increases with the $Ag^+$ or $Ag^0$ content in the composites. More importantly, for the same bacteria and the same silver content, [CEL+KER+$Ag^0$] composite exhibits relatively greater

antimicrobial activity against bacteria compared to the corresponding [CEL+KER+$Ag^+$] composite. Experimental results confirm that there was hardly any $Ag^0$NPs release from the [CEL+KER+$Ag^0$NPs] composite, and hence its antimicrobial activity and biocompatibility is due, not to any released $Ag^0$NPs but rather entirely to the $Ag^0$NPs embedded in the composite. Both of $Ag_2O$NPs and $Ag^0$NPs were found to be toxic to human fibroblasts at higher concentration (>0.72 mmol), and that for the same silver content, [CEL+KER+$Ag_2O$NPs] composite is relatively more toxic than [CEL+KER+$Ag^0$NPs] composite. As expected, by lowering the $Ag^0$NPs concentration to 0.48 mmol or less, the [CEL+KER+$Ag^0$NPs] composite can be made biocompatible while still retaining its antimicrobial activity against bacteria such are *E. coli, S. aureus, P. aeruginosa,* MRSA, VRE. These results together with our previous finding that [CEL+KER] composites can be used for controlled delivery of drugs such as ciprofloxacin clearly indicate that the [CEL+KER+$Ag^0$NPs] composite possess all required properties for successfully used as high performance dressing to treat chronic ulcerous infected wounds.

Introduction

Interest in nanoparticles particularly silver nanoparticles (AgNPs) has increased significantly recent years because, among other unique features, the NPs are known to exhibit both antimicrobial and antiviral activities.[1-8] It has been shown that AgNPs exhibit highly antimicrobial activity against both Gram-positive and negative bacteria.[1-8] They have also shown to be effective antiviral agent.[1-9] The size, morphology and stability of NPs are known to strongly affect their antimicrobial and antiviral activity.[1-8] Colloidal NPs are known to undergo coagulation and aggregation in solution, which, in turn, lead to changes in their size and morphology and hence their antibacterial and antiviral properties. It is, therefore, important to develop an effective and reliable method to anchor the NPs into a supporting material in order to prevent their coagulation and aggregation so that they can maintain their activity. In fact, AgNPs have been encapsulated in various man-made polymers and/or biopolymers, and such systems have been reported to retain some of their antimicrobial and antiviral activity.[1-18] For example, anchoring AgNPs onto methacrylic acid copolymer beads have proved to be highly effective against a few bacteria.[1-18] However, antimicrobial property of all reported AgNPs-encapsulated composites was tested for only very few bacteria, and more importantly, their biocompatibility has not been determined.[1-18] The lack of the latter information is critical since toxicity of AgNPs is known to be dependent on concentration, and without information on biocompatibility, application of such composite is rather limited. It is, therefore, of particular importance to develop a novel method to anchor AgNPs onto composites biopolymers such as cellulose and keratin, and thoroughly and systematically investigate the antimicrobial and biocompatibility of the composites.

Keratins (KER) are a group of cysteine-rich fibrous proteins found in filamentous or hard structures such as hairs, wools, feathers, nails and horns.[19-28] KER possess amino acid sequences similar to those found on extracellular matrix (ECM), and since ECM is known to interact with integrins which enable it to support cellular attachment, proliferation and migration, KER-based materials are expected to have such properties as well.[19-28] Furthermore, KER is known to possess advantages for wound care, tissue reconstruction, cell seeding and diffusion, and drug delivery.[11-20] Unfortunately, in spite of its unique properties, KER has relatively poor mechanical properties, and as a consequence, it was not possible to fully exploit unique properties of keratin for various applications.[19-28] To increase the structural strength of KER-based materials, attempts have been made to crosslink KER chains with a crosslinking agent or introduce functional groups on its amino acid residues via chemical reaction(s).[19-28] The rather complicated, costly and multistep process is not desirable as it may inadvertently alter its unique properties, making the KER-based materials less biocompatible and toxic, and removing or lessening its unique properties. A new method which can improve the structural strength of KER-based products not by synthetic methods rather by use of naturally occurring polysaccharides such as CEL, is particularly needed.

We have demonstrated recently that a simple ionic liquid (IL), butylmethylimmidazolium chloride ([$BMIm^+Cl^-$]), can dissolve both cellulose (CEL) and KER and by use of this IL as the sole solvent, we developed a simple, GREEN and totally recyclable method to synthesize [CEL+KER] composites just by dissolution without using any chemical modifications or reactions.[29-35] Spectroscopy (FTIR, NIR, $^{13}$C CP-MAS-NMR) results indicate that there was no chemical alteration in the structure of CEL and KER.[29-35] While there may be some changes in the molecular weights of CEL and KER, by use of newly developed partial least square regression to analyze FTIR spectra of the [CEL+KER] composites, we found that KER retains some of it secondary structure in the composites.[31,35] The [CEL+KER] composites obtained were found to retain unique properties of their components, namely, superior mechanical strength from CEL and controlled release of drugs by KER.[29-35]

The information presented clearly indicates that it is possible to use [CEL+KER] as a biocompatible composite to encapsulate AgNPs. Such considerations prompted us to initiate this study which aims to hasten the breakthrough by systematically exploiting advantages of ILs, a green solvent, to develop a novel, simple method to synthesize the [CEL+KER] composite containing silver in either $Ag^+$ or $Ag^0$ forms. As will be demonstrated, by initially introducing silver salt into the [CEL+KER] composite during the dissolution of CEL and KER by

[$BMIm^+Cl^-$], and subsequently reducing the $Ag^+$ into $Ag^0$ NPs directly in the composite, we successfully synthesize the [CEL+KER+$Ag^0$NPs] composite. Alternatively, by not carrying out the reduction reaction, we can obtain the [CEL+KER+$Ag^+$NPs] composite. Because the [CEL+KER+$Ag^0$NPs] and [CEL+KER+$Ag^+$NPs] composites obtained can prevent the $Ag^+$NPs and $Ag^0$NPs from changing size and morphology as well as undergo coagulation, they can, therefore, fully retain the unique property of the silver nanoparticles for repeated use without any complication of reducing activity and not fully recover after each use. With these two composites, we will be able to finally address the important question which, to date, still remains unanswered, namely, the antimicrobial activity of silver nanoparticles due to either $Ag^+$ or $Ag^0$ or both, and if both forms are active, which NPs have higher activity. We will also systematically investigate biocompatibility of the two composites; information obtained will be used to guide selection and use of the nanoparticle composites. The synthesis, characterization, antimicrobial activity and biocompatibility of the [CEL+KER+$Ag^+$NPs] and [CEL+KER+$Ag^0$NPs] composites are reported herein.

Experimental Section

Chemicals. Microcrystalline cellulose (DP≈300) and $AgCl_2$ were from Sigma-Aldrich and used as received. Raw (untreated) sheep wool, obtained from a local farm, was cleaned by Soxhlet extraction using a 1:1 (v/v) acetone/ethanol mixture at 80±3 °C for 48 h. The wool was then rinsed with distilled water and dried at 100±1 °C for 12 h.[30-32] Methylimidazole and n-chlorobutane (both from Alfa Aesar, Ward Hill, MA) were distilled and subsequently used to synthesize [$BMIm^+Cl^-$] using method previously reported.[19-35] Nutrient broth (NB) and nutrient agar (NA) were obtained from VWR (Radnor, PA). Minimal essential medium (MEM), Fetal Bovine Serum (FBS), and Penicillin-Streptomycin were obtained from Sigma-Aldrich (St. Louis, MO), whereas Dulbecco's Modified Eagle Medium (DMEM), PBS, trypsin solution (Gibco) were obtained from Thermo Fischer Scientific (Waltham, MA). CellTiter 96® AQueous One Solution Cell Proliferation Assay was obtained from Promega (Madison, WI).

Bacterial and Cell Cultures. The bacterial cultures used were either obtained from the American Type Culture Collection (ATCC, Rockville, MD) or from the Leibniz Institute DSMZ - German Collection of Microorganisms and Cell Cultures (Braunschweig, Germany). The cell cultures of human fibroblasts were obtained from ATTC (Rockville, MD).

Synthesis. [CEL+KER+$Ag^+$NPs] and [CEL+KER+$Ag^0$NPs] composites were synthesized with minor modification to the procedure we developed previously for the synthesis of [CEL+CS+KER] composites.[30-32,35] As shown in Scheme 1, washed wool was dissolved in $BMIm^+Cl^-$ at 120 °C. Once dissolved, the solution temperature was reduced to 90 °C before CEL was added to the KER solution. Using this procedure, [$BMIm^+Cl^-$] solution of CEL and KER containing up to total concentration of 6 wt% (relative to IL) with various compositions and concentrations were prepared. Concurrently, in a separate flash, AgCl was dissolved in 2 mL of [$BMIm^+Cl^-$], and the mixture will then be added dropped wise to the $BMIm^+Cl^-$ solution of [CEL+KER]. The resulted solution was then casted onto PTFE molds with desired thickness on Mylar films to produce thin composite films with different compositions and concentrations of CEL, KER and $Ag^+$. They were then kept in the dark and at room temperature for 24 hrs to allow gelation to yield Gel Films. The $Ag^+$ doped Gel Film was then washed with water for 3 days to remove $BMIm^+Cl^-$, and then dried slowly (3-5 days), in the dark at room temperature in a humidity controlled chamber to yield [CEL+KER+$Ag^+$NPs] composite. Alternatively, the $Ag^+$ doped Gel Film was reduced with $NaBH_4$ to $Ag^0$NPs. For example, the Gel Film, sandwiched between two PTFE meshes, was placed in an aqueous solution of either $NaBH_4$, in the dark and at room temperature for 48hrs. Subsequently, the reduced film was washed and dried slowly (~3-5 days) in the dark and at room temperature in a humidity-controlled chamber to yield [CEL+KER+$Ag^0$NPs] composite.

Analytical Characterization. FTIR spectra (from 450-4,000 $cm^{-1}$ were recorded on a Spectrum 100 Series FTIR spectrometer (Perkin Elmer, USA) at resolution of 2 $cm^{-1}$ by the KBr method. Each spectrum was an average of 64 individual spectra. X-ray diffraction (XRD) measurements were taken on a Rigaku MiniFlex II diffractometer utilizing the Ni filtered Cu Kα radiation (1.54059Å). The voltage and current of the X-ray tube were 30kV and 15mA respectively. The samples were measured within the 2θ angle range from 2.0 to 40.00. The scan rate was $5^0$ per minute. Data processing procedures were performed with the Jade 8 program package.[29-35] The surface and cross-sectional morphologies of the composite films were examined under vacuum with a JEOL JSM-6510LV/LGS Scanning Electron Microscope with standard secondary electron (SEI) and backscatter electron (BEI) detectors. Prior to SEM examination, the film specimens were made conductive by applying a 20 nm gold-palladium-coating onto their surfaces using an Emitech K575x Peltier Cooled Sputter Coater (Emitech Products, TX).

In Vitro Antibacterial Assays. The antibacterial characteristics of the newly synthesized composites were tested against *E. coli* (ATCC 8739, DSMZ 498), *Staphylococcus aureus* (ATCC 25923, DSMZ 1104), methicillin resistant S. *aureus* (ATCC 33591, DSMZ 11729), vancomycin resistant *Enterococcus faecalis* (ATCC 51299, DSMZ 12956), and *Pseudomonas aeruginosa* (ATCC 9027, DSMZ 1128) using previously published protocol.[29,33,34] The cultures were grown in a sterile nutrient broth medium overnight at 37°C and 150 rpm. Composites of dimensions of 3 × 20 mm were prior to the assay thermally sterilized at 121°C, 15 psi for

20 min. They were placed in a diluted overnight culture (2 $\mu$L of overnight culture in 2 mL of nutrient broth) and incubated for 24 hours at 37°C and 200 rpm. Bacteria were plated in serial dilutions onto sterile nutrient agar plates at time 0 and after 24 hours, and incubated overnight at 37°C. Colony forming units (CFUs) were quantified on statistically significant plates (30 - 300 CFUs) and compared to a control (no added material). Log of reduction of bacteria as follows was calculated for each experiment:

$$Log\ of\ reduction = \log \frac{N_0}{N_t}$$

where $N_0$ is the number of bacteria at the beginning of the experiment, and $N_t$ is the number of bacteria after 24 hours.

In Vitro Biocompatibility Assays. The biocompatibility of [AgNPsCEL:KER] composites was assessed by the adherence and growth of fibroblasts in the presence of the composites, as previously reported.[29,33,34] Human fibroblasts (ATCC CRL-2522 or ATCC CCL-186) were grown in a sterile minimal essential medium (MEM) or in a sterile Dulbecco's Modified Eagle Medium (DMEM) supplemented with 10% FBS and 1% Penicillin-Streptomycin according to ATCC guidelines. The inoculated culture was grown at 37°C in a humidified atmosphere of 5% $CO_2$ until the 3rd passage. Between passages cells at approximately 80% confluency were subjected to trypsinization and recovered by centrifugation at 1000 g for 10 min. The cell pellets were resuspended homogenously into the culture media and transferred into a 75 $cm^2$ tissue culture flask for further passages. Cells were seeded into the wells of the 24-well plate at a concentration of $2 \times 10^4$ cells/mL and left for 1 day to allow for their attachment (approximately 50% confluency). Circle-shaped composites with either 15 or 7 mm in diameter were autoclaved at 121°C, 15 psi for 20 min and placed into the wells with attached cells the following day. Some wells contained cells without any added material and served as a control. After incubating for 3 days, viability and fitness of the cells was evaluated both, with a colorimetric CellTiter 96® AQueous One Solution Cell Proliferation Assay, and visually with an Olympus DP12 digital microscope camera and. The procedure for the CellTiter 96® AQueous One Solution Cell Proliferation Assay was followed as specified in the manufacturer's manual. In brief, the MTS reagent was added in a 1:5 ratio to each well after the medium in wells was supplemented with a colorless MEM or colorless DMEM. The cells were then incubated at standard culture conditions for 3 h. Then 100 $\mu$L from each well was transferred to a new 96-well cell culture plate and optical density (OD value) of the extracted supernatant was measured with a Perkin Elmer HTS 7000 Bio Assay Reader at 490 nm. The percent viability was calculated using the following equation:

$$\%\ cell\ viability = \frac{OD_{Test\ sample}}{OD_{Control}} \times 100$$

where $OD_{Test\ sample}$ is the measured OD at 490 nm of the extract from the test sample well, and $OD_{Control}$ is the measured OD at 490 nm of the extract from the control well.

Measurements of $A_2^0$NPs released from [CEL+KER+ $A_2^0$NPs] Composites by Thermal Lens Method.+ with sodium borohydride, there is a remote possibility that some minute amount of $Ag^+$ may remained unreduced and remained in the composites (even though XRD results indicate that no $Ag^+$ is present in the composite) which was subsequently released. Because this thermal lens detection technique cannot detect any released $Ag^+$ as it does not have any surface plasmon resonance absorption, any released $Ag^+$ was converted into AgNPs by sodium borohydride directly by use of the FIA so that they can be readily detected. As a consequence, results obtained will provide information on two concentrations: colloidal silver concentration or (concentration of released AgNPs) and total silver concentration which is the sum of released AgNPs concentration plus released $Ag^+$ concentration.

The experimental setup to measure silver release mirrored the experimental setup used in bioassays. Composite materials of dimensions 3 x 20 $mm^2$ were put in sterile falcon tubes with 2 mL of sterile 1x PBS at pH 7.4. Three replicates each of blank samples ([CEL+KER]) and [CEL+KER + 500 mg $Ag^0$ NPs] composites were used. Tubes were put on a shaker at 400 rpm and kept at 37°C in darkness for 7 days. Samplings were conducted at time 0, 24 hrs, 3 days and 7 days. At every sampling 200 $\mu$L of sample was taken out of each tube and replaced with 200 $\mu$L of fresh PBS. The dilution was taken into account when calculating final concentrations. 100 $\mu$L of sample was reduced with 0.60 mM sodium borohydride ($NaBH_4$) in order to measure total silver (AgNPs + $Ag^+$), whereas the other 100 $\mu$L of sample was not reduced in order to measure only colloidal silver (AgNPs) released from the sample. Sample preparation was done as shown in Figure 14.

Sample preparation was done in glass tubes wrapped in aluminum foil to protect it from light. Dilution made at sample preparation was taken into account when calculating measured concentrations.

All measurements were conducted on an in-house-built FIA system with a dual beam TLS detection unit.[51,52] The instrumental setup is schematically presented in Figure 15. Krypton laser operating at 407 nm (150 mW power) was used as a source of the pump-beam. The emission of a He-Ne laser (632.8 nm, 2 mW) served as a probe beam. The pump-beam modulation frequency was 40 Hz. Flow rate of the carrier (dd $H_2O$) was 0.600 mL/min.

Sample was injected through the metal free injection valve, equipped with a 100 $\mu$L PEEK sample

loop. Separate calibration curve was prepared every time a set of samples was measured. Limit of detection (LOD) for this method was calculated as follows:

$$LOD = \frac{3 \cdot SD_{blank}}{k}$$

where $SD_{blank}$ corresponds to standard deviation of blank signal, and k is the slope of the calibration curve. To further confirm that the signals obtained are from the Ag0NPs released from the [CEL+KER + Ag°NPs] composites, additional experiment was designed in which nitric acid ($HNO_3$) was added to the released sample solution to dissolve the released $Ag^0$NPs. Specifically, 2.0 μL of concentrated $HNO_3$ was added to 6 mL of released sample to dissolve the $Ag^0$NPs. The $Ag^+$ obtained was then reconverted back to $Ag^0$NPs by addition of 6.0 mL PBS (pH 12.5) and 600 μL 0.6 mM $NaBH_4$ to 6 mL of dissolved sample. Samples at each stage of the experiment (before dissolution, after dissolution, and after recovery) were measured on the FIAthermal lens setup described above using the same conditions.

Statistical Analysis. All experiments had sample size of n = 3 and are representative of repeated trials. Sample error bars on plots represent ± standard error of mean (SEM), where applicable. Tests for statistical significance of the difference of the means were performed using a two-tailed Student's t-test assuming unequal variances using Microsoft Office Excel. P-values are indicated as follows on figures: (*P < 0.05); (**P < 0.005); (***P < 0.001).

## Results and Discussion

FTIR. FTIR spectrum of the [CEL+KER+$Ag^0$NPs] composite is presented as the orange spectrum in Figure 8. For reference, spectrum of the [CEL+KER] composite is also added (blue spectrum). As expected, the blue spectrum of the [CEL+KER] is similar to those previously observed for the [CEL+KER] composites, namely bands at 1700-1600 $cm^{-1}$ and 1550 $cm^{-1}$ are due to amide C=O stretch (amide I) and C-N stretch (amide II) vibrations, and at 1300-1200 $cm^{-1}$ are from the in-phase combination of the N-H bending and the C-N stretch vibrations (amide III).[30-32,36-33,] Major bands between 1200- and 900- $cm^{-1}$ are due to sugar ring deformations of the CEL.[30-32,36-38] The fact that the orange spectrum of the [CEL+KER+$Ag^0$NPs] composite is relatively similar to the green spectrum of the [CEL+KER] composite seems to indicate that there may not be strong interaction between the $Ag^0$NPs and CEL and KER in the composite. However, careful inspection of the spectra revealed that there are indeed minor differences in the amide bands at around 1700-1600 $cm^{-1}$ and 1550 $cm^{-1}$ between the two spectra. Specifically, interaction between $Ag^0$NP and C=O group leads to the shift in the amide band at 1650 $cm^{-1}$ (of the [CEL+KER] composite) to 1655 $cm^{-1}$ (of the [CEL+KER+ $Ag^0$NP] composite). Also, the small shoulder at ~1449 $cm^{-1}$disappers upon adding $Ag^0$NP to the composite. These results seem to indicate that there may be some interactions between the $Ag^0$NP and the amide groups of the KER. Furthermore, difference of the band at ~2870 $cm^{-1}$ between the spectra of the two composites suggests that there may be some modifications in the hydrogen bonding when $Ag^0$NP was incorporated into the [CEL+KER] composite.[30-32]

Powder X-ray Diffraction (XRD). X-ray diffractograms of [CEL+KER+$Ag^+$NPs] and [CEL+KER+$Ag^0$NPs] composites are shown in Figure 9. Because CEL and KER are present in both composites, it is as expected that both spectra have similar two broad bands at around $2\theta=0.75^0$ and 20.85° which are due to CEL and KER. Since the valency of the silver nanoparticles is different in the composites, narrow crystalline bands which are due to the silver nanoparticles are distinctly different for the two composites. Specifically, the diffractogram of [CEL+KER+$Ag^+$NPs] composite (blue spectrum) exhibits three major peaks at $(2\theta)$= $27.94^0$, 32.35° and 46.37° which are characteristic of the (1 1 0), (1 1 1) and (2 1 1) peaks, respectively, of silver oxide nanoparticles ($Ag_2$ONPs).[39-43] The fact that these peaks are the same as those previously reported for $Ag_2O$ NPs [40-43] as well as the reference diffractogram of $Ag_2O$ reported in the JCPDS file No 42-0874 seems to indicate that $Ag^+$ reacted with oxygen to form $Ag_2O$ following by aggregation to form $Ag_2$ONPs. Conversely, the diffraction peaks at 38.47°, 44.57°, 64.87° and 77.66° in the orange spectrum of the [CEL+KER+$Ag^0$NPs] composite can be attributed to the (1 1 1), (2 0 0), (2 2 0) and (3 1 1) bands of $Ag^0$. [44-46] The fact that there is no diffraction peak of $Ag^0$ in the [CEL+KER+$Ag^+$NPs] suggests that this composite contains only silver oxide nanoparticles. Similarly, since there is no peak due to $Ag_2$ONPs is seen in the diffractogram of the [CEL+KER+Ag°NPs] composite, it is reasonable to infer that silver ion was completely reduced to metallic silver nanoparticles during the synthesis.

Scherrer equation was then used to determine the size (τ value) of the $Ag_2$ONPs and $Ag^0$NPs in the composites from the full width at half maximum (FWHM, β value in the equation) of their corresponding XRD peaks:[47,48]

$$\tau = \frac{k\lambda}{\beta \cos\theta}$$

where τ is the size of the nanoparticle, λ is the X-ray wavelength and k is a constant.[31,32] The size of the metallic silver nanoparticle in the [CEL+KER+$Ag^0$] composite was found to be (9 ± 1) nm while the $Ag_2$ONPs in the [CEL+KER+$Ag^+$] composite has the size of (27 ± 2) nm. It is unclear why the size of the silver oxide is much larger than that of the metallic silver NPs. It may be

possible that the stirring and reducing with $NaBH_4$ further dispersed the silver ion NPs in the [CEL+KER] composite thereby preventing them from coagulation upon reducing to $Ag^0$NPs.

Scanning Electron Microscope (SEM) Images and Energy Disperse Spectroscopy (EDS) Analysis. Shown in Figure 10A are surface (left) and cross section SEM images of the [CEL+KER+$Ag^0$NPs] composite. As expected, the images of the composite are similar to those we previously observed for the [CEL+KER] composites.[30-32] That is CEL and KER are homogeneously distributed throughout the composite. While CEL is known to have rather smooth structure, the presence KER in the composite gives it a rough and porous structure with a three-dimensional interconnection throughout the film. More information on the chemical composition and homogeneity of the composite can be seen in Figure 10B and 10C which show the EDS spectrum of the composite (3B) and images taken with EDS detector specifically set for carbon (3C left), silver (3C center) and oxygen (3C right). As evident from Figure 10C, the silver nanoparticles were not only well incorporated into the composites, but were also present as well distributed nanoparticles throughout the composite.

Antibacterial Assay. To assess the antimicrobial effect of AgNPs in the [CEL+KER+AgNPs] composites, bacteria were grown in the presence of the composites and then plated out onto nutrient agar and measured by the number of colonies formed compared to those for the blank ([CEL+KER] composite) and the control (no composite). Results for the microbial log of reduction of different composites are shown in both Figure 11 top (for composites with 3.5 mmol of either $Ag^+$ or $Ag^0$) and bottom A-E (for NPs with three different concentrations: 3.5 mmol, 0.72 mmol and 0.48 mmol). It is evident that bactericidal activity of [CEL+KER+AgNPs] composites increases with the concentration of silver NPs in both $Ag^0$ and $Ag^+$ forms for all bacteria tested. Specifically, as shown in Figure 11 top, [CEL+KER+$Ag^0$] composites (black bar) with 3.5 mmol of silver exhibited the highest bactericidal activity against all selected bacteria with up to 6-logs of reduction in number of bacteria, which corresponds to 99.9999% growth reduction. Even at silver concentration as low as 0.48 mmol, the composite still exhibited up to 0.5-logs of reduction, or 68% growth reduction for most of bacteria, with the exception of VRE, where 1-log of reduction was observed (Figure 1 bottom A-E). As expected, controls and blank samples (light grey bars) did not exhibit any statistical significantly reduction in number of bacteria, and there was no significant difference between them.

While it is known that AgNPs are bactericidal, to date, it is still unclear if the antimicrobial activity is due to $Ag^0$ or $Ag^+$ (as in $Ag_2O$). As described above, by judiciously selecting the synthetic method, the [CEL+KER+AgNPs] can be synthesized with the silver NPs in either $Ag^0$ or $Ag^+$ form. This makes it possible, for the first time, to elucidate the mechanism of antimicrobial activity of AgNPs. Accordingly, microbial assays were carried out in the presence of either [CEL+KER+ $Ag^0$NPs] composites (black bars) or [CEL+KER+ $Ag^+$] composites (hatched bars). Results obtained, shown in both Figures 11 top and bottom, clearly show that for the same bacteria and the same silver content, [CEL+KER+$Ag^0$] composites (black bars) exhibit relatively greater antimicrobial activity against bacteria compared to the corresponding [CEL+KER+$Ag^+$] composites (hatched bars). For example, as shown in Figures bottom 11A-D, up to 6-log of reduction of growth was found by [CEL+KER+$Ag^0$NPs] composite for all four bacteria (*E. coli, S. aureus,* MRSA and VRE) whereas [CEL+KER+ $Ag^+$] composite exhibits only 3.5-log of reduction. Surprisingly, within experimental errors, there was no significant difference between these two nanoparticle composites for *P. aeruginosa* (Figure 11E). Results obtained also indicate that [CEL+KER+$Ag^0$NPs ] composites not only have relatively stronger antimicrobial activity compared to corresponding [CEL+KER+$Ag^+$] composites, but that the rather limited antimicrobial activity of the latter cannot be enhanced by increasing the concentration of $Ag^+$ in the composites because, as will be shown in the following section, $Ag^+$ is not biocompatible and as a consequence, increasing $Ag^+$ concentration would undesirably lead to damaging and killing human cells Again, as expected, there was no statistically significant decrease in number of bacteria after 24 hours in control experiments (no composite) and blank samples.

Biocompatibility Assay. To assess a potential cytotoxicity of the [CEL+KER+AgNPs] composites with different concentrations of silver NPs, the morphology and the proliferation capabilities of adherent human fibroblasts in presence or absence of the nanoparticle composites were analyzed. The proliferation capability was assessed using a colorimetric assay CellTiter 96® AQueous Non-Radioactive Cell Proliferation Assay (or CellTiter 96® AQueous One Solution Cell Proliferation Assay), whereas the morphology of fibroblasts was examined microscopically. Three trials were performed for this assay, employing composites with different sizes (circle of either 15 or 7 mm diameter) and silver concentrations. Fibroblasts were exposed to the composites for 3 days. Proliferation and viability of fibroblasts in the presence or absence of the composites with different concentrations of AgNPs over time 3 days is shown in Figure 12. Statistical significance in differences between the sample wells and control wells were evaluated with two-tailed student's t-test, and the degree of significance is indicated with P values in different significance levels (alpha = 0.05, 0.005, or 0.001). In the first trial, the composites of 15 mm diameter and with either 3.5 mmol of $Ag^+$ or $Ag^0$ concentration were tested (Figure 12A). The fibroblasts in contact with either the 3.5 mmol [CEL+KER+$Ag^0$] or the 3.5 mmol [CEL+KER+$Ag^+$] exhibited low absorbances at 490 nm, indicating that cells were not viable. Morphological data obtained through microscopic examination indicated that the fibroblasts

in these wells were not attached and exhibit unusual round morphology (data not shown). This seems to indicate that the cells were not healthy and possibly not viable. To reduce the concentration of silver NPs in the composites, in the second trial, the diameter of composites used was reduced from 15 mm to 7 mm which corresponds to 4.6 reduction in the area of the composites. As shown in Figure 12B, cells in the sample wells exhibited slightly increased viability after 3 days compared to that in the first trial. Morphological data showed round unattached cells (data not shown). Because results obtained so far indicate that the biocompatibility of the [CEL+KER+$Ag^0$] composites are relatively better than that of the corresponding [CEL+KER+$Ag^+$] composites, subsequent experiments were carried out using only the former. Specifically, [CEL+KER+$Ag^0$] composites with relatively lower $Ag^0$NPs concentrations (0.48 mmol and 0.72 mmol) were used, (Figure 12C). In this case, the viability of cells in the composite wells after 3 days of exposure was high, approximately 83% for 0.48 mmol of $Ag^0$NPs and (64$\pm$5) % for 0.72 mmol of $Ag^0$NPs compared to control. It is evidently clear that within experimental error, there was no statistically significant difference between cells in the 0.72 mmol $Ag^0$NPs well and 0.48 mmol $Ag^0$NPs well and that in the control well. Morphological data presented as images of cells in the 0.48 mmol $Ag^0$NPs well (Figure 13C) and in the 0.72 mmol $Ag^0$NPs well (Figure 13D) show a mix of healthy-looking cells and round unattached cells, similar to those observed for cells in the absence of composite (Figure 13A) and with [CEL+KER] composite (Figure 13B). Taken together, the results clearly indicate that both $Ag^+$ and $Ag^0$NPs are toxic to human fibroblasts at higher concentration (>0.72 mmol). At the same concentration, $Ag^+$ is relatively more toxic than $Ag^0$. More importantly, at or below the silver concentration of 0.48 mmol, the [CEL+KER+$Ag^0$NPs] composite is not only fully biocompatible but also fully retains its antimicrobial activity against bacteria such as *E. coli, S. aureus, P. aeruginosa,* MRSA, VRE.

Release of AgONPs from the [CEL+KER+Ag0NPs] Composites. We also carried out experiments to determine if any $Ag^0$NPs are leaking out from the [CEL+KER+$Ag^0$NPs] composites during the microbial and biocompatibility assays. Such information is particularly important as it would clarify the mechanism of antibacterial activity and biocompatibility of the composites. That is the activity is due either to the $Ag^0$NPs in the composites and/or $Ag^0$NPs released from the composites. As described in the experimental section, since the [CEL+KER+$Ag^0$NPs] composites were exhaustedly washed with water for a total of up to 10 days, it is expected that if there is any leaking of silver NPs from the composites, their concentration should be extremely low. Accordingly, we used a modified version of the recently developed ultrasensitive method based on the thermal lens technique to determine the concentration of any possible leaking of the $Ag^0$NPs from the composites during the bioassay.[49,50] No experiment was carried out to measure release of $Ag^+$ form the [CEL+KER+$Ag^+$] composites because compared to the [CEL+KER+Ag °NPs] composites the [CEL+KER+$Ag^+$] composites are not readily usable as they are not biocompatible and have relatively lower antimicrobial activity. This thermal lens detection method is so sensitive that it can detect released silver NPs at concentration as low as 0.51 $\mu$g/L.[33] As described in the Experimental Section above, two different concentration values can be obtained from this method: colloidal silver concentration or concentration of released $Ag^0$NPs, and total silver concentration which is the sum of the released $Ag^0$NPs concentration plus released $Ag^+$ concentration. As described above, XRD results show that there is no $Ag^+$ in the [CEL+KER+$Ag^0$NPs] composites; i.e., all $Ag^+$ was reduced by $NaBH_4$ to $Ag^0$NPs during the preparation. However, there is a possibility that concentration of $Ag^+$ remained in the composites was so low that it cannot be detected by XRD. Because this thermal lens detection is so sensitive that it can detect any $Ag^+$ that is released from the $Ag^+$ remaining in the composites.

Results obtained, presented in Figure 16 and plotted as concentration of released silver against time the composites were immersed in the solution similar to the media used in the microbial and biocompatibility assays. The fact that, within experimental errors, and at all times (from the beginning to 7 days), obtained concentration of released $Ag^0$NPs (black bars) was the same as that of the total concentration of released silver (grey bars) clearly indicates that all released silver were $Ag^0$NPs, there was no $Ag^+$ released from the composites. Also, concentrations of released $Ag^0$NPs after 3 days were the same, within experimental errors, to those after 7 days indicate that no more $Ag^0$NPs was released beyond 3 days. More importantly, even after reaching a plateau at about 3 days and continued beyond 7 days, only 2.3 $\mu$g of $Ag^0$NPs was released from [CEL+KER+$Ag^0$NPs]. Since the total concentration of silver in the composite used in the measurements was about 12 mg, there was less than 0.02% of $Ag^0$NPs was released from the [CEL+KER+Ag°NPs] composites even after they were soaked in the solution for 7 days. Taken together, the results obtained clearly indicate that there was hardly any $Ag^0$NPs release from the [CEL+KER+$Ag^0$NPs] composite, and hence its antimicrobial activity and biocompatibility is due, not to any released $Ag^0$NPs but rather entirely to the $Ag^0$NPs embedded in the composite.

## Conclusions

In summary, we have shown that biocompatible composites containing 50% CEL and 50% KER and silver either in the ionic ($Ag^+$, presented as $Ag_2$ONPs) or metallic (Ag°NPs) were successfully synthesized in a simple process in which [$BMIm^+Cl^-$], an simple ionic liquid, was used as the sole solvent, and AgCl was added

to the [$BMIm^+Cl^-$] solution of [CEL+KER] during the dissolution process. The silver in the composite can be maintained as $Ag^+$ or completely converted to $Ag^0$NPs by reducing it with $NaBH_4$. Results of spectroscopy (FTIR, XRD) and imaging (SEM) measurements confirm that CEL and KER remain chemically intact and homogeneously distributed in the composites. XRD and SEM results show that the silver in the [CEL+KER+$Ag^+$] and [CEL+KER+$Ag^0$] composites are homogeneously distributed throughout the composites in either $Ag_2O$ NPS or $Ag^0$NPs form with size of (9 ± 1) nm or (27 ± 2) nm, respectively. Both composites were found to exhibit excellent antibacterial activity against many bacteria including *E. coli, S. aureus, P. aeruginosa,* MRSA, VRE. The bacterial activity of both composites increases with the $Ag^+$ or $Ag^0$NPs content in the composites. More importantly, for the same bacteria and the same silver content, [CEL+KER+$Ag^0$NPs] composite exhibits relatively greater antimicrobial activity against bacteria compared to the corresponding [CEL+KER+$Ag^+$] composite. Experimental results confirm that there was hardly any $Ag^0$NPs release from the [CEL+KER+$Ag^0$NPs] composite, and hence its antimicrobial activity and biocompatibility is due, not to any released $Ag^0$NPs but rather entirely to the $Ag^0$NPs embedded in the composite. Both of $Ag^+$ and $Ag^0$NPs were found to be toxic to human fibroblasts at higher concentration (>0.72 mmol), and that for the same silver content, [CEL+KER+$Ag^+$] composite is relatively more toxic than [CEL+KER+$Ag^0$NPs] composite. As expected, by lowering the $Ag^0$NPs concentration to 0.48 mmol or less, the [CEL+KER+$Ag^0$NPs] composite is biocompatible while still retaining antimicrobial activity against bacteria such as *E. coli, S. aureus, P. aeruginosa,* MRSA, VRE. These results together with our previous finding that [CEL+KER] composites can be used for controlled delivery of drugs such as ciprofloxacin clearly indicate that the [CEL+KER+$Ag^0$NPs] composite possess all required properties for successfully used as high performance dressing to treat chronic ulcerous infected wounds.

References

(1) Yang, X.; Yang, M.; Pang, B.; Vara, M.; Xia, Y. Gold Nanomaterials at Work in Biomedicine. Chem. Rev. 2015, 115, 10410-10488.

(2) Xia, X.; Zeng, J.; Zhang, Q.; Moran, C. H.; Xia, Y. Recent Developments in Shape-Controlled Synthesis of Silver Nanocrystals. J. Phys. Chem. C 2012, 116, 21647-21656.

(3) Chan, W. C. W.; et al. A Year for Nanoscience. ACS Nano 2014, 8, 11901-11903.

(4) Pelaz, B.; et al. The State of Nanoparticle-Based Nanoscience and Biotechnology: Progress, Promises, and Challenges. ACS Nano 2012, 6, 8468-8483.

(5) Sardar, R.; Shumaker-Parry, J. S. Spectroscopic and Microscopic Investigation of Gold Nanoparticle Formation: Ligand and Temperature Effects on Rate and Particle Size. J. Am. Chem. Soc. 2011, 133, 8179-8190.

(6) Wang, Z.; Bharathi, M. S.; Hariharaputran, R.; Xing, H.; Tang, L.; Li, J.; Zhang, Y.-W.; Lu, Y. pH-Dependent Evolution of Five-Star Gold Nanostructures: An Experimental and Computational Study. ACS Nano 2013, 7, 2258-2265.

(7) Wright, A. R.; Li, M.; Ravula, S.; Cadigan, M.; El-Zahab, B.; Das, S.; Baker, G. A.; Warner, I. M. Soft- and Hard-Templated Organic Salt Nanoparticles with the Midas Touch: Gold-Shelled NanoGUMBOS. J. Mater. Chem. C 2014, 2, 8996-9003.

(8) Takagai, Y.; Miura, R.; Endo, A.; Hinze, W. L. One-pot Synthesis with in situ Preconcentration of Spherical Monodispersed Gold Nanoparticles using Thermoresponsive 3-(alkyldimethylammonio)-propyl sulfate Zwitterionic Surfactants. Chem. Commun. 2016, 52, 10000-10003.

(9) Trefry, J. C.; Wooley, D. P. Silver Nanoparticles Inhibit Vaccinia Virus Infection by Preventing Viral entry through a Macro-pinocytosis-dependent Mechanism. J. Biomed. Nanotechnol. 2013, 9, 1624-1635.

(10) Noh, H. J.; Im, A.-R.; Kim, H.-S.; Sohng, J. K.; Kim, C.-K.; Kim, Y. S.; Cho, S.; Park, Y. Antibacterial Activity and Increased Freeze-drying Stability of Sialyllactosereduced Silver Nanoparticles using Sucrose and Trehalose. J. Nanosci. Nanotechnol. 2012, 12, 3884-3895.

(11) Guzman, M.; Dille, J.; Godet, S. Synthesis and Antibacterial Activity of Silver Nanoparticles against Gram-positive and Gram-negative Bacteria. Nanomedicine 2012, 8, 37-45.

(12) Mallakpour, S.; Dinari, M.; Talebi, M. A Facile, Efficient, and Green Fabrication of Nanocomposites based on L-leucine Containing Poly(amide-imide) and PVA-modified Ag Nanoparticles by Ultrasonic Irradiation Colloid. Colloid Polym. Sci. 2015, 293, 1827-1833.

(13) Gangadharan, D.; Harshvardan, K.; Gnanasekar, G.; Dixit, D.; Popat, K. M.; Anand, P. S. Polymeric Microspheres Containing Silver Nanoparticles as a Bactericidal Agent for Water Disinfection. Water

Res. 2010, 44, 5481-5487.

(14) Wei, D.; Sun, W.; Qian, W.; Ye, Y.; Ma, Y. The Synthesis of Chitosan-based Silver Nanoparticles and their Antibacterial Activity. Carbohydr. Res. 2009, 344, 2375-2382.

(15) Johnston, J. H.; Nilsson, T. Nanogold and Nanosilver Composites with Lignin-containing Cellulose Fibres. J. Mater. Sci. 2012, 47, 1103-1112.

(16) Wu, J.; Zheng, Y.; Song, W.; Luan, J.; Wen, X.; Wu, Z.; Chen, X.; Wang, Q.; Guo, S. In situ Synthesis of Silver-Nanoparticles/ bacterial Cellulose Composites for Slow-released Antimicrobial Wound Dressing. Carbohydr. Polym. 2014, 102, 762-771.

(17) Kelly, F. M.; Johnston, J. H. Colored and Functional Silver Nanoparticle-Wool Fiber Composites. ACS Appl. Mater. Interfaces 2011, 3, 1083-1092.

(18) Boroumand, M. N.; Montazer, M.; Simon, F.; Liesiene, J.; Šaponjic, Z.; Dutschk, V. Novel Method for Synthesis of Silver Nanoparticles and their Application on Wool. Appl. Surf. Sci. 2015, 346, 477-483.

(19) Hill, P.; Brantley, H.; Van Dyke, M. Some Properties of Keratin Biomaterials: Kerateines. Biomaterials 2010, 31, 585-593.

(20) Vasconcelos, A.; Cavaco-Paulo, A. The Use of Keratin in Biomedical Applications. Curr. Drug Targets 2013, 14, 612-619.

(21) Sando, L.; Kim, M.; Colgrave, M. L.; Ramshaw, J. A.; Werkmeister, J. A.; Elvin, C. M. Photochemical Crosslinking of Soluble Wool Keratins Produces a Mechanically Stable Biomaterial that Supports Cell Adhesion and Proliferation. J. Biomed. Mater. Res., Part A 2010, 95, 901-911.

(22) Yamauchi, K.; Maniwa, M.; Mori, T. Cultivation of Fibroblast Cells on Keratin-coated Substrates. J. Biomater. Sci., Polym. Ed. 1998, 9, 259-270.

(23) Cui, L.; Gong, J.; Fan, X.; Wang, P.; Wang, Q.; Qiu, Y. Trans Glutaminase-modified Wool Keratin Film and its Potential Application in Tissue Engineering. Eng. Life Sci. 2013, 13, 149-155.

(24) Xu, S.; Sang, L.; Zhang, Y.; Wang, X.; Li, X. Biological Evaluation of Human Hair Keratin Scaffolds for Skin Wound Repair and Regeneration. Mater. Sci. Eng., C 2013, 33, 648-655.

(25) de Guzman, R. C.; Merrill, M. R.; Richter, J. R.; Hamzi, R. I.; Greengauz-Roberts, O. K.; Van Dyke, M. E. Mechanical and Biological Properties of Keratose Biomaterials. Biomaterials 2011, 32, 8205-8217.

(26) Iqbal, H. M.N.; Kyazze, G.; Locke, I. C.; Tron, T.; Keshavarz, T. In situ Development of Self-defensive Antibacterial Biomaterials: Phenol-g-keratin-EC based Biocomposites with Characteristics for Biomedical Applications. Green Chem. 2015, 17, 3858-3869.

(27) Aluigi, A.; Vineis, C.; Varesano, A.; Mazzuchetti, G.; Ferrero, F.; Tonin, C. Structure and Properties of Keratin/PEO blend Nanofibers. Eur. Polym. J. 2008, 44, 2465-2475.

(28) Khosa, M. A.; Ullah, A. A Sustainable Role of Keratin Biopolymer in Green Chemistry: A Review. J. Food Proc. Beverages 2013, 1, 8-15.

(29) Rosewald, M.; Hou, F. Y. S.; Mututuvari, T.; Harkins, A. L.; Tran, C. D. Cellulose-Chitosan- Keratin Composite Materials: Synthesis and Immunological and Antibacterial Properties. ECS Trans. 2014, 64 (4), 499-505.

(30) Tran, C. D.; Mututuvari, T. Cellulose, Chitosan and Keratin Composite Materials. Controlled Drug Release. Langmuir 2015, 31, 1516-1526.

(31) Tran, C. D.; Mututuvari, T. Cellulose, Chitosan and Keratin Composite Materials. Facile and Recyclable Synthesis, Conformation and Properties. ACS Sustainable Chem. Eng. 2016, 4, 1850-1861.

(32) Tran, C. D.; Prosenc, F.; Franko, M.; Benzi, G. Synthesis, Structure and Antimicrobial Property of Green Composites from Cellulose, Wool, Hair and Chicken Feather. Carbohydr. Polym. 2016, 151, 1269-1276.

(33) Tran, C. D.; Duri, S.; Harkins, A. L. Recyclable Synthesis, Characterization and Antimicrobial Activity of Chitosan-based Polysaccharide Composite Materials. J. Biomed. Mater. Res., Part A 2013, 101, 2248-2257.

(34) Harkins, A. L.; Duri, S.; Kloth, L. C.; Tran, C. D. Chitosan- Cellulose Composite for Wound Dressing Material. Part 2. Antimicrobial Activity, Blood Absorption Ability and Biocompatibility. J. Biomed. Mater. Res., Part B 2014, 102 (6), 1199-1206.

(35) Mututuvari, T. M., Supramolecular Biopolymeric Composite Materials: Green Synthesis, Characterization and Applications. Dissertation, Marquette University, Wilwaukee, WI, 2014.

(36) Li, R.; Wang, D. Preparation of Regenerated Wool Keratin Films from Wool Keratin-ionic liquid Solutions. J. Appl. Polym. Sci. 2013, 127, 2648-2653.

(37) Peplow, P. V.; Roddick-Lanzilotta, A. D. Orthopaedic Materials Derived from Keratin. U.S. Patent 2005/0232963 A1, 2005.

(38) Fang, J. Y.; Chen, J. P.; Leu, Y. L.; Wang, H. Y. Characterization and Evaluation of Silk Protein Hydrogels for Drug Delivery. Chem. Pharm. Bull. 2006, 54, 156-162.

(39) JCPDS file No 31-1238.

(40) Dong, R.; Tian, B.; Zeng, C.; Li, T.; Wang, T.; Zhang, J. Ecofriendly Synthesis and Photocatalytic Activity of Uniform Cubic Ag@AgCl Plasmonic Photocatalyst. J. Phys. Chem. C 2013, 117, 213-220.

(41) Veronica da Silva Ferreiraa, V. D. S.; ConzFerreiraa, M. E.; Lima, L. M. T. R. Green Production of Microalgae-based Silver Chloride Nanoparticles with Antimicrobial Activity against Pathogenic Bacteria Enzym. Microbial. Tech. 2016 Ahead of print http://dx.doi. org/10.1016/j.enzmictec.2016.10.018.

(42) Dhas, T. S.; Kumar, V. G.; Karthick, V.; Angel, K. J.; Govindaraju, K. Facile Synthesis of Silver Chloride Nanoparticles using Marine Alga and its Antibacterial Efficacy, Spectrochim. Acta A Mol. Biomol. Spectrosc. Acta A Mol. Biomol. Spectrosc. 2014, 120, 416-420.

(43) Sohrabnezhad, Sh.; Rassa, M.; Dahanesari, E. M. Spectroscopic Study of Silver Halides in Montmorillonite and their Antibacterial Activity. J. Photochem. Photobiol., B 2016, 163, 150-155.

(44) Sharma, P.; Sanpui, P.; Chattopadhyay, A.; Ghosh, S. Fabrication of Antibacterial Silver Nanoparticle - Sodium Alginate - Chitosan Composite Films. RSC Adv. 2012, 2, 5837-5843.

(45) Sathishkumar, M.; Sneha, K.; Yun, Y. S. Immobilization of Silver Nanoparticles Synthesized using Curcuma Longa Tuber Powder and Extract on Cotton Cloth for Bactericidal Activity. Bioresour. Technol. 2010, 101, 7958-7965.

(46) JCPDS 04-0783.

(47) Scherrer, P. Bestimmung der Grösse und der Inneren Struktur von Kolloidteilchen Mittels Röntgenstrahlen,. Nachr. Ges. Wiss. Göttingen 1918, 26, 98-100.

(48) Langford, J. J.; Wilson, A. J. C. Scherrer after Sixty Years: A Survey and Some New Results in the Determination of Crystallite Size. J. Appl. Crystallogr. 1978, 11, 102-113.

(49) Korte, D.; Concetta Bruzzoniti, M.; Sarzanini, C.; Franko, M. Thermal Lens Spectrometric Determination of Colloidal and Ionic Silver in Water. Int. J. Thermophys. 2011, 32, 818-827.

(50) Tran, C. D.; Franko, M. Thermal Lens Spectroscopy. In Encyclopedia of Analytical Chemistry; Meyers, R. A., Ed.; John Wiley: Chichester, U.K., 2010; DOI: 10.1002/9780470027318.a9079.

(51) Korte, D.; Bruzzoniti, M. C.; Sarzanini, C.; Franko, M. Thermal Lens Spectrometric Determination of Colloidal and Ionic Silver in Water. Int. J. Thermophys., 2011, 32, 818-827.

(52) Tran, C. D.; Franko, M. "Thermal Lens Spectroscopy" In: Encyclopedia of Analytical Chemistry, ed.: R.A. Meyers, John Wiley: Chichester., 2010. DOI: 10.1002/9780470027318.a9079.

Citations to a number of patent and non-patent references may be made herein. In the event that there is an inconsistency between a definition of a term in the specification as compared to a definition of the term in a cited reference, the term should be interpreted based on the definition in the specification.

## Claims

**1.** An ionic liquid composition comprising:

a) a structural polysaccharide, wherein the structural polysaccharide is a polymer comprising 6-carbon monosaccharides linked via beta-1,4 linkages;
b) a structural protein; and
c) metal nanoparticles and/or metal oxide nanoparticles,

wherein the structural polysaccharide and structural protein are dissolved in an ionic liquid,

wherein the structural protein comprises keratin, and
wherein the metal nanoparticles comprise gold, silver, or copper nanoparticles and/or wherein the metal oxide nanoparticles comprise gold, silver, or copper oxide nanoparticles.

**2.** The composition of claim 1, wherein:

a) the structural polysaccharide comprises:

i) cellulose; and/or
ii) chitin; and/or
iii) chitosan; and/or

b) the metal nanoparticles comprise silver nanoparticles and/or wherein the metal oxide nanoparticles comprise silver oxide nanoparticles; and/or
c) the ionic liquid is:

i) an alkylated imidazolium salt, optionally wherein the alkylated imidazolium salt is selected from a group consisting of 1-butyl-3-methylimidazolium salt, 1-ethyl-3-methylimidazolium salt, and 1-allyl-3-methylimidazolium salt; and/or
ii)1-butyl-3-methylimidazolium chloride; and/or

d) the ionic liquid composition comprises at least 4% w/w of the dissolved structural polysaccharide; and/or
e) the ionic liquid composition comprises at least 10% w/w of the dissolved structural polysaccharide.

**3.** A method for preparing a composite material comprising a structural polysaccharide, keratin, and metal nanoparticles and/or metal oxide nanoparticles, wherein the metal nanoparticles comprise gold, silver, or copper nanoparticles and/or wherein the metal oxide nanoparticles comprise gold, silver, or copper oxide nanoparticles, wherein the structural polysaccharide is a polymer comprising 6-carbon monosaccharides linked via beta-1,4 linkages, and wherein the method comprises adding the metal nanoparticles and/or metal oxide nanoparticles to an ionic liquid composition comprising the structural polysaccharide and keratin dissolved therein and removing the ionic liquid from the ionic liquid composition to prepare the composite material.

**4.** The method of claim 3, wherein:

a) the method further comprises contacting the metal oxide nanoparticles with a reducing agent, optionally wherein the reducing agent comprises watermelon rind; and/or
b) the ionic liquid is removed by steps that include washing the ionic liquid composition with an aqueous solution to obtain the composite material and drying the composite material thus obtained.

**5.** A composite material prepared by the method of claim 3 or 4.

**6.** A method for removing a contaminant from a stream, the method comprising contacting the stream with the composite material of claim 5.

**7.** A method for killing or eliminating microbes, the method comprising contacting the microbes with the composite material of claim 5.

**8.** A method of purifying a compound from a stream, the method comprising contacting the compound with the composite material of claim 5.

**9.** The method of claim 8, wherein the compound is an enantiomer and the stream comprises a racemic mixture of the compound.

**10.** A method for catalyzing a reaction, the method comprising contacting a reaction mixture with the composite material of claim 5.

**11.** A method for delivering a compound, the method comprising contacting the compound with the composite material of claim 5 and allowing the compound to diffuse from the composite material.

**12.** A filter comprising the composite material of claim 5.

**13.** A bandage comprising the composite material of claim 5.

**14.** A method of purifying an enantiomer of a compound from a racemic mixture of the compound, the method comprising contacting the racemic mixture with a composite material, wherein the composite material is prepared according to the method of claim 3.

**15.** The method of claim 14, wherein:

a) the structural polysaccharide is a mixture of cellulose and chitosan; and/or
b) the metal nanoparticles comprise silver nanoparticles, and/or the metal oxide nanoparticles comprise silver oxide nanoparticles.

## Patentansprüche

**1.** Ionische flüssige Zusammensetzung, umfassend:

a) ein Strukturpolysaccharid, wobei das Strukturpolysaccharid ein Polymer ist, das über Beta-1,4-Bindungen verknüpfte 6-Kohlenstoff-Monosaccharide umfasst;
b) ein Strukturprotein; und
c) Metallnanopartikel und/oder Metalloxidnanopartikel,

wobei das Strukturpolysaccharid und das Strukturprotein in einer ionischen Flüssigkeit gelöst sind,
wobei das Strukturprotein Keratin umfasst und
wobei die Metallnanopartikel Gold-, Silber- oder Kupfernanopartikel umfassen und/oder wobei die Metalloxidnanopartikel Gold-, Silber- oder Kupferoxidnanopartikel umfassen.

**2.** Zusammensetzung nach Anspruch 1, bei der:

a) das Strukturpolysaccharid

(i) Cellulose; und/oder
(ii) Chitin; und/oder
(iii) Chitosan

umfasst; und/oder
b) die Metallnanopartikel Silbernanopartikel umfassen und/oder bei der die Metalloxidnanopartikel Silberoxidnanopartikel umfassen; und/oder
c) die ionische Flüssigkeit

i) ein alkyliertes Imidazoliumsalz, wobei das alkylierte Imidazoliumsalz optional aus einer Gruppe ausgewählt ist, die aus 1-Butyl-3-methylimidazoliumsalz, 1-Ethyl-3-methylimidazoliumsalz und 1-Allyl-3-methylimidazoliumsalz besteht; und/oder
ii) 1-Butyl-3-methylimidazoliumchlorid ist; und/oder

d) die ionische flüssige Zusammensetzung mindestens 4 Gew.-% des gelösten Strukturpolysaccharids umfasst; und/oder
e) die ionische flüssige Zusammensetzung umfasst mindestens 10 Gew.-% des gelösten Strukturpolysaccharids umfasst.

**3.** Verfahren zum Herstellen eines Verbundmaterials, das ein Strukturpolysaccharid, Keratin und Metallnanopartikel und/oder Metalloxidnanopartikel umfasst, wobei die Metallnanopartikel Gold-, Silber- oder Kupfernanopartikel umfassen und/oder wobei die Metalloxidnanopartikel Gold-, Silber- oder Kupferoxidnanopartikel umfassen, wobei das Strukturpolysaccharid ein Polymer ist, das über Beta-1,4-Bindungen verknüpfte 6-Kohlenstoff-Monosaccharide umfasst, und wobei das Verfahren das Hinzufügen der Metallnanopartikel und/oder Metalloxidnanopartikel zu einer ionischen flüssigen Zusammensetzung, die das Strukturpolysaccharid und darin gelöstes Keratin umfasst, und das Entfernen der ionischen Flüssigkeit aus der ionischen flüssigen Zusammensetzung zum Herstellen des Verbundmaterials umfasst.

**4.** Verfahren nach Anspruch 3, bei dem:

a) das Verfahren ferner das Inkontaktbringen der Metalloxidnanopartikel mit einem Reduktionsmittel umfasst, wobei das Reduktionsmittel optional Wassermelonenschale umfasst; und/oder
b) die ionische Flüssigkeit durch Schritte entfernt wird, die das Spülen der ionischen flüssigen Zusammensetzung mit einer wässrigen Lösung, um das Verbundmaterial zu erhalten, und das Trocknen des so erhaltenen Verbundmaterials umfassen.

**5.** Verbundmaterial, hergestellt durch das Verfahren nach Anspruch 3 oder 4.

**6.** Verfahren zum Entfernen einer Verunreinigung aus einem Strom, wobei das Verfahren das Inkontaktbringen des Stroms mit dem Verbundmaterial nach Anspruch 5 umfasst.

**7.** Verfahren zum Abtöten oder Eliminieren von Mikroben, wobei das Verfahren das Inkontaktbringen der Mikroben mit dem Verbundmaterial nach Anspruch 5 umfasst.

**8.** Verfahren zum Reinigen einer Verbindung aus einem Strom, wobei das Verfahren das Inkontaktbringen der Verbindung mit dem Verbundmaterial nach Anspruch 5 umfasst.

**9.** Verfahren nach Anspruch 8, bei dem die Verbindung ein Enantiomer ist und der Strom eine racemische Mischung der Verbindung umfasst.

**10.** Verfahren zum Katalysieren einer Reaktion, wobei das Verfahren das Inkontaktbringen eines Reaktionsgemischs mit dem Verbundmaterial nach Anspruch 5 umfasst.

**11.** Verfahren zum Freisetzen einer Verbindung, wobei das Verfahren das Inkontaktbringen der Verbindung mit dem Verbundmaterial nach Anspruch 5 und das Diffundierenlassen der Verbindung aus dem Verbundmaterial umfasst.

**12.** Filter, umfassend das Verbundmaterial nach Anspruch 5.

**13.** Verband, umfassend das Verbundmaterial nach Anspruch 5.

**14.** Verfahren zum Reinigen eines Enantiomers einer Verbindung aus einer racemischen Mischung der Verbindung, wobei das Verfahren das Inkontaktbringen der racemischen Mischung mit einem Verbundmaterial umfasst, wobei das Verbundmaterial gemäß dem Verfahren nach Anspruch 3 hergestellt ist.

**15.** Verfahren nach Anspruch 14, bei dem:

a) das Strukturpolysaccharid ein Gemisch von Cellulose und Chitosan ist; und/oder
b) die Metallnanopartikel Silbernanopartikel umfassen und/oder die Metalloxidnanopartikel Silberoxidnanopartikel umfassen.

## Revendications

**1.** Composition liquide ionique, comprenant :

a) un polysaccharide structurel, dans laquelle le polysaccharide structurel est un polymère comprenant des monosaccharides à 6 carbones liés par des liaisons bêta-1, 4 ;
b) une protéine structurelle ; et
c) des nanoparticules de métal et/ou des nanoparticules d'oxyde de métal,

dans laquelle le polysaccharide structurel et la protéine structurelle sont dissous dans un liquide ionique,
dans laquelle la protéine structurelle comprend de la kératine, et
dans laquelle les nanoparticules de métal comprennent des nanoparticules d'or, d'argent ou de cuivre et/ou dans laquelle les nanoparticules d'oxyde de métal comprennent des nanoparticules d'oxyde d'or, d'argent ou de cuivre.

**2.** Composition selon la revendication 1, dans laquelle :

a) le polysaccharide structurel comprend :

i) de la cellulose ; et/ou
ii) de la chitine ; et/ou
iii) du chitosane ; et/ou

b) les nanoparticules de métal comprennent des nanoparticules d'argent et/ou dans laquelle les nanoparticules d'oxyde de métal comprennent des nanoparticules d'oxyde d'argent ; et/ou
c) le liquide ionique est :

i) un sel d'imidazolium alkylé, facultativement dans laquelle le sel d'imidazolium alkylé est choisi dans un groupe constitué d'un sel de 1-butyl-3-méthylimidazolium, d'un sel de 1-éthyl-3-méthylimidazolium et d'un sel de 1-allyl-3-méthylimidazolium ; et/ou
ii) du chlorure de 1-butyl-3-méthylimidazolium ; et/ou

d) la composition liquide ionique comprend au moins 4 % p/p du polysaccharide structurel dissous ; et/ou
e) la composition liquide ionique comprend au moins 10 % p/p du polysaccharide structurel dissous.

**3.** Procédé de préparation d'un matériau composite comprenant un polysaccharide structurel, de la kératine et des nanoparticules de métal et/ou des nanoparticules d'oxyde de métal, dans lequel les nanoparticules de métal comprennent des nanoparticules d'or, d'argent ou de cuivre et/ou dans lequel les nanoparticules d'oxyde de métal comprennent des nanoparticules d'oxyde d'or, d'argent ou de cuivre, dans lequel le polysaccharide structurel est un polymère comprenant des monosaccharides à 6 carbones liés par des liaisons bêta-1,4, et dans lequel le procédé comprend l'ajout des nanoparticules de métal et/ou des nanoparticules d'oxyde de métal à une composition liquide ionique comprenant le polysaccharide structurel et la kératine dissous dans celui-ci, et l'élimination du liquide ionique à partir de la composition liquide ionique pour préparer le matériau composite.

**4.** Procédé selon la revendication 3, dans lequel :

a) le procédé comprend en outre une mise en contact des nanoparticules d'oxyde de métal avec un agent réducteur, facultativement dans lequel l'agent réducteur comprend de l'écorce de pastèque ; et/ou
b) le liquide ionique est éliminé par des étapes qui incluent un lavage de la composition de liquide ionique avec une solution aqueuse pour obtenir le matériau composite, et un séchage du matériau composite ainsi obtenu.

**5.** Matériau composite préparé par le procédé selon la revendication 3 ou 4.

**6.** Procédé d'élimination d'un contaminant à partir d'un flux, le procédé comprenant une mise en contact du flux avec le matériau composite de la revendication 5.

**7.** Procédé pour tuer ou éliminer des microbes, le procédé comprenant une mise en contact des microbes avec le matériau composite de la revendication 5.

**8.** Procédé de purification d'un composé à partir d'un flux, le procédé comprenant une mise en contact du composé avec le matériau composite de la revendication 5.

**9.** Procédé selon la revendication 8, dans lequel le composé est un énantiomère, et le flux comprend un mélange racémique du composé.

**10.** Procédé de catalyse d'une réaction, le procédé comprenant une mise en contact d'un mélange réactionnel avec le matériau composite de la revendication 5.

**11.** Procédé d'administration d'un composé, le procédé comprenant une mise en contact du composé avec le matériau composite de la revendication 5 et une autorisation de diffusion du composé à partir du matériau composite.

**12.** Filtre comprenant le matériau composite selon la revendication 5.

**13.** Bandage comprenant le matériau composite selon la revendication 5.

**14.** Procédé de purification d'un énantiomère d'un composé à partir d'un mélange racémique du composé, le procédé comprenant une mise en contact du mélange racémique avec un matériau composite, dans lequel le matériau composite est préparé selon le procédé de la revendication 3.

**15.** Procédé selon la revendication 14, dans lequel :

a) le polysaccharide structurel est un mélange de cellulose et de chitosane ; et/ou
b) les nanoparticules de métal comprennent des nanoparticules d'argent, et/ou les nanoparticules d'oxyde de métal comprennent des nanoparticules d'oxyde d'argent.

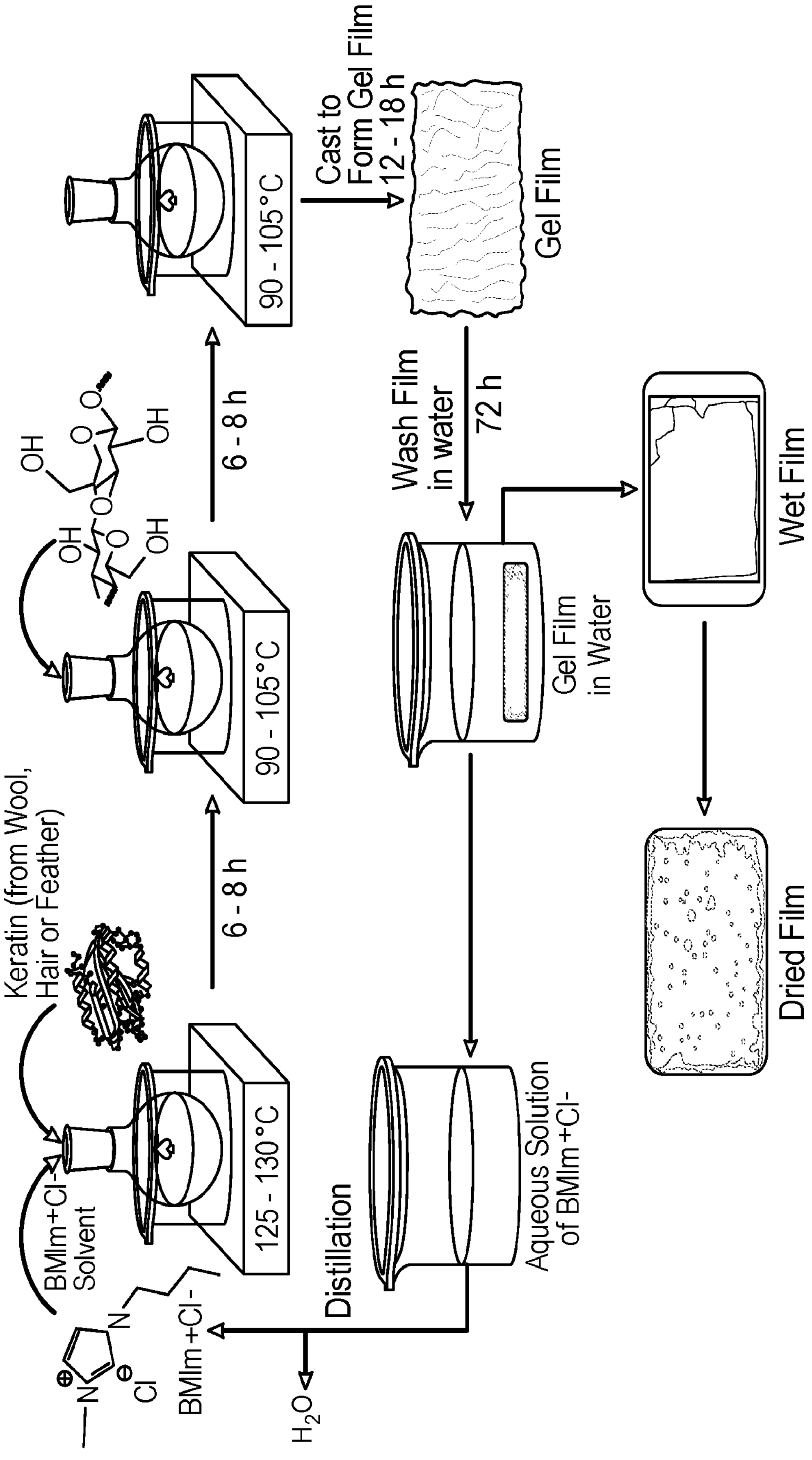
BMIm+Cl-
Solvent
Keratin (from Wool,
Hair or Feather)
125 - 130°C
6 - 8 h
90 - 105°C
6 - 8 h
90 - 105°C
Cast to
Form Gel Film
12 - 18 h
Gel Film
Wash Film
in water
72 h
Gel Film
in Water
Wet Film
Dried Film
Aqueous Solution
of BMIm+Cl-
Distillation
BMIm+Cl-
$H_2O$

*FIG. 1*

Figure 2

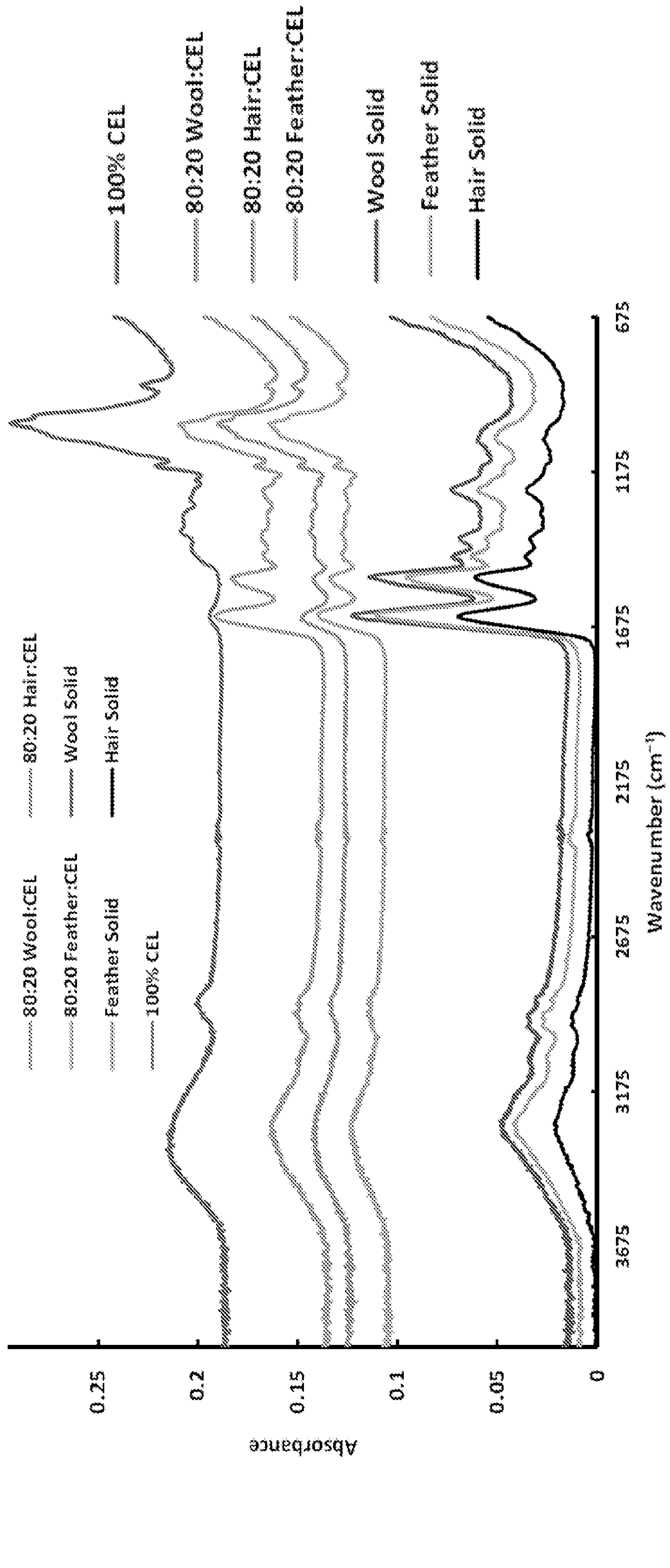
Absorbance
0
0.05
0.1
0.15
0.2
0.25
Wavenumber (cm$^{-1}$)
3675
3175
2675
2175
1675
1175
675
80:20 Wool:CEL
80:20 Hair:CEL
80:20 Feather:CEL
Wool Solid
Feather Solid
Hair Solid
100% CEL

Figure 3

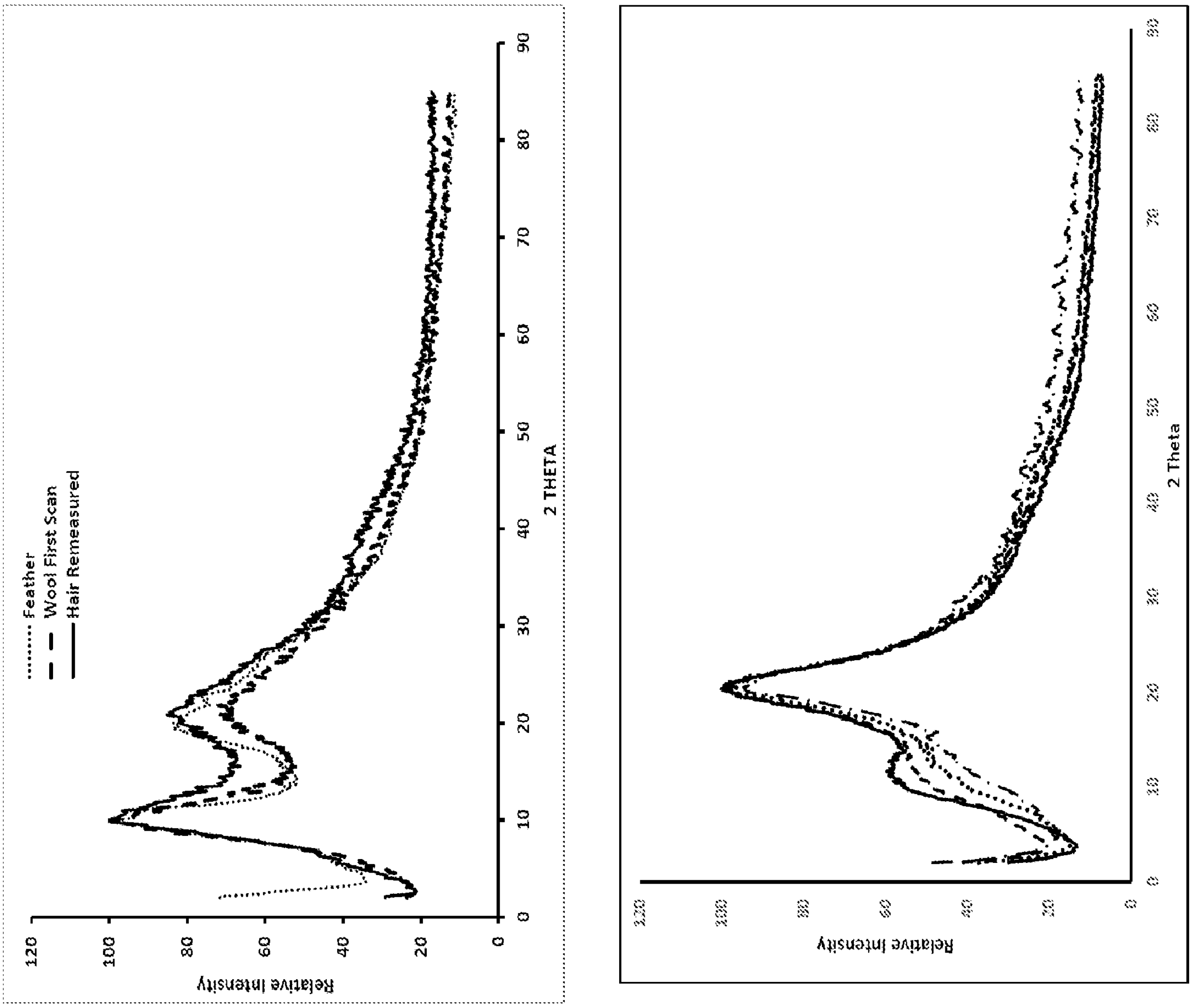
Feather
Wool First Scan
Hair Remeasured
Relative Intensity
2 THETA
Relative Intensity
2 Theta

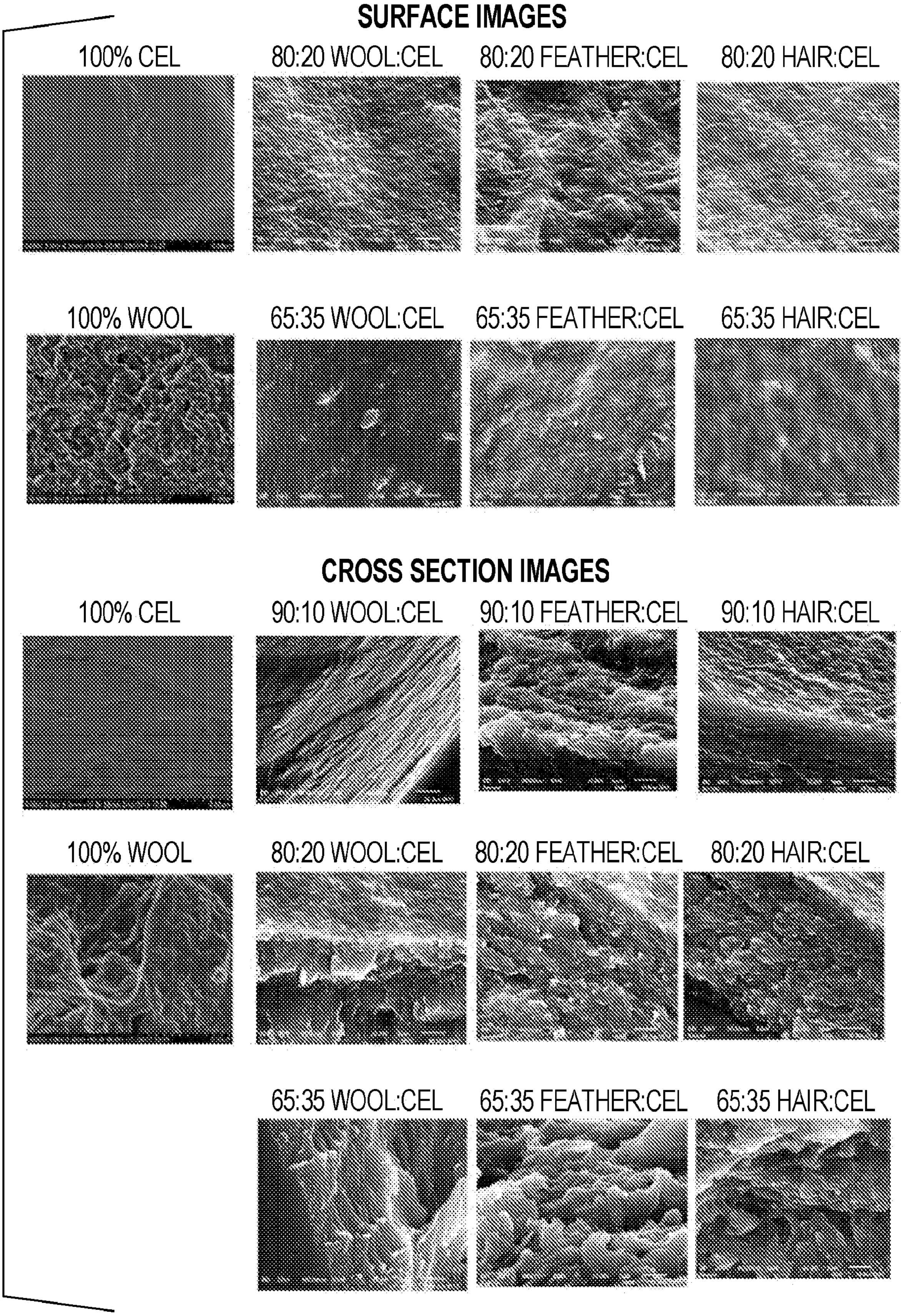
SURFACE IMAGES
100% CEL
80:20 WOOL:CEL
80:20 FEATHER:CEL
80:20 HAIR:CEL
100% WOOL
65:35 WOOL:CEL
65:35 FEATHER:CEL
65:35 HAIR:CEL
CROSS SECTION IMAGES
100% CEL
90:10 WOOL:CEL
90:10 FEATHER:CEL
90:10 HAIR:CEL
100% WOOL
80:20 WOOL:CEL
80:20 FEATHER:CEL
80:20 HAIR:CEL
65:35 WOOL:CEL
65:35 FEATHER:CEL
65:35 HAIR:CEL

*FIG. 4*

Figure 5

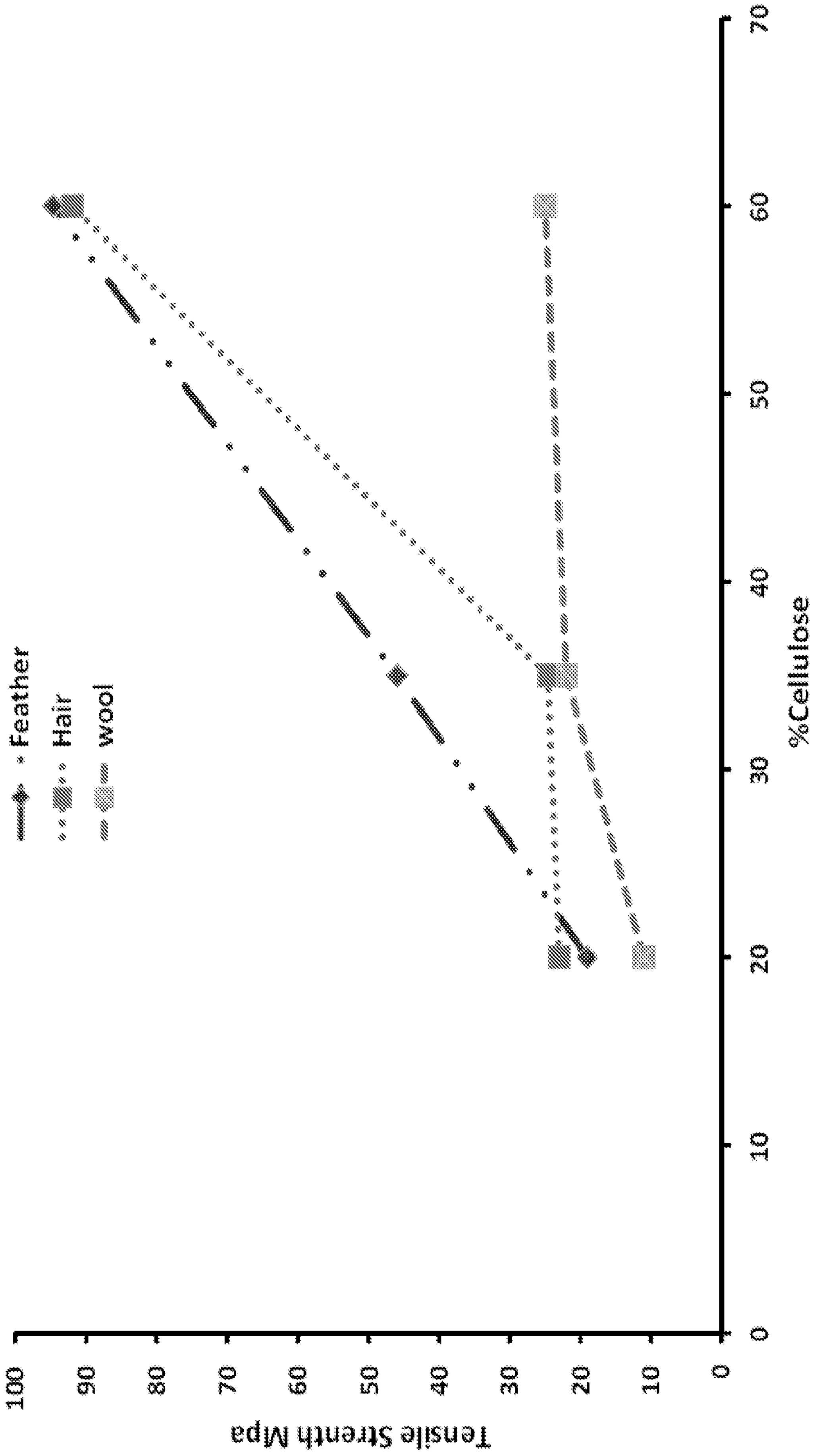
Feather
Hair
wool
Tensile Strenth Mpa
%Cellulose
0
10
20
30
40
50
60
70
80
90
100
0
10
20
30
40
50
60
70

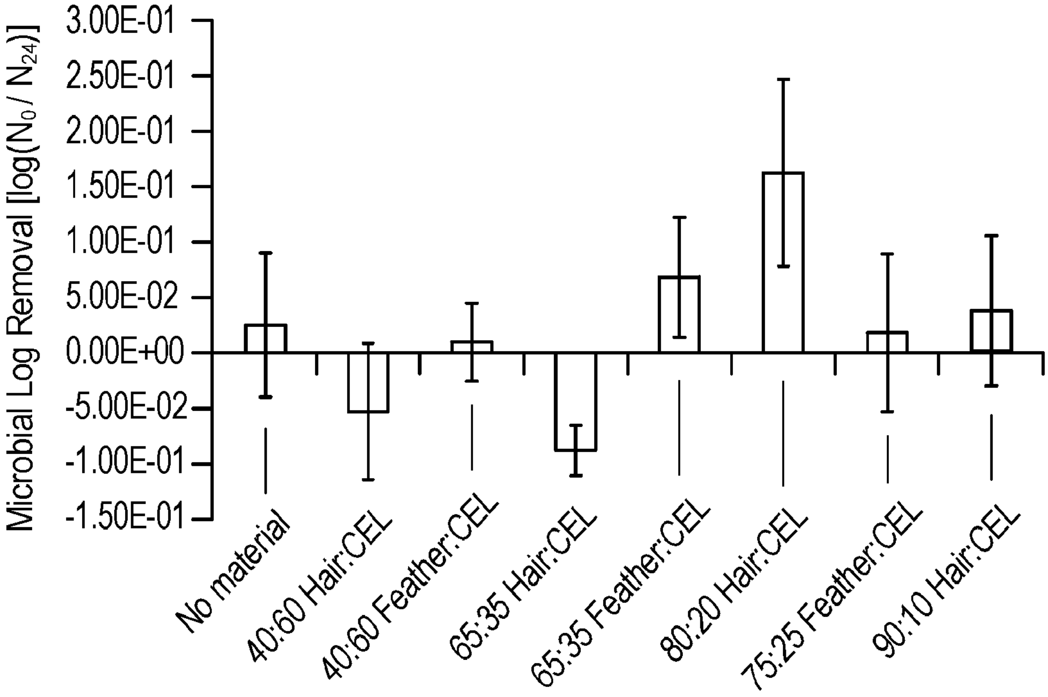
Microbial Log Removal [log($N_0$ / $N_{24}$)]
3.00E-01
2.50E-01
2.00E-01
1.50E-01
1.00E-01
5.00E-02
0.00E+00
-5.00E-02
-1.00E-01
-1.50E-01
No material
40:60 Hair:CEL
40:60 Feather:CEL
65:35 Hair:CEL
65:35 Feather:CEL
80:20 Hair:CEL
75:25 Feather:CEL
90:10 Hair:CEL

*FIG. 6A*

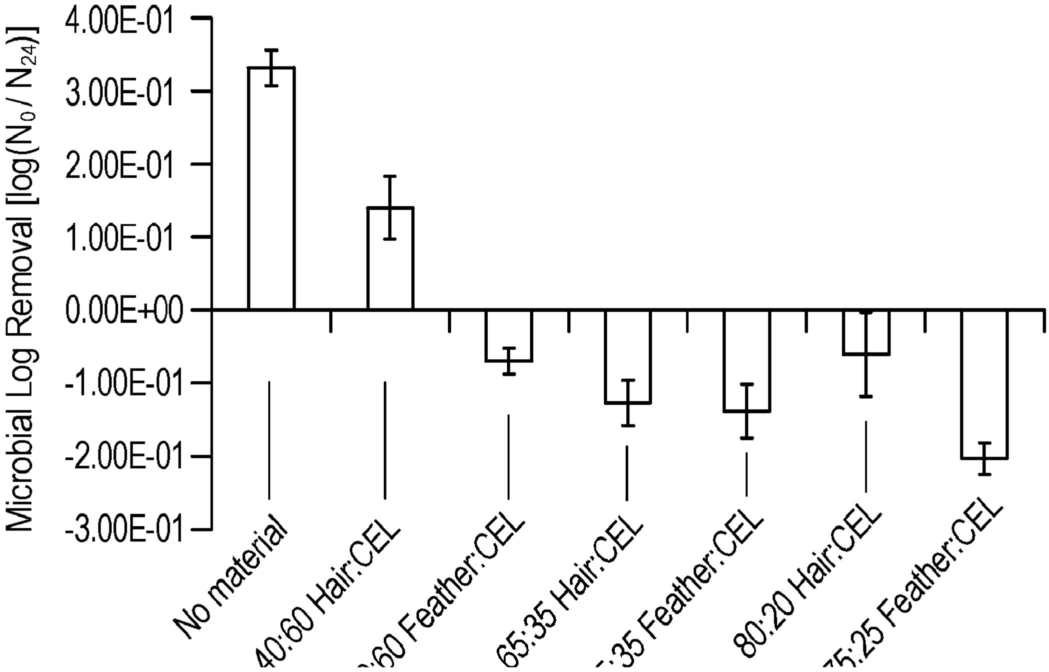
Microbial Log Removal [log($N_0$ / $N_{24}$)]
4.00E-01
3.00E-01
2.00E-01
1.00E-01
0.00E+00
-1.00E-01
-2.00E-01
-3.00E-01
No material
40:60 Hair:CEL
:60 Feather:CEL
65:35 Hair:CEL
:35 Feather:CEL
80:20 Hair:CEL
5:25 Feather:CEL

*FIG. 6B*

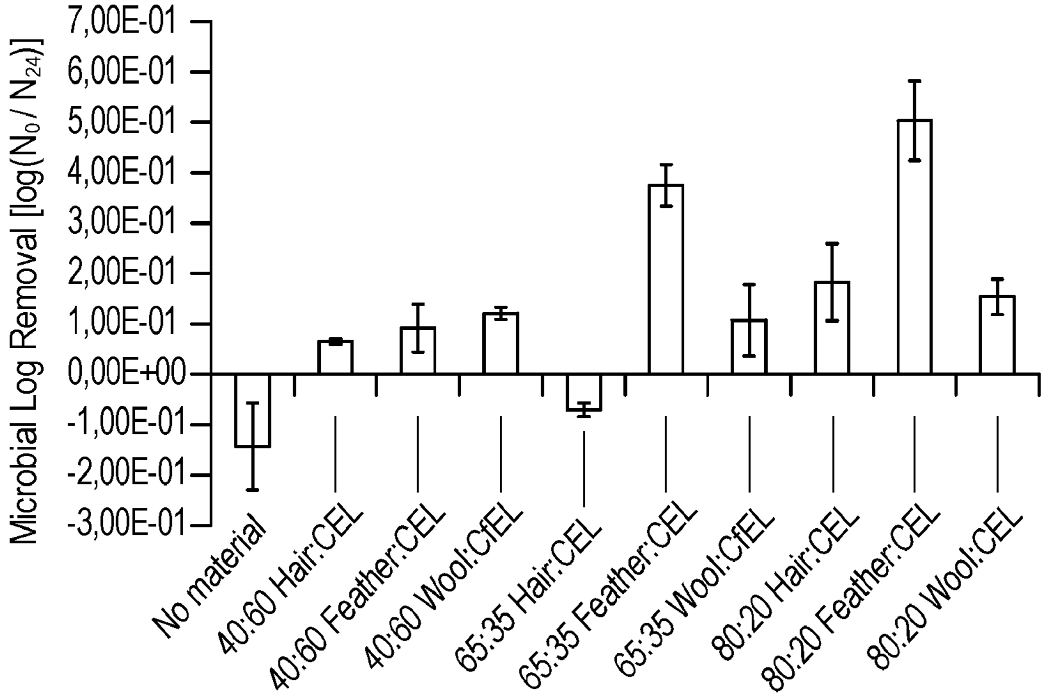
Microbial Log Removal [log(N0 / N24)]
7,00E-01
6,00E-01
5,00E-01
4,00E-01
3,00E-01
2,00E-01
1,00E-01
0,00E+00
-1,00E-01
-2,00E-01
-3,00E-01
No material
40:60 Hair:CEL
40:60 Feather:CEL
40:60 Wool:CfEL
65:35 Hair:CEL
65:35 Feather:CEL
65:35 Wool:CfEL
80:20 Hair:CEL
80:20 Feather:CEL
80:20 Wool:CEL

*FIG. 6C*

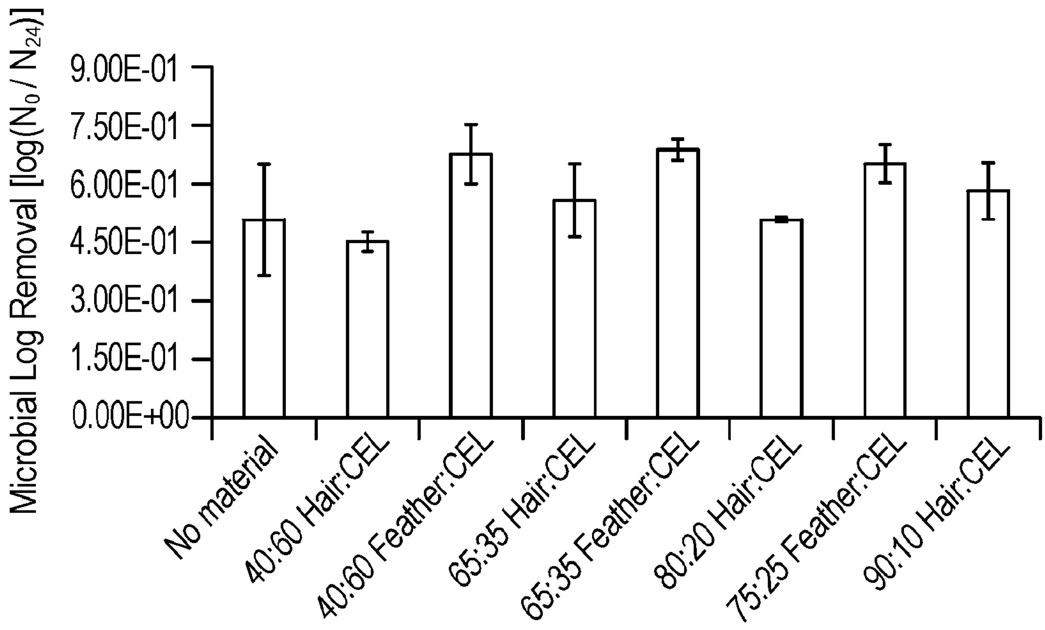
Microbial Log Removal [log(N0 / N24)]
9.00E-01
7.50E-01
6.00E-01
4.50E-01
3.00E-01
1.50E-01
0.00E+00
No material
40:60 Hair:CEL
40:60 Feather:CEL
65:35 Hair:CEL
65:35 Feather:CEL
80:20 Hair:CEL
75:25 Feather:CEL
90:10 Hair:CEL

*FIG. 6D*

Figure 7

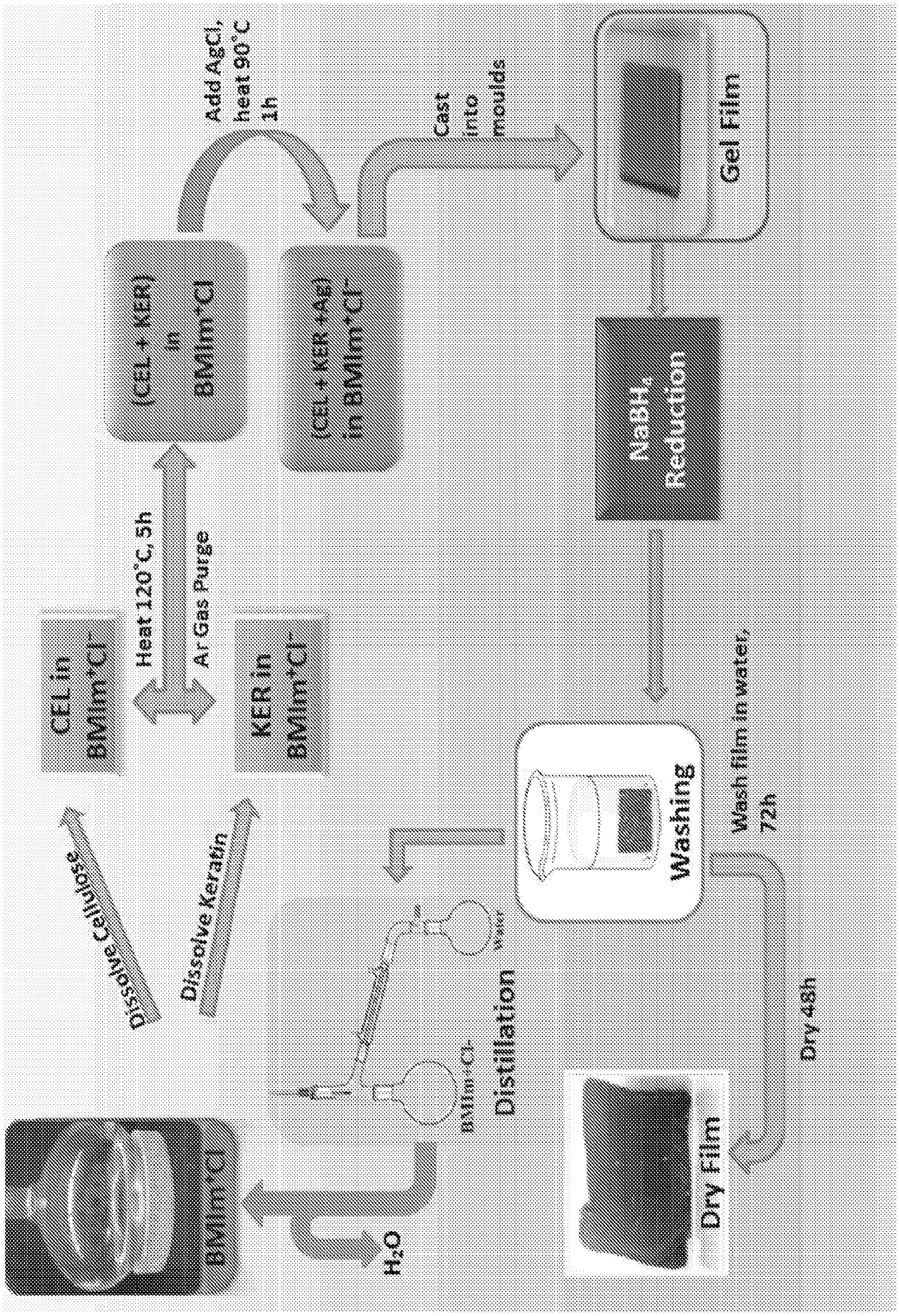
BMIm+Cl−
H2O
BMIm+Cl−
Water
Distillation
Dissolve Cellulose
Dissolve Keratin
CEL in BMIm+Cl−
KER in BMIm+Cl−
Heat 120°C, 5h
Ar Gas Purge
(CEL + KER) in BMIm+Cl−
Add AgCl, heat 90°C 1h
(CEL + KER +Ag) in BMIm+Cl−
Cast into moulds
Gel Film
NaBH4 Reduction
Washing
Wash film in water, 72h
Dry 48h
Dry Film

Figure 8

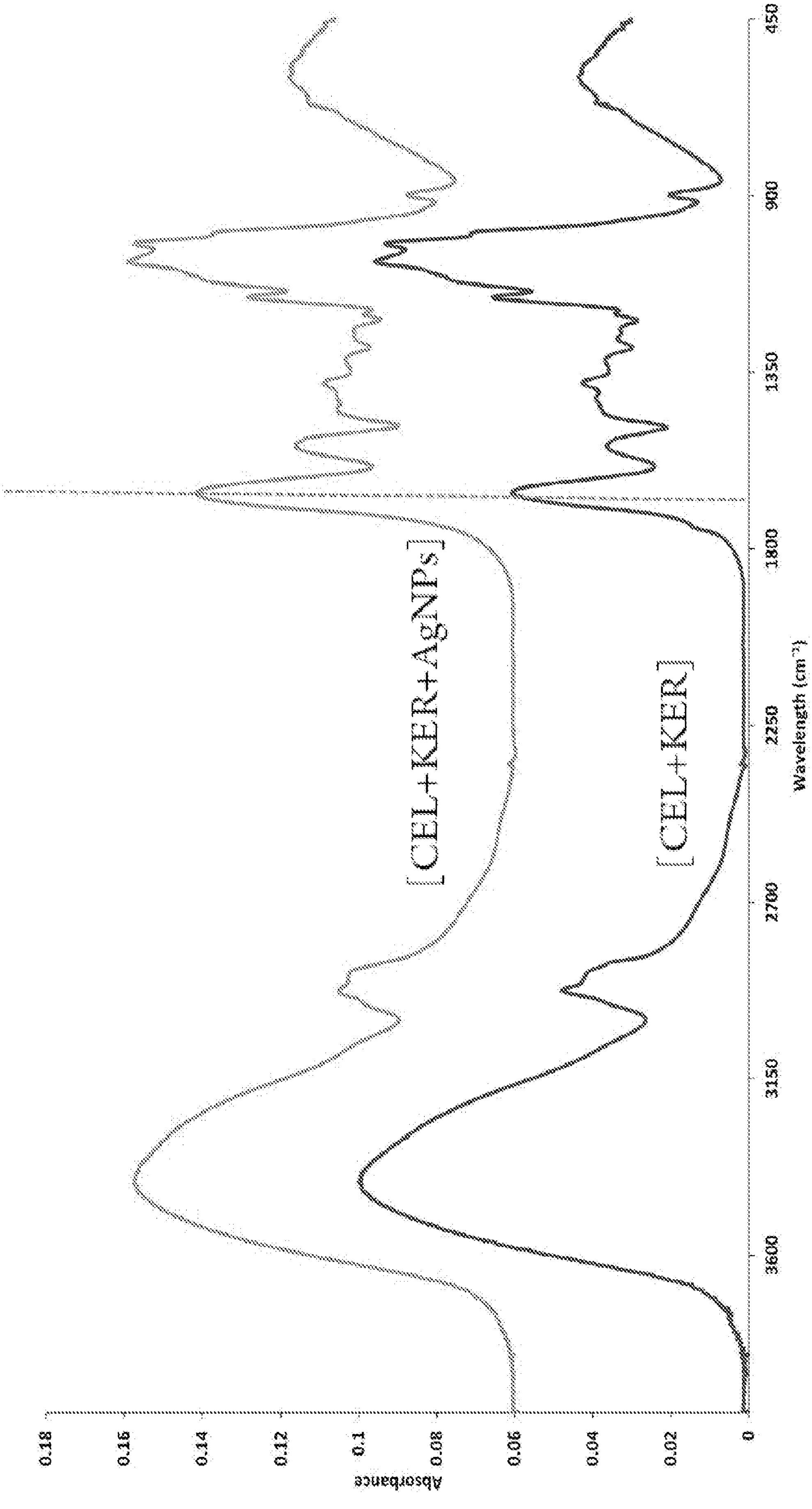
Absorbance
0
0.02
0.04
0.06
0.08
0.1
0.12
0.14
0.16
0.18
Wavelength (cm$^{-1}$)
3600
3150
2700
2250
1800
1350
900
450
[CEL+KER+AgNPs]
[CEL+KER]

**Figure 9**

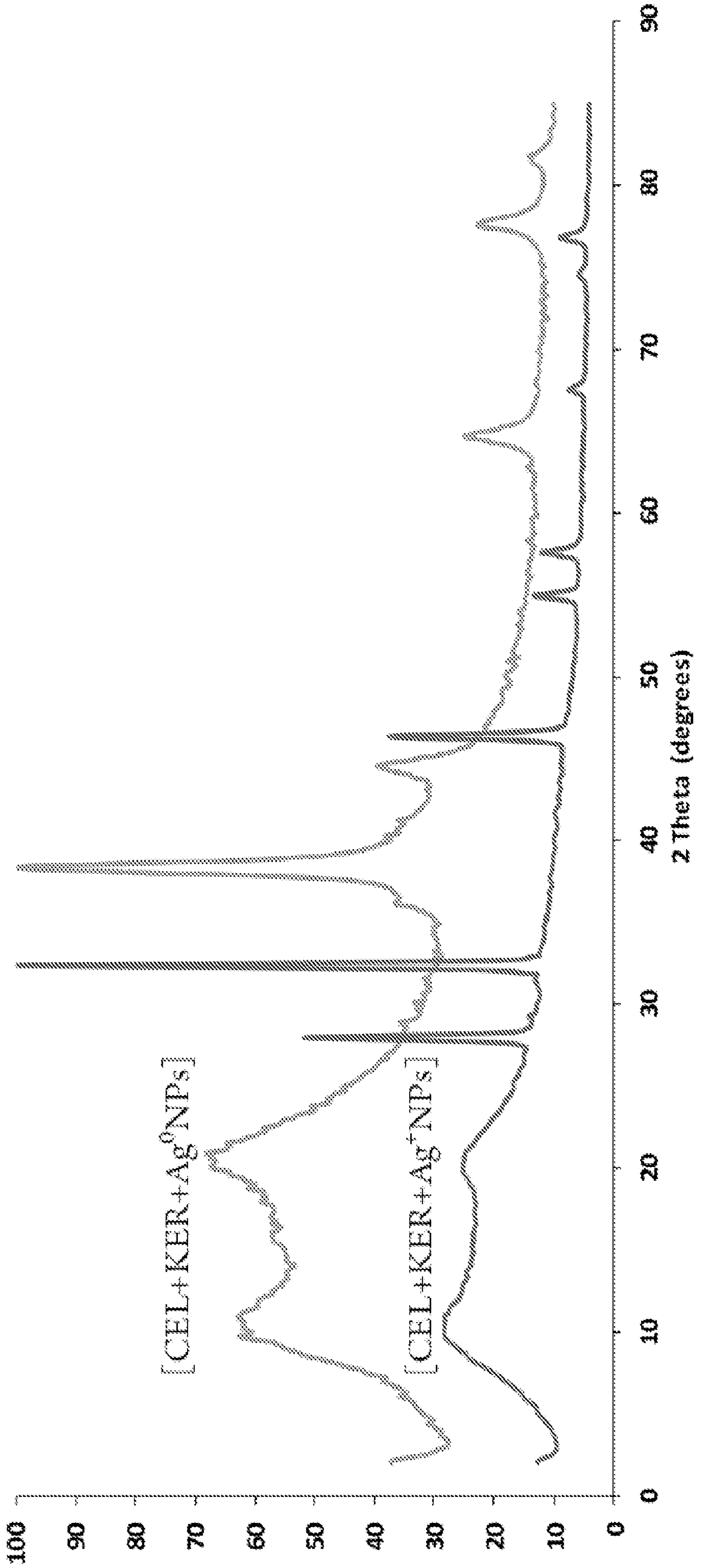
[CEL+KER+Ag$^0$NPs]
[CEL+KER+Ag$^+$NPs]
Relative Intensity (%)
2 Theta (degrees)
0
10
20
30
40
50
60
70
80
90
100

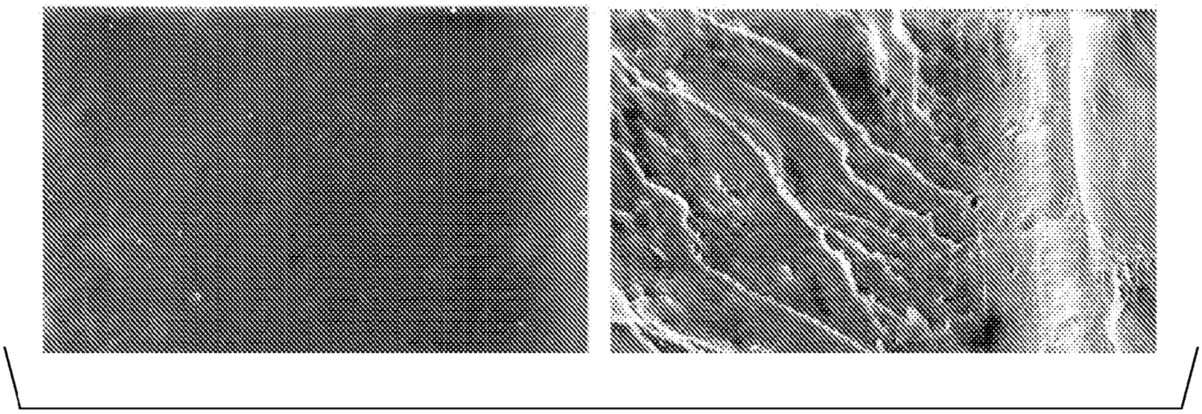

FIG. 10A

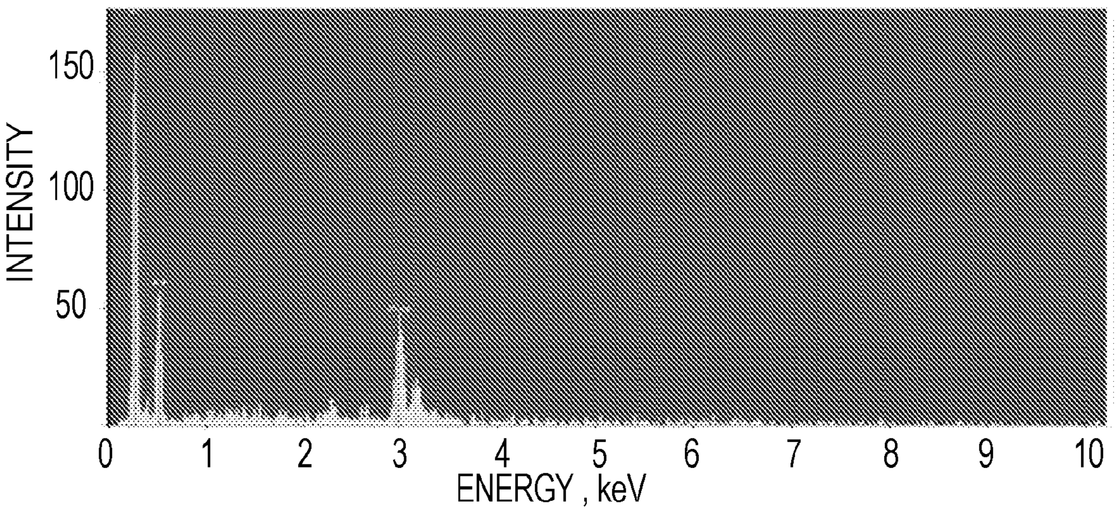


FIG. 10B

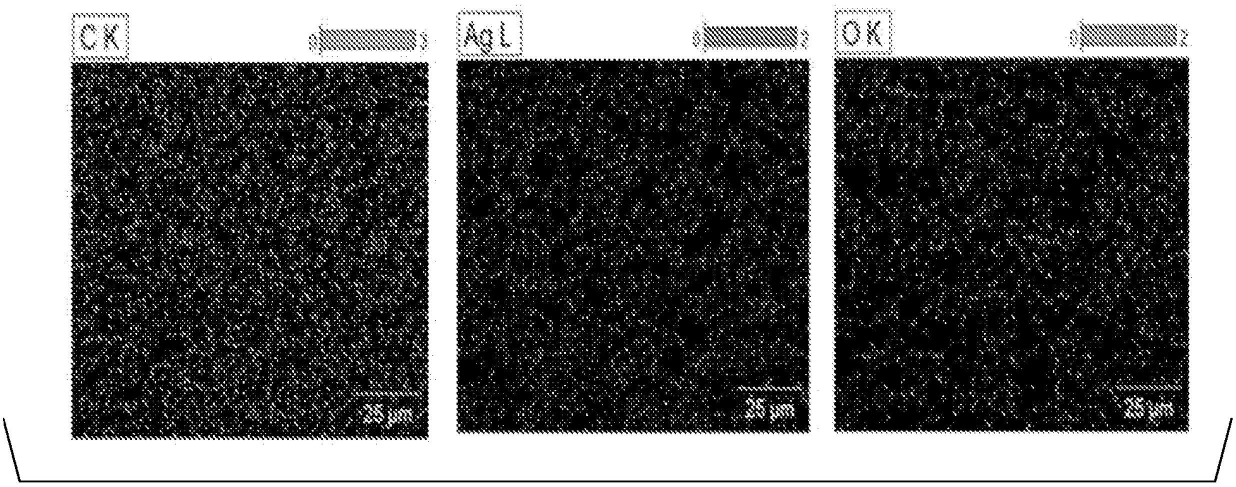


FIG. 10C

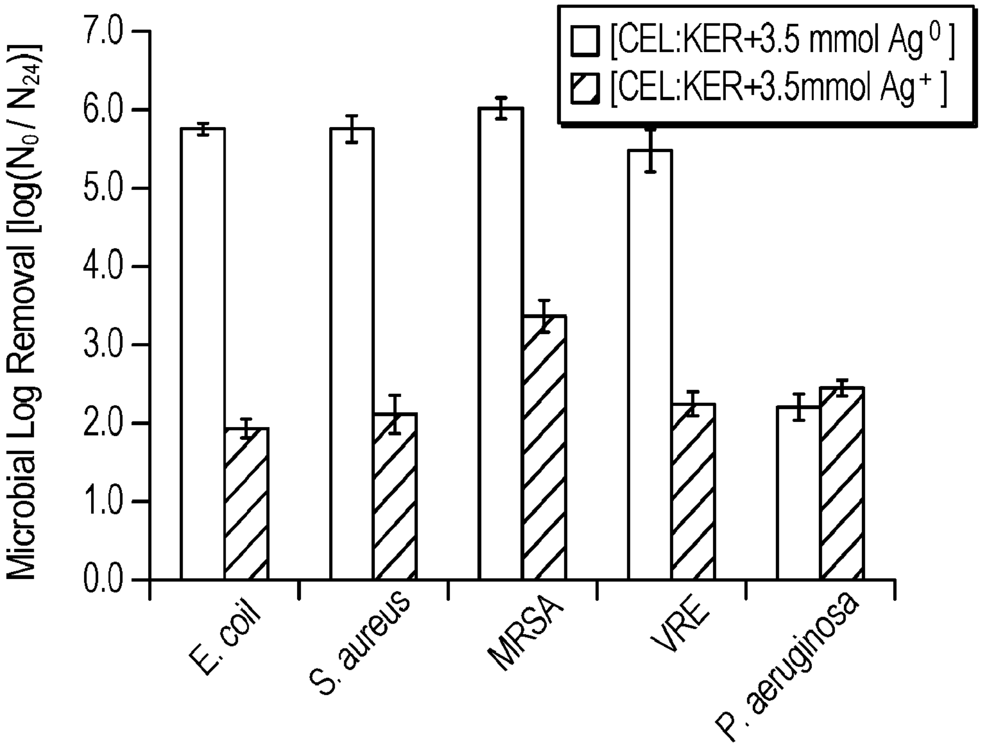


*FIG. 11*

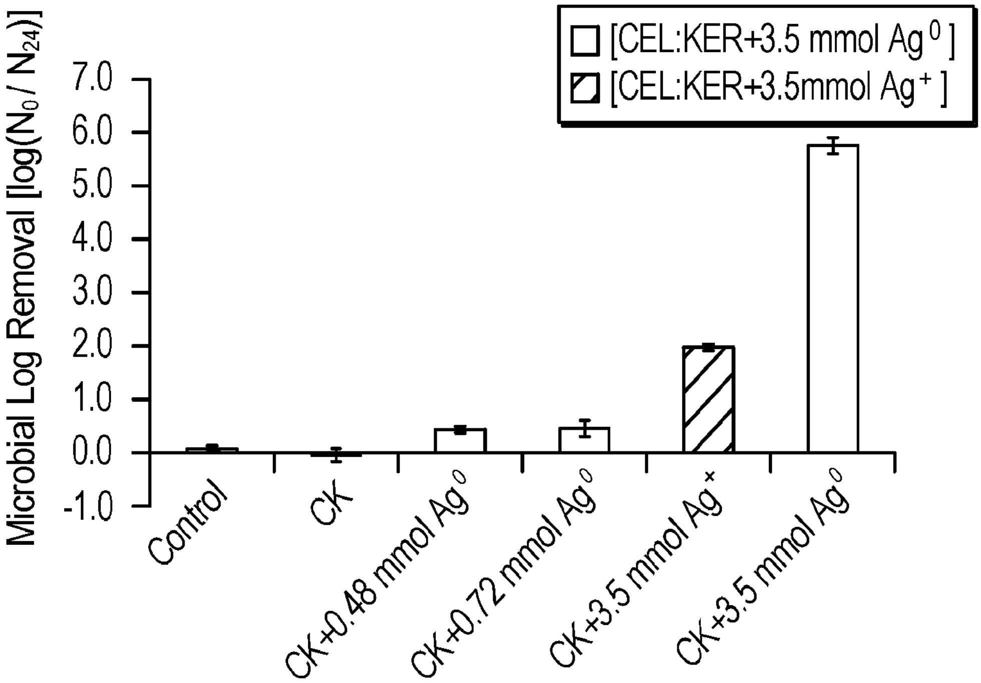


*FIG. 11A*

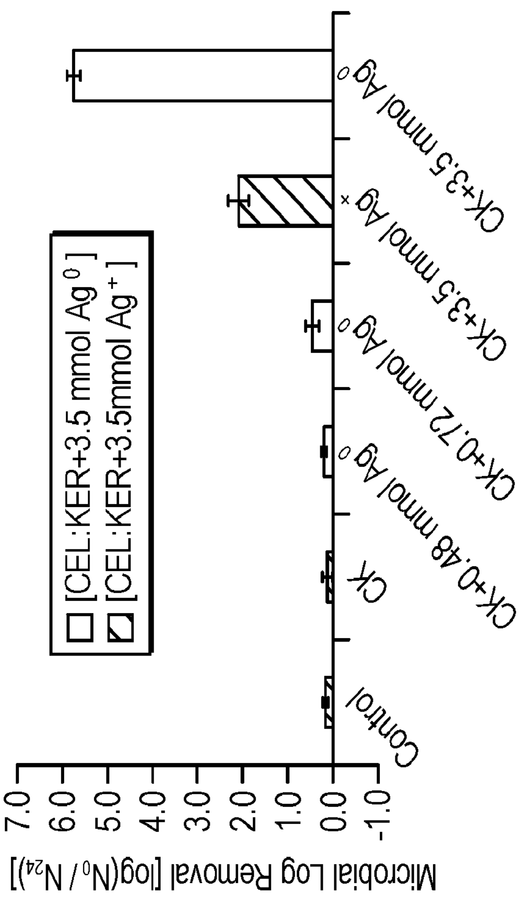


**FIG. 11B**

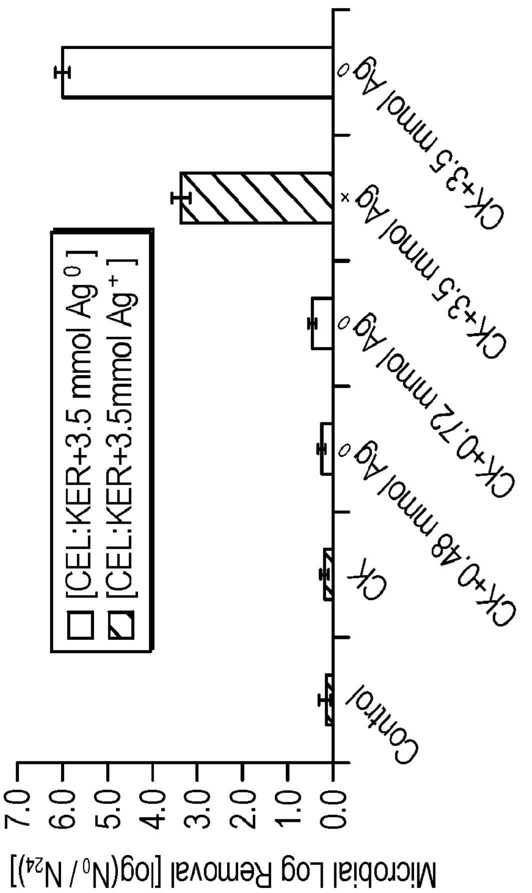


**FIG. 11D**

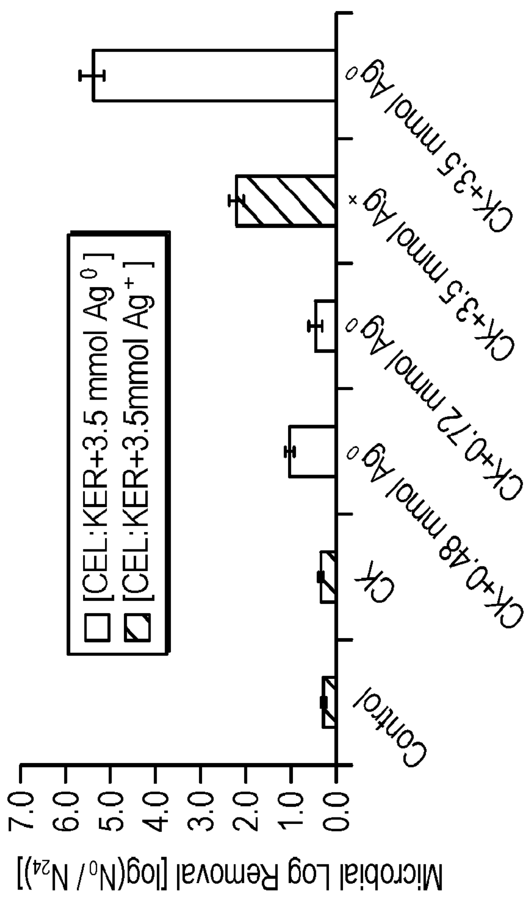


**FIG. 11C**

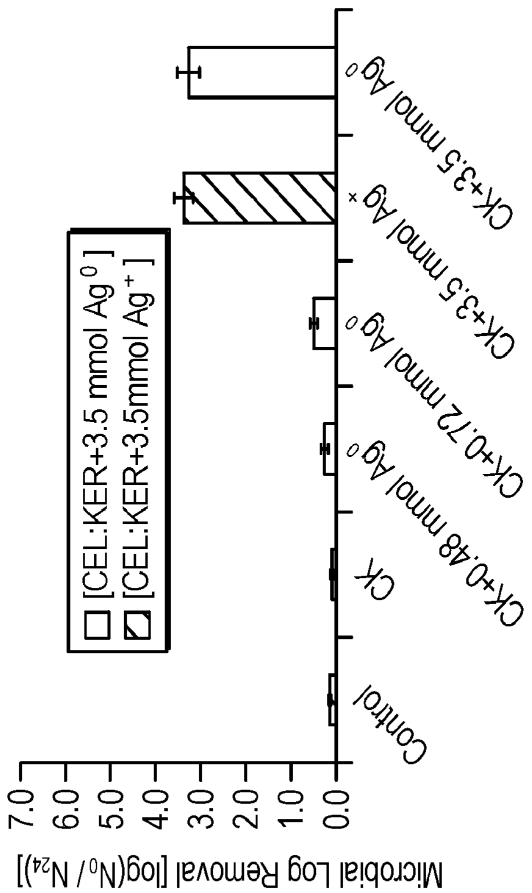


**FIG. 11E**

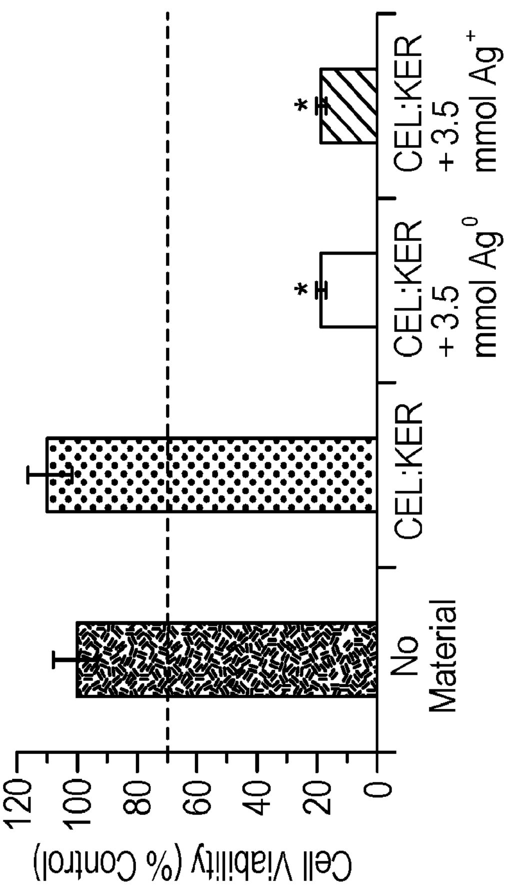


**FIG. 12A**

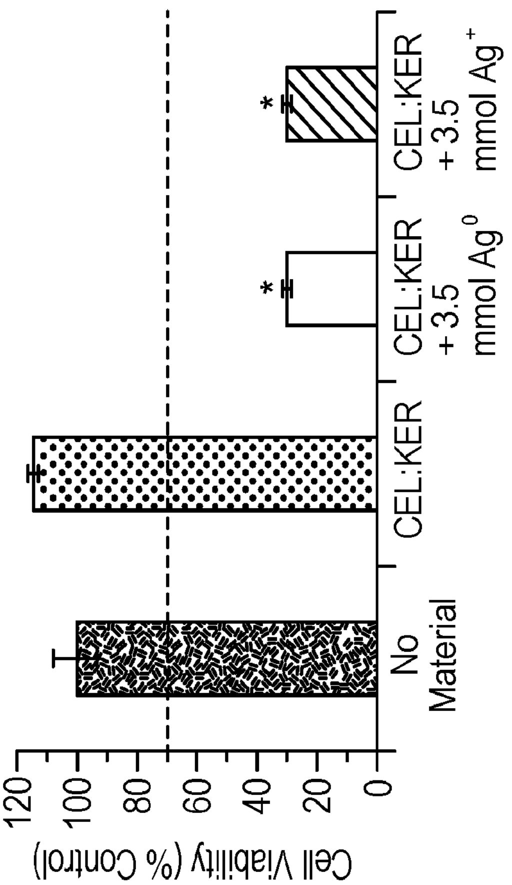


**FIG. 12B**

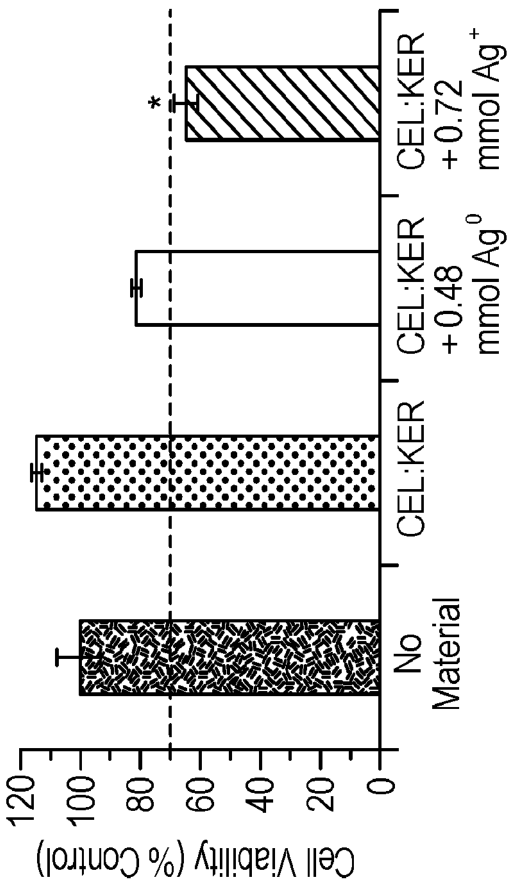


**FIG. 12C**

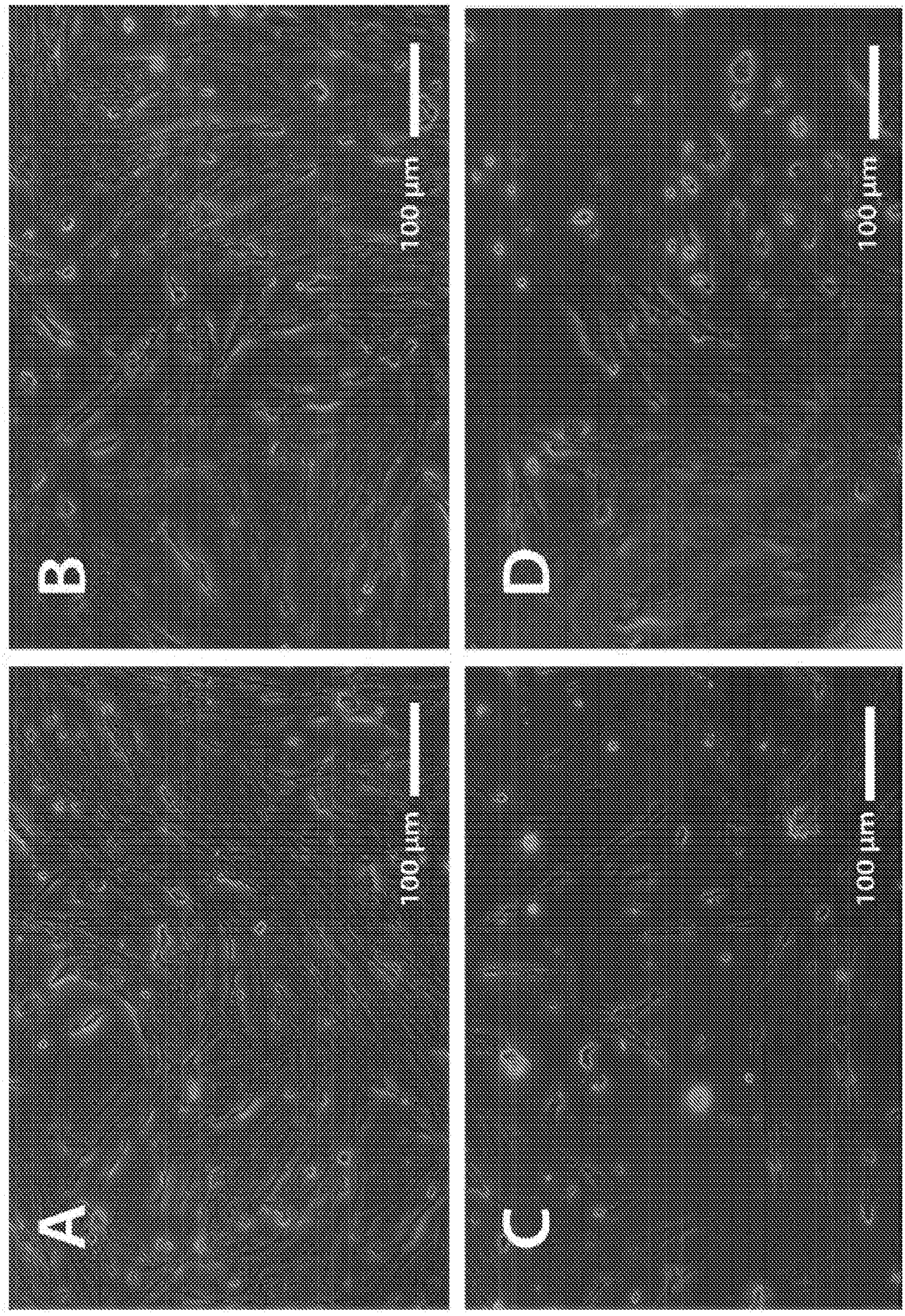


Figure 13

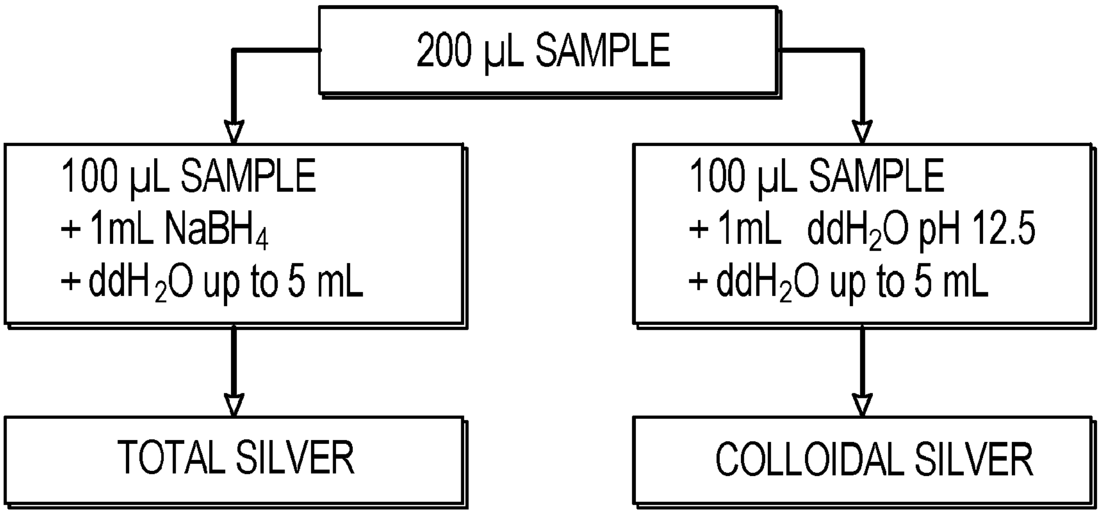
200 µL SAMPLE
100 µL SAMPLE
+ 1mL $NaBH_4$
+ $ddH_2O$ up to 5 mL
100 µL SAMPLE
+ 1mL $ddH_2O$ pH 12.5
+ $ddH_2O$ up to 5 mL
TOTAL SILVER
COLLOIDAL SILVER

*FIG. 14*

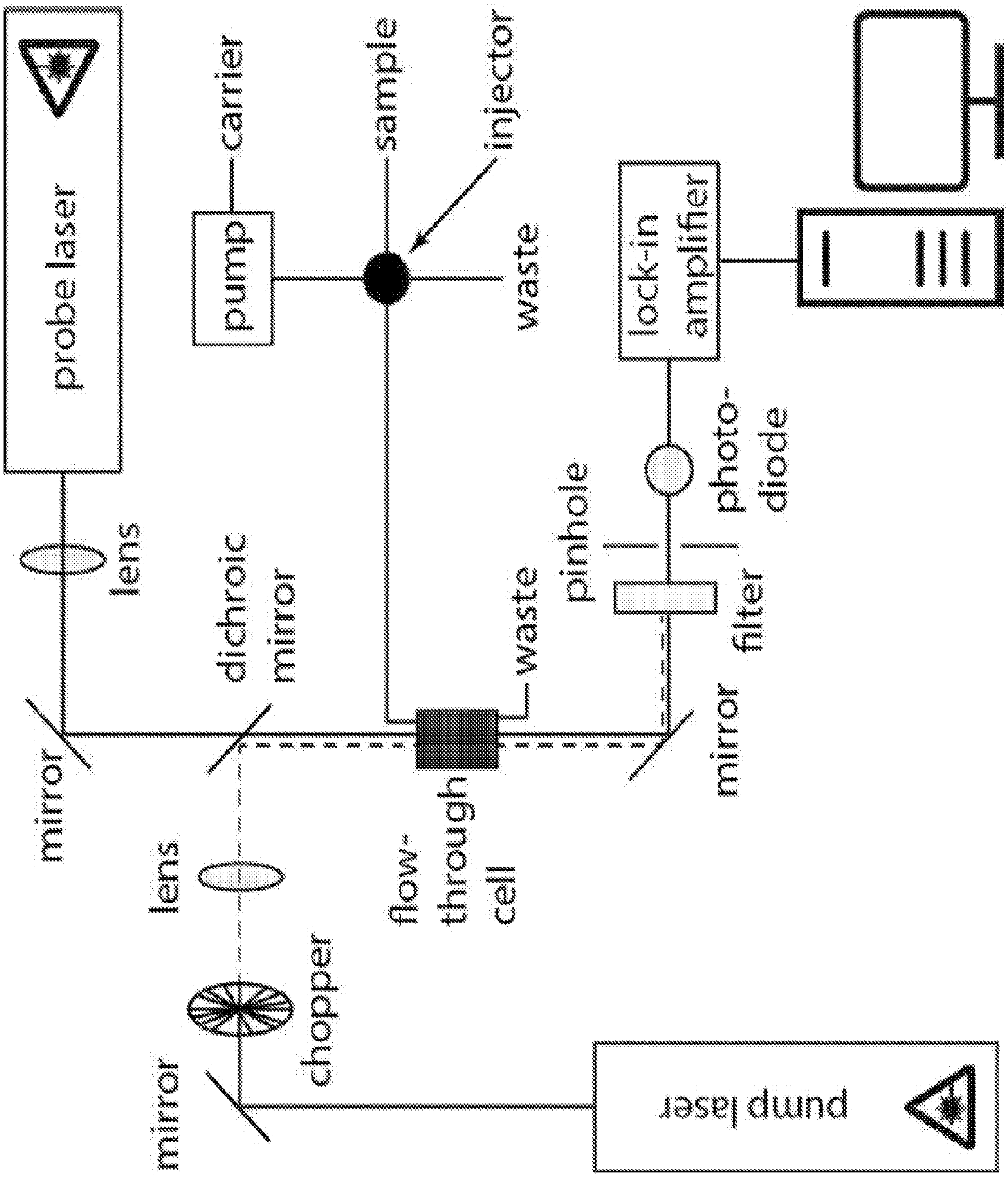
probe laser
lens
mirror
dichroic
mirror
lens
chopper
mirror
pump laser
flow-
through
cell
waste
pump
carrier
sample
injector
waste
mirror
filter
pinhole
photo-
diode
lock-in
amplifier

Figure 15

Figure 16

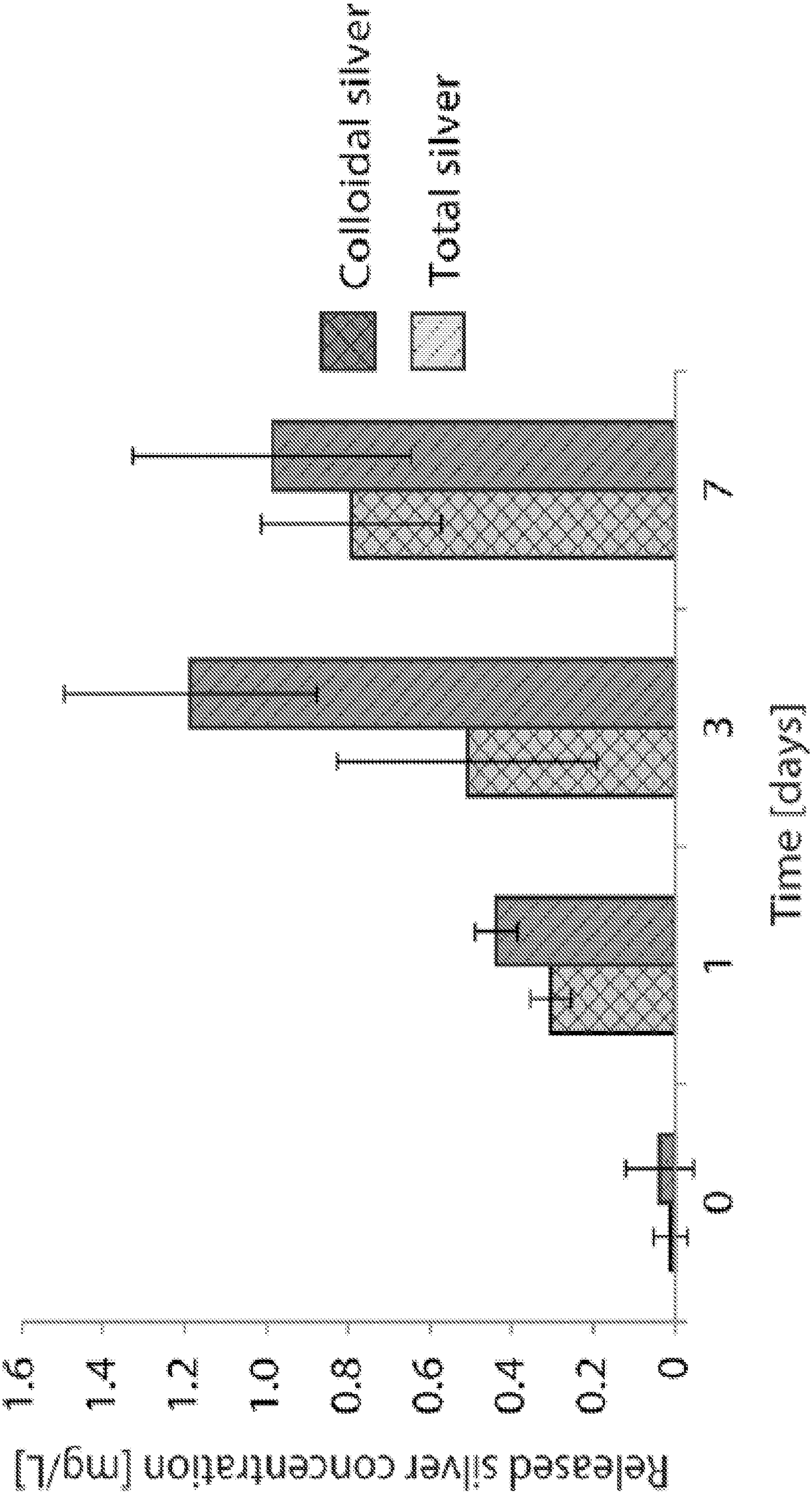
Colloidal silver
Total silver
Released silver concentration [mg/L]
1.6
1.4
1.2
1.0
0.8
0.6
0.4
0.2
0
0
1
3
7
Time [days]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62305757 A
- WO 2018075614 A
- WO 2004084627 A
- WO 2014186702 A
- US 20050232963 A1


### Non-patent literature cited in the description


- **WU et al.** *Industrial & Engineering Chemistry Research*, 2009, vol. 48 (15), 7132-7136
- **PENG et al.** *Industrial & Engineering Chemistry Research*, 2014, vol. 53 (6), 2106-2113
- **VENKATESWARLU et al.** *Dalton Trans.*, 2015, vol. 44, 18427-18437
- **TRAN et al.** *J. Biomed. Mater. Res. Part A*, 2013, vol. 101A, 2248-2257
- **TRAN et al.** *J. of Hazard. Mat.*, 2013, 252-253, 355-366
- **DURI et al.** *Langmuir*, 2014, vol. 30 (2), 642-650
- **TRAN et al.** *J. Biomed. Mater. Res.*, 2013, vol. 101A, 2248-2257
- **HARKINS AL** ; **DURI S** ; **KLOTH LC** ; **TRAN CD et al.** Chitosan-cellulose composite for wound dressing material. Part 2. Antimicrobial activity, blood absorption ability, and biocompatibility. *J Biomed Mater Res Part B*, 2014, vol. 00B, 000-000
- **DURI et al.** Supramolecular Composition Materials from Cellulose, Chitosan, and Cyclodextrins: Facile Preparation and Their Selective Inclusion Complex Formation with Endocrine Disruptors. *Langmuir*, 2013, vol. 29 (16), 5037-49
- **TRAN et al.** Synthesis, structure and antimicrobial property of green composites from cellulose, wool, hair and chicken feather. *Carbohydrate Polymers*, 2016, vol. 151, 1269-1276
- **APPELBAUM, P. C.** Microbiology of antibiotic resistance in Staphylococcus aureus.. *Clinical Infectious Diseases*, 2007, vol. 45, S165-S170
- **CHEN, J.** ; **VONGSANGA, K.** ; **WANG, X.** ; **BYRNE, N.** What happens during natural protein fibre dissolution in ionic liquids. *Material*, 2014, vol. 7, 6158-6168
- **CILURZO, C.** ; **SELMIN, F.** ; **ALUIGI, A.** ; **BELLOSTA, S.** Regenerated keratin proteins as potential biomaterial for drug delivery. *Polymers for Advance Technologies*, 2013, vol. 24, 1025-1028
- **CUI, L.** ; **GONG, J.** ; **FAN, X.** ; **WANG, P.** ; **WANG, Q.** ; **QIU, Y.** Trans glutaminase-modified wool keratin film and its potential application in tissue engineering. *Engineering in Life Sciences*, 2013, vol. 13, 149-155
- Keratin: structure, properties, and applications. Hauppauge. Nova Science Publishers, 2012
- Supramolecular composite materials from cellulose, chitosan and cyclodextrin: facile preparation and their selective inclusion complex formation with endocrine disruptors. **DURI, S** ; **TRAN, C. D.** Langmuir. 2013, vol. 29, 5037-5049
- **DURI, S.** ; **MAJONI, S.** ; **HOSSENLOPP, J. M.** ; **TRAN, C. D.** Determination of chemical homogeneity of fire retardant polymeric nanocomposite materials by near-infrared multispectral imaging microscopy. *Analytical Letters*, 2010, vol. 43, 1780-1789
- **GREVE, T. M., ANDERSEN, K. B., & NIELSEN, O. F.** Penetration mechanism of dimethyl sulfoxide in human and pig ear skin: an ATR-FTIR and near-FT Raman Spectroscopic in vivo and in vitro study. *Spectroscopy*, 2008, vol. 22, 405-417
- **HARKINS, A. L.** ; **DURI, S** ; **KLOTH, L. C.** ; **TRAN, C. D**. Chitosan-cellulose composite for wound dressing material. Part 2. Antimicrobial activity, blood absorption ability, and biocompatibility.. *Journal of Biomedical Materials Research Part B*, 2014, vol. 102, 1199-1206
- **HAVERHALS, L. M.** ; **REICHERT, W. M.** ; **NAZARE, N.** ; **ZAMMARANO, M.** ; **GILMAN, J. W.** ; **DE LONG, H. C. et al.** Ionic liquid facilitated introduction of functional materials into biopolymer polymer substrates, in molten salts and ionic liquids 18.. *ECS Transactions*, 2012, vol. 50 (11), 631-640
- **HILL, P.** ; **BRANTLEY, H.** ; **VAN DYKE, M.** Some properties of keratin biomaterials: kerateines. *Biomaterials*, 2010, vol. 1, 585-593
- **JORGENSEN, J. H.** ; **FERRARO, M. J.** ; **JORGENSEN, J. H** ; **FERRARO, M. J.** Antimicrobial susceptibility testing: a review of general principles and contemporary practices. *Clinical Infectious Diseases*, 2009, vol. 49 (11), 1749-1755

- **JUSTIN, M.** ; **SAUL, M. D.** ; **ELLENBURG, R.** ; **DE GUZMAN, C.** ; **VAN DYKE, M.** Keratin hydrogels support the sustained release of bioactive ciprofloxacin. *Journal of Biomedical Materials Research Part A*, 2011, vol. 98, 544-553
- **MCKITTRICK, J.** ; **CHEN, P. Y.** ; **BODDE, S. G.** ; **YANG, W.** ; **NOVITSKAYA, E. E.** ; **MEYERS, M. A.** The structure, functions, and mechanical properties of keratin. *JOM*, 2012, vol. 64, 449-468
- **MUTUTUVARI, T. M.** ; **TRAN, C. D**. Synergistic adsorption of heavy metal ions and organic pollutants by polysaccharide supramolecular composite materials from cellulose, chitosan and crown ether.. *Journal of Hazardous Materials*, 2014, vol. 264, 449-459
- **MUTUTUVARI, T. M.** ; **HARKINS, A. L.** ; **TRAN, C. D.** Facile synthesis, characterization and antimicrobial activity of cellulose-Chitosan-Hydroxy apatite composite material, a potential material for bone tissue engineering. *Journal of Biomedical Materials Research Part A*, 2013, vol. 101 (11), 3266-3277
- **SANDO, L.** ; **KIM, M.** ; **COLGRAVE, M. L.** ; **RAMSHAW, J. A** ; **WERKMEISTER, J. A** ; **ELVIN, C. M**. Photochemical crosslinking of soluble wool keratins produces a mechanically stable biomaterial that supports cell adhesion and proliferation.. *Journal of Biomedical Materials Research Part A*, 2010, vol. 95, 901-911
- **SOWA, M. G.** ; **WANG, J.** ; **SCHULTZ, C. P.** ; **AHMED, M. K.** ; **MANTSCH, H. H.** Infrared spectroscopic investigation of in vivo and ex vivo human nails.. *Vibrational Spectroscopy*, 1995, vol. 10, 49-56
- **TANABE, T.** ; **OKITSU, N.** ; **TACHIBANA, A.** ; **YAMAUCHI, K.** Preparation and characterization of keratin-chitosan composite film. *Biomaterials*, 2002, vol. 23, 817-825
- **TRAN, C. D** ; **MUTUTUVARI, T. M.** Cellulose, chitosan and keratin composite materials controlled drug release. *Langmuir*, 2015, vol. 31, 1516-1526
- **TRAN, C. D.** ; **DURI, S.** ; **HARKINS, A. L.** Recyclable synthesis, characterization, and antimicrobial activity of chitosan-based polysaccharide composite materials.. *Journal of Biomedical Materials Research Part A*, 2013, vol. 101, 2248-2257
- **TRAN, C. D.** ; **DURI, S.** ; **DELNERI, A.** ; **FRANKO, M.** Chitosan-cellulose composite materials: preparation, characterization and application for removal of microcystin.. *Journal of Hazardous Materials*, 2013, vol. 252, 355-366
- **VASCONCELOS, A.** ; **CAVACO-PAULO, A.** The use of keratin in biomedical applications.. *Current Drug Targets*, 2013, vol. 14, 612-619
- **VERMA, V.** ; **VERMA, P.** ; **RAY, A. R.** Preparation of scaffolds from human hair proteins for tissue-engineering applications.. *Biomedical Materials*, 2008, vol. 3, 2500
- **VILAPLANA, F.** ; **STROEMBERG, E.** ; **KARLSSON, S.** Environmental and resource aspects of sustainable biocomposites.. *Polymer Degradation and Stability*, 2010, vol. 95 (11), 2147-2161
- **XIE, H.** ; **LI, S** ; **ZHANG, S.** Ionic liquids as novel solvents for the dissolution and blending of wool keratin fibers.. *Green Chemistry*, 2005, vol. 7, 606-608
- **TRAN et al.** One-Pot Synthesis of Biocompatible Silver Nanoparticle Composites from Cellulose and Keratin: Characterization and Antimicrobial Activity. *Applied Materials & Interfaces*, 2016, vol. 8, 34791-34801
- **YANG, X.** ; **YANG, M.** ; **PANG, B.** ; **VARA, M.** ; **XIA, Y.** Gold Nanomaterials at Work in Biomedicine.. *Chem. Rev*, 2015, vol. 115, 10410-10488
- **XIA, X** ; **ZENG, J.** ; **ZHANG, Q** ; **MORAN, C. H.** ; **XIA, Y.** Recent Developments in Shape-Controlled Synthesis of Silver Nanocrystals.. *J. Phys. Chem. C*, 2012, vol. 116, 21647-21656
- **CHAN, W. C. W. et al.** A Year for Nanoscience.. *ACS Nano*, 2014, vol. 8, 11901-11903
- **PELAZ, B. et al.** The State of Nanoparticle-Based Nanoscience and Biotechnology: Progress, Promises, and Challenges. *ACS Nano*, 2012, vol. 6, 8468-8483
- **SARDAR, R.** ; **SHUMAKER-PARRY, J. S.** Spectroscopic and Microscopic Investigation of Gold Nanoparticle Formation: Ligand and Temperature Effects on Rate and Particle Size.. *J. Am. Chem. Soc.*, 2011, vol. 133, 8179-8190
- **WANG, Z.** ; **BHARATHI, M. S.** ; **HARIHARAPUTRAN, R.** ; **XING, H.** ; **TANG, L.** ; **LI, J.** ; **ZHANG, Y.-W.** ; **LU, Y.** pH-Dependent Evolution of Five-Star Gold Nanostructures: An Experimental and Computational Study.. *ACS Nano*, 2013, vol. 7, 2258-2265
- **WRIGHT, A. R** ; **LI, M.** ; **RAVULA, S.** ; **CADIGAN, M** ; **EL-ZAHAB, B.** ; **DAS, S** ; **BAKER, G. A.** ; **WARNER, I. M.** Soft-and Hard-Templated Organic Salt Nanoparticles with the Midas Touch: Gold-Shelled NanoGUMBOS.. *J. Mater. Chem. C*, 2014, vol. 2, 8996-9003
- **TAKAGAI, Y.** ; **MIURA, R.** ; **ENDO, A.** ; **HINZE, W. L.** One-pot Synthesis with in situ Preconcentration of Spherical Monodispersed Gold Nanoparticles using Thermoresponsive 3-(alkyldimethylammonio)-propyl sulfate Zwitterionic Surfactants.. *Chem. Commun.*, 2016, vol. 52, 10000-10003
- **TREFRY, J. C.** ; **WOOLEY, D. P.** Silver Nanoparticles Inhibit Vaccinia Virus Infection by Preventing Viral entry through a Macro-pinocytosis-dependent Mechanism.. *J. Biomed. Nanotechnol.*, 2013, vol. 9, 1624-1635

- **NOH, H. J.** ; **IM, A.-R.** ; **KIM, H.-S.** ; **SOHNG, J. K.** ; **KIM, C.-K.** ; **KIM, Y. S.** ; **CHO, S.** ; **PARK, Y.** Antibacterial Activity and Increased Freeze-drying Stability of Sialyllactosereduced Silver Nanoparticles using Sucrose and Trehalose. *J. Nanosci. Nanotechnol.*, 2012, vol. 12, 3884-3895
- **GUZMAN, M.** ; **DILLE, J.** ; **GODET, S.** Synthesis and Antibacterial Activity of Silver Nanoparticles against Gram-positive and Gram-negative Bacteria.. *Nanomedicine*, 2012, vol. 8, 37-45
- **MALLAKPOUR, S.** ; **DINARI, M.** ; **TALEBI, M. A FACILE**. Efficient, and Green Fabrication of Nanocomposites based on L-leucine Containing Poly(amide-imide) and PVA-modified Ag Nanoparticles by Ultrasonic Irradiation Colloid. *Colloid Polym. Sci.*, 2015, vol. 293, 1827-1833
- **GANGADHARAN, D.** ; **HARSHVARDAN, K.** ; **GNANASEKAR, G** ; **DIXIT, D.** ; **POPAT, K. M.** ; **ANAND, P. S.** Polymeric Microspheres Containing Silver Nanoparticles as a Bactericidal Agent for Water Disinfection.. *Water Res*, 2010, vol. 44, 5481-5487
- **WEI, D.** ; **SUN, W.** ; **QIAN, W.** ; **YE, Y.** ; **MA, Y.** The Synthesis of Chitosan-based Silver Nanoparticles and their Antibacterial Activity. *Carbohydr. Res.*, 2009, vol. 344, 2375-2382
- **JOHNSTON, J. H.** ; **NILSSON, T.** Nanogold and Nanosilver Composites with Lignin-containing Cellulose Fibres.. *J. Mater. Sci.*, 2012, vol. 47, 1103-1112
- **WU, J.** ; **ZHENG, Y.** ; **SONG, W.** ; **LUAN, J.** ; **WEN, X.** ; **WU, Z.** ; **CHEN, X.** ; **WANG, Q.** ; **GUO, S.** In situ Synthesis of Silver-Nanoparticles/ bacterial Cellulose Composites for Slow-released Antimicrobial Wound Dressing. Carbohydr.. *Polym*, 2014, vol. 102, 762-771
- **KELLY, F. M.** ; **JOHNSTON, J. H.** Colored and Functional Silver Nanoparticle-Wool Fiber Composites.. *ACS Appl. Mater. Interfaces*, 2011, vol. 3, 1083-1092
- **HILL, P.** ; **BRANTLEY, H.** ; **VAN DYKE, M.** Some Properties of Keratin Biomaterials: Kerateines. *Biomaterials*, 2010, vol. 31, 585-593
- **VASCONCELOS, A.** ; **CAVACO-PAULO, A.** The Use of Keratin in Biomedical Applications.. *Curr. Drug Targets*, 2013, vol. 14, 612-619
- **SANDO, L.** ; **KIM, M** ; **COLGRAVE, M. L.;** ; **RAMSHAW, J. A.** ; **WERKMEISTER, J. A.** ; **ELVIN, C. M.** Photochemical Crosslinking of Soluble Wool Keratins Produces a Mechanically Stable Biomaterial that Supports Cell Adhesion and Proliferation.. *J. Biomed. Mater. Res., Part A*, 2010, vol. 95, 901-911
- **YAMAUCHI, K.** ; **MANIWA, M.** ; **MORI, T.** Cultivation of Fibroblast Cells on Keratin-coated Substrates.. *J. Biomater. Sci., Polym. Ed*, 1998, vol. 9, 259-270
- **CUI, L.** ; **GONG, J.** ; **FAN, X.** ; **WANG, P.** ; **WANG, Q** ; **QIU, Y**. Trans Glutaminase-modified Wool Keratin Film and its Potential Application in Tissue Engineering. *Eng. Life Sci.*, 2013, vol. 13, 149-155
- **XU, S.** ; **SANG, L.** ; **ZHANG, Y.** ; **WANG, X** ; **LI, X.** Biological Evaluation of Human Hair Keratin Scaffolds for Skin Wound Repair and Regeneration. *Mater. Sci. Eng., C*, 2013, vol. 33, 648-655
- **DE GUZMAN, R. C.** ; **MERRILL, M. R.** ; **RICHTER, J. R.** ; **HAMZI, R. I** ; **GREENGAUZ-ROBERTS, O. K.** ; **VAN DYKE, M. E.** Mechanical and Biological Properties of Keratose Biomaterials.. *Biomaterials*, 2011, vol. 32, 8205-8217
- **IQBAL, H. M.N.** ; **KYAZZE, G.** ; **LOCKE, I. C.** ; **TRON, T.** ; **KESHAVARZ, T.** In situ Development of Self-defensive Antibacterial Biomaterials: Phenol-g-keratin-EC based Biocomposites with Characteristics for Biomedical Applications.. *Green Chem*, 2015, vol. 17, 3858-3869
- **ALUIGI, A.** ; **VINEIS, C.** ; **VARESANO, A.** ; **MAZZUCHETTI, G.** ; **FERRERO, F.** ; **TONIN, C.** Structure and Properties of Keratin/PEO blend Nanofibers.. *Eur. Polym. J*, 2008, vol. 44, 2465-2475
- **KHOSA, M. A.** ; **ULLAH, A.** A Sustainable Role of Keratin Biopolymer in Green Chemistry: A Review.. *J. Food Proc. Beverages*, 2013, vol. 1, 8-15
- **ROSEWALD, M.** ; **HOU, F. Y. S** ; **MUTUTUVARI, T.** ; **HARKINS, A. L.** ; **TRAN, C. D.** Cellulose-Chitosan-Keratin Composite Materials: Synthesis and Immunological and Antibacterial Properties.. *ECS Trans*, 2014, vol. 64 (4), 499-505
- **TRAN, C. D.** ; **MUTUTUVARI, T.** Cellulose, Chitosan and Keratin Composite Materials. Controlled Drug Release. *Langmuir*, 2015, vol. 31, 1516-1526
- **TRAN, C. D.** ; **MUTUTUVARI, T.** Cellulose, Chitosan and Keratin Composite Materials. Facile and Recyclable Synthesis, Conformation and Properties.. *ACS Sustainable Chem. Eng.*, 2016, vol. 4, 1850-1861
- **TRAN, C. D.** ; **PROSENC, F.** ; **FRANKO, M.** ; **BENZI, G.** Synthesis, Structure and Antimicrobial Property of Green Composites from Cellulose, Wool, Hair and Chicken Feather.. *Carbohydr. Polym*, 2016, vol. 151, 1269-1276
- **TRAN, C. D** ; **DURI, S.** ; **HARKINS, A. L.** Recyclable Synthesis, Characterization and Antimicrobial Activity of Chitosan-based Polysaccharide Composite Materials.. *J. Biomed. Mater. Res., Part A*, 2013, vol. 101, 2248-2257
- **HARKINS, A. L.** ; **DURI, S** ; **KLOTH, L. C.** ; **TRAN, C. D.** Chitosan- Cellulose Composite for Wound Dressing Material. Part 2. Antimicrobial Activity, Blood Absorption Ability and Biocompatibility.. *J. Biomed. Mater. Res., Part B*, 2014, vol. 102 (6), 1199-1206

- **MUTUTUVARI, T. M.** Supramolecular Biopolymeric Composite Materials: Green Synthesis, Characterization and Applications. Dissertation. Marquette University, 2014
- **LI, R.** ; **WANG, D.** Preparation of Regenerated Wool Keratin Films from Wool Keratin-ionic liquid Solutions. *J. Appl. Polym. Sci.*, 2013, vol. 127, 2648-2653
- **FANG, J. Y.** ; **CHEN, J. P.** ; **LEU, Y. L.** Wang, H. Y. Characterization and Evaluation of Silk Protein Hydrogels for Drug Delivery. *Chem. Pharm. Bull.*, 2006, vol. 54, 156-162
- **DONG, R.** ; **TIAN, B.** ; **ZENG, C.** ; **LI, T.** ; **WANG, T** ; **ZHANG, J.** Ecofriendly Synthesis and Photocatalytic Activity of Uniform Cubic Ag@AgCl Plasmonic Photocatalyst.. *J. Phys. Chem. C*, 2013, vol. 117, 213-220
- **VERONICA DA SILVA FERREIRAA, V. D. S** ; **CONZFERREIRAA, M. E.** ; **LIMA, L. M. T. R.** Green Production of Microalgae-based Silver Chloride Nanoparticles with Antimicrobial Activity against Pathogenic Bacteria Enzym.. *Microbial. Tech.*, 2016, http://dx.doi. org/10.1016/j.enzmictec.2016.10.018
- **DHAS, T. S.** ; **KUMAR, V. G.** ; **KARTHICK, V** ; **ANGEL, K. J.** ; **GOVINDARAJU, K.** Facile Synthesis of Silver Chloride Nanoparticles using Marine Alga and its Antibacterial Efficacy, Spectrochim. Acta A Mol. Biomol. Spectrosc.. *Acta A Mol. Biomol. Spectrosc*, 2014, vol. 120, 416-420
- **SOHRABNEZHAD, SH.** ; **RASSA, M.** ; **DAHANE-SARI, E. M.** Spectroscopic Study of Silver Halides in Montmorillonite and their Antibacterial Activity.. *J. Photochem. Photobiol., B*, 2016, vol. 163, 150-155
- **SHARMA, P.** ; **SANPUI, P.** ; **CHATTOPADHYAY, A.** ; **GHOSH, S.** Fabrication of Antibacterial Silver Nanoparticle - Sodium Alginate - Chitosan Composite Films.. *RSC Adv*, 2012, vol. 2, 5837-5843
- **SATHISHKUMAR, M** ; **SNEHA, K.** ; **YUN, Y. S.** Immobilization of Silver Nanoparticles Synthesized using Curcuma Longa Tuber Powder and Extract on Cotton Cloth for Bactericidal Activity.. *Bioresour. Technol*, 2010, vol. 101, 7958-7965
- **SCHERRER, P.** Bestimmung der Grösse und der Inneren Struktur von Kolloidteilchen Mittels Röntgenstrahlen,.. *Nachr. Ges. Wiss. Göttingen*, 1918, vol. 26, 98-100
- **LANGFORD, J. J** ; **WILSON, A. J. C.** Scherrer after Sixty Years: A Survey and Some New Results in the Determination of Crystallite Size.. *J. Appl. Crystallogr.*, 1978, vol. 11, 102-113
- **KORTE, D.** ; **CONCETTA BRUZZONITI, M** ; **SARZANINI, C.** ; **FRANKO, M.** Thermal Lens Spectrometric Determination of Colloidal and Ionic Silver in Water.. *Int. J. Thermophys.*, 2011, vol. 32, 818-827
- Thermal Lens Spectroscopy.. **TRAN, C. D.** ; **FRANKO, M.** Encyclopedia of Analytical Chemistry. John Wiley, 2010
- **KORTE, D** ; **BRUZZONITI, M. C.** ; **SARZANINI, C** ; **FRANKO, M.** Thermal Lens Spectrometric Determination of Colloidal and Ionic Silver in Water.. *Int. J. Thermophys.*, 2011, vol. 32, 818-827
- Thermal Lens Spectroscopy. **TRAN, C. D.** ; **FRANKO, M.** Encyclopedia of Analytical Chemistry. John Wiley, 2010